(19) **Europäisches Patentamt / European Patent Office / Office européen des brevets**

(11) **EP 4 503 047 A2**

(12) # EUROPEAN PATENT APPLICATION

(43) Date of publication:
05.02.2025 Bulletin 2025/06

(21) Application number: 24206702.3

(22) Date of filing: 16.03.2015

(51) International Patent Classification (IPC):
**G16H 20/60** (2018.01)

(52) Cooperative Patent Classification (CPC):
**G16H 20/30; A61B 5/14532; A61B 5/7239;
A61B 5/7405; A61B 5/742; A61B 5/746;
A61B 5/7475; G16H 20/60; G16H 40/63;**
A61B 2560/0295

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: 10.04.2014 US 201461978151 P

(62) Document number(s) of the earlier application(s) in
accordance with Art. 76 EPC:
**18194755.7 / 3 437 558
15714332.2 / 3 128 913**

(71) Applicant: DexCom, Inc.
San Diego, CA 92121 (US)

(72) Inventors:
- **RACK-GOMER, Anna Leigh**
San Diego, 92121 (US)
- **HAMPAPURAM, Hari**
San Diego, 92121 (US)
- **KAMATH, Apurv Ullas**
San Diego, 92121 (US)
- **REIHMAN, Eli**
San Diego, 92121 (US)
- **BOWMAN, Leif N.**
San Diego, 92121 (US)
- **GARCIA, Arturo**
San Diego, 92121 (US)

- **BHAVARAJU, Naresh C.**
San Diego, 92121 (US)
- **DRAEGER, Rian**
San Diego, 92121 (US)
- **KRAMER, Paul**
San Diego, 92121 (US)
- **NOBLE-CAMPBELL, Paul**
San Diego, 92121 (US)
- **GRUBSTEIN, Katherine Yerre**
San Diego, 92121 (US)

(74) Representative: **Dentons UK and Middle East LLP
One Fleet Place
London EC4M 7WS (GB)**

Remarks:
•A request for correction of the description has been filed pursuant to Rule 139 EPC. A decision on the request will be taken during the proceedings before the Examining Division (Guidelines for Examination in the EPO, A-V, 3.).
•This application was filed on 15.10.2024 as a divisional application to the application mentioned under INID code 62.
•Claims filed after the date of filing of the application (Rule 68(4) EPC).

(54) **GLYCEMIC URGENCY ASSESSMENT AND ALERTS INTERFACE**

(57)     Systems and methods are disclosed that employ several or numerous factors in the determination of a glycemic urgency index (GUI), which may be based on a measured blood glucose level as well as other factors. The other factors may include time derivatives of the glucose level and/or other factors, e.g., user - entered data, data measured by other sensors or received from a network source, or historical data. The GUI is then presented to the user in an interesting way, e.g., via a background color or other inconspicuous notifier, e.g., on a mobile device such as a smart phone. The GUI may also be employed in the triggering of actionable alerts and alarms on an electronic device for the user. The GUI, or another index calculated from combinations of the variables and parameters described, may further be employed to drive a medicament delivery device such as a pump.

EP 4 503 047 A2

FIG. 1

**Description**

Incorporation by Reference to Related Application

Any and all priority claims identified in the Application Data Sheet, or any correction thereto, are hereby incorporated by reference under 37 CFR 1.57. This application claims the benefit of U.S. Provisional Application No. 61/978,151 filed April 10, 2014. The aforementioned application is incorporated by reference herein in its entirety, and is hereby expressly made a part of this specification.

Technical Field

The present embodiments relate to continuous analyte monitoring, and, in particular, to signal analysis and result presentation of a continuous analyte monitoring system.

Background

Diabetes mellitus is a disorder in which the pancreas cannot create sufficient insulin (Type I or insulin-dependent) and/or in which insulin is not effective (Type II or non-insulin-dependent). In the diabetic state, the victim suffers from high blood sugar, which can cause an array of physiological derangements associated with the deterioration of small blood vessels, for example, kidney failure, skin ulcers, or bleeding into the vitreous of the eye. A hypoglycemic reaction (low blood sugar) can be induced by an inadvertent overdose of insulin, or after a normal dose of insulin or glucose - lowering agent accompanied by extraordinary exercise or insufficient food intake.

Conventionally, a person with diabetes carries a self - monitoring blood glucose (SMBG) monitor, which typically requires uncomfortable finger pricking methods. Due to the lack of comfort and convenience, a person with diabetes normally only measures his or her glucose levels two to four times per day. Unfortunately, such time intervals are so far spread apart that the person with diabetes likely finds out too late of a hyperglycemic or hypoglycemic condition, sometimes incurring dangerous side effects. It is not only unlikely that a person with diabetes will become aware of a dangerous condition in time to counteract it, but it is also likely that he or she will not know whether his or her blood glucose value is going up (higher) or down (lower) based on conventional method. Diabetics thus may be inhibited from making educated insulin therapy decisions.

Another device that some diabetics used to monitor their blood glucose is a continuous analyte sensor, e.g., a continuous glucose monitor (CGM). A CGM typically includes a sensor that is placed invasively, minimally invasively or non-invasively. The sensor measures the concentration of a given analyte within the body, e.g., glucose, and generates a raw signal that is generated by electronics associated with the sensor. The raw signal is converted into an output value that is displayed on a display. The output value that results from the conversion of the raw signal is typically expressed in a form that provides the user with meaningful information, and in which form users become familiar with analyzing, such as blood glucose expressed in mg/dL.

SUMMARY

The present embodiments have several features, no single one of which is solely responsible for their desirable attributes. Without limiting the scope of the present embodiments as expressed by the claims that follow, their more prominent features now will be discussed briefly. After considering this discussion, and particularly after reading the section entitled "Detailed Description," one will understand how the features of the present embodiments provide the advantages described herein.

Systems and methods are disclosed that employ numerous variables or parameters in the determination and/or calculation of a glycemic urgency index (GUI), which may be based in part on a measured blood glucose level and which generally includes consideration of other factors. The other factors may include first and/or second derivatives of the blood glucose level with respect to time, and/or other factors as will be described, e.g., user - entered data, data measured by other sensors or received from a network source, or historical data. The GUI is then presented to the user in an interesting way, e.g., via a background color or other inconspicuous notifier, e.g., on a mobile device such as a smart phone. In this way, the GUI may be continuously presented to the user (whenever the display screen is on or otherwise activated). The GUI may also be employed in the triggering of actionable alerts and alarms (or other outputs) on an electronic device for the user. The GUI, or another index calculated from combinations of the variables and parameters described, may also be employed to drive a medicament delivery device such as a pump. Generally, a given GUI will generally cause the same notification (or actionable alert) for a given user, although variations in the notifier or alert will be seen for the user, depending on current sensitivity, how the user has configured their electronic device, the mode the user has enabled for the device, and so on.

In more detail, a first type of data may be received that is associated with a physiological condition. For example, a glucose concentration may be measured that bears a functional relationship with the GUI of a patient with diabetes. A second type of data may then be received, or in some cases calculated, e.g., a time rate of change of the first type of data. In the case of diabetes management, the second type of data may be a rate of change of the glucose concentration, i.e., whether the concentration is going up or down and how quickly. The second type of data may also be an acceleration of the glucose concentration, e.g., which may indicate a turnaround in the glucose concentration. Besides time rates of change, the second type of data may also include: pattern data, deviations from normal glucose patterns, predicted glucose values, durations over which glucose values (or time rates of change thereof) are within a predetermined range, local maxima or minima, or the like. As with the first type of data, the second type of data bears a functional relationship with the GUI. For example, while in some cases the second type of data may be derived or calculated from the first type of data, it is an independent variable that bears on the determination of a dependent variable, i.e., the GUI.

A third type of data may also be received, these corresponding to other factors besides the analyte concentration and parameters derived therefrom. For example, data entered by a user may be a third type of data, e.g., data corresponding to health, exercise, meal data, medicament injection data, or numerous other inputs. In some cases, the third type of data may be received from another device, e.g., a GPS or exercise application running on a mobile device may indicate exercise performed by a user. Other applications may be employed to monitor meal intake and the like. Automatic medicament delivery devices may be interfaced with the system as well, e.g., to indicate insulin pump actions for consideration in the GUI determination. In the same way, the GUI can influence pump actions. As with the first and second types of data, the third type of data bears a functional relationship with the GUI. In particular, the third type of data is generally an independent variable that bears on the determination of the dependent variable, i.e., the GUI (although it is noted that the third type of data may be characterized by parameters and variables that themselves may have some interrelationship).

An indication of the GUI may be presented to the user on a user interface, e.g., on a mobile device such as a smart phone. The GUI may be indicated in such a way that it is a naturally appearing feature, e.g., a background color or design, that need not overtly reveal itself as an indicator of user health. If the user is satisfied with the indication, the interaction may end there. If the user desires additional data, the user may perform an action such as an unlocking action, swiping action, or other application action in order to retrieve additional details, e.g., about how urgent the situation is, relevant measurements and parameters indicating the situation, potential steps to take to correct the situation, and the like. Where the GUI is indicated by a design, the design features may graphically (but in many cases not numerically) indicate a current value, e.g., glucose concentration, a direction in which the value is changing, whether the value is turning around, recent past values, and the like. The user interface may enable a user to input parameters and variables as well, which may then bear on the GUI determination.

The monitoring device, e.g., a CGM, may be embodied as an application running on a mobile device, e.g., a smart phone, and downloadable thereto. The application may run in particular an urgency assessment module, which may perform a sophisticated assessment of the urgency of a user's glycemic state, generally with "higher" assessments associated with "higher" urgency or "higher" risk states. The mobile device may present a continuous notification or presentation of a GUI indication, and may also provide alerts or alarms when warranted.

In a first aspect, the invention is directed towards a method of assessing a user's urgency state associated with a physiological condition, including: receiving data of a first type associated with a physiological condition; calculating data of a second type associated with the physiological condition; receiving data of a third type associated with the physiological condition; determining an urgency index based at least on the received data of the first, second, and third types; and providing an indication of the determined urgency index on a mobile device.

The data of the first type may be analyte, e.g. glucose, data from a continuous analyte sensor implanted in the body. The second data may be a first order and/or second order time derivative of the first data. The third data may be data external of analyte data from the sensor, but which represents a factor affecting a health risk of the physiological condition reflected by the analyte in the body, e.g. a factor affecting a risk of an extreme hyperglycemic or hypoglycemic state, which states are reflected by glucose data sensed by the continuous analyte data. In particular, the third data may represent a factor affecting analyte levels reflecting the physiological condition, thereby affecting the health risk.

The mobile device may be a smartphone.

The method may comprise, outputting an audible and/or tactile alert on the mobile device and/or overriding other applications or processes operating on the mobile device, such that the indication of the urgency index is displayed on the mobile device regardless of other running applications or processes, when the urgency index reaches or exceeds a threshold indicative of the physiological condition reaching a health risk state, e.g. indicative of risk of an extreme hypoglycemic or hyperglycemic state, unless mediating action is taken by the user.

The method may comprise providing the indication on the mobile device so that the indication is visible to a user even before a lock screen of the mobile device has been passed, at least when the urgency index indicates a high urgency with respect to the physiological condition.

Determination of the urgency index may be performed by an urgency assessment module.

The urgency assessment module may take as an input analyte data representative of the physiological condition, e.g. glucose data representative of a diabetic condition, as the first type of data, wherein as the data approaches a health risk of the physiological condition, e.g. an extreme hyperglycemic risk or an extreme hypoglycemic risk, the analyte data input tends to adjust the urgency index to a value representative of greater urgency.

The urgency assessment module may take as an input first order and/or second order time derivative data as the second type of data that tends to adjust the urgency index to a value representative of lesser urgency when the first and/or second derivate data indicates that the health risk, e.g. risk of extreme hyperglycemia or hypoglycemia, is moderating or greater urgency when the first and/or second derivate data indicates that the health risk, e.g. risk of hyperglycemia or hypoglycemia, is worsening.

The urgency assessment module may take as an input external data relating to user or other action that will, in the future, affect the analyte data as the third type of data, the input external data tending to adjust the urgency index to a value representative of lesser risk when the effect on the analyte data of the user or other action will moderate analyte levels with respect to the health risk and the input external data tending to adjust the urgency index to a value representative of greater risk when the effect on the analyte data of the user or other action will worsen analyte levels with respect to the health risk. In an embodiment, the external data is representative of time of insulin injection in the body (whether through a body internal pump or a body external injector) and/or representative of time of food/drink ingestion and/or representative of time of a user performing exercise, wherein the health risk is risk of extreme hyperglycemia or hypoglycemia and the analyte is blood glucose.

The urgency assessment module may take as an input duration data as the second data, the duration data representing a time that the analyte data is outside of a normal range defined for the physiological condition, e.g. the duration that analyte data is continuously representative of a hyperglycemic or hypoglycemic state, the duration data tending to adjust the urgency index so that a greater urgency is determined as the duration increases.

The step of determining the urgency index may comprise utilizing a mathematical risk function having terms for each of analyte data, first order time derivative of analyte data and second order time derivative of analyte data and/or duration over which analyte data is outside of a normal range with respect to the physiological condition, wherein the data forming said terms constitute the first and second types of data.

Implementations of the first aspect may include one or more of the following. The physiological condition may be diabetes, the urgency index may be a glycemic urgency index, and the data of the first type may be a glucose concentration. The glucose concentration may be a current measured glucose concentration, a past measured glucose concentration, or a future predicted glucose concentration.

The data of the second type may be derived from the data of the first type, such as a first derivative or a second derivative with respect to time. The data of the second type derived from the data of the first type may further include: deviations from normal glucose patterns, pattern data of glucose values over a time period, predicted glucose values, a duration over which a glucose value is within a predetermined range, weightings of parameters or variables to be considered in the determining of the urgency index, or local maxima or minima in the data of the first type.

The receiving data of a third type may include receiving data entered by a user, such as on a user interface of a mobile device.

Where the physiological condition is diabetes, the urgency index may be a glycemic urgency index, and the data of the first type may be a glucose concentration, and the received data entered by a user may include a user weight, a user indication of activity level, a user indication of food or drink ingested or to be ingested, anthropometric data, data about prior insulin provided to the user, stress data, health data, data about a placement of the sensor measuring the data of the first type, age, or gender. The received data entered by a user may also include a user indication of food or drink ingested or to be ingested, or data about prior insulin provided to the user or to be provided to the user, and may further include modifying the determined glycemic urgency index to be a lower urgency based on the received data.

The receiving data of a third type may include receiving data from a sensor. Where the physiological condition is diabetes, the urgency index may be a glycemic urgency index and the data of the first type may be a glucose concentration, and the sensor may include at least one of the following: a scale, a glucometer, a thermometer, an accelerometer, a camera, a GPS device, or a microphone. The receiving data of a third type may also include: a user weight, a user indication of activity level, an indication of food or drink ingested or to be ingested, anthropometric data, data about prior insulin provided to the user, physiological data, stress data, or health data. The receiving data of a third type may also include receiving data from a query processing engine, an electronic device configured for machine to machine communication, or an electronic user record. The data of the third type may be received from the mobile device, and may correspond to a level of user interaction with an application through which the indication was provided.

The method may further include providing an alert or alarm if the glycemic urgency index reaches a predetermined alerting or alarming threshold, respectively. Such a predetermined alerting or alarming threshold may indicate that the user is in a hypoglycemic or hyperglycemic state.

Besides diabetes, the physiological condition may also include one or more of obesity, malnutrition, hyperactivity, depression, or fertility.

The method may further include providing an advanced output based on the received inputs.

The step of providing the indication may include displaying an indication of the urgency index on a user interface of the mobile device. The displaying step may further include overriding other applications or processes operating on the mobile device, such that the indication of the urgency index is displayed regardless of other running applications or processes.

Where the displaying step is caused by a user action, the user action may be selected from the group consisting of: handling the mobile device, unlocking the mobile device, or performing a swiping action on the mobile device.

The indication of the urgency index may be a rendered element, e.g., a color, where the rendered element is rendered as at least a portion of a home screen or a background, native to an operating system of the mobile device. Where the rendered element is color, the color is different depending upon the urgency index. The rendered element may also be an icon, where a position, size, or color of the icon is based on the urgency index.

The method may further include receiving an indication that a user has activated the icon; and displaying additional information or an advanced output about the urgency index.

The third type of data may comprise a past or future medicament parameter entered by a user or received from an integrated pump, the medicament parameter representative of time and/or amount of medicament injected in the user to address the physiological condition. Where the analyte is glucose, the urgency index may be a glycemic urgency index, and the medicament parameter may correspond to insulin.

The step of providing the indication may further include rendering features as follows. For example, a series of elements may be rendered on the user interface of the mobile device, the series of elements indicating past or predicted future values of glucose concentration. The indication of the urgency index may be an audible or visual alert rendered or sounded, respectively, on the mobile device. The method may further include displaying a prompt for a user to enter data, such that user data may be associated with the urgency index. The prompt for a user to enter data may indicate a type of data, and the type of data may be selected from the group consisting of: exercise or activity level, meal data, insulin data, stress or health data, or emotional data, which may be utilized to form the third data. The method may further include displaying at least one potential action a user may perform in response to the displayed indication of the urgency index. The indication may be an actionable alert. The displaying an indication may include displaying occupancy of a band, the band corresponding to a predetermined range of urgency index values.

The method may further include storing the determined urgency index in a storage of a mobile device. The method may include transmitting the stored urgency index to an integrated pump. For example, the method may further include transforming the stored urgency index to a pump action, and transmitting the pump action to an integrated pump.

The method may comprise displaying a current analyte data value indicative of the physiological condition and/or displaying an indication of whether a trend of analyte date is increasing or decreasing and optionally also displaying an indicator of the rate of increase or decrease of the trend. In one implementation, the trend is displayed by a generally upwardly directed arrow indicating increasing trend and a generally downwardly directed arrow indicating decreasing trend and optionally the angle of the arrow is indicative of the rate, with more vertical indicating greater rate.

The indication of the urgency index may be provided by changing color depending on the degree of urgency. Red may be chosen to indicate a highest urgency state.

In a second aspect, the invention is directed towards a system for performing any of the above methods of the first aspect.

In a third aspect, the invention is directed towards a method of determining an urgency index associated with a physiological condition, including: determining the urgency index based on an analyte concentration and at least two variables selected from the group consisting of: a first or second time derivative of the analyte concentration, a duration the analyte concentration has occupied a predetermined range, a duration the first or second time derivative of the analyte concentration has occupied a predetermined range, a second time derivative of the analyte concentration, a duration the second time derivative of the analyte concentration has occupied a predetermined range, a past or future meal intake parameter entered by a user, a past or future medicament parameter entered by a user or received from an integrated pump, or a body temperature.

The method may comprise storing the determined urgency index in a storage of a mobile device.

The urgency index may be representative of an urgency with which intervening action is required in order to bring analyte concentration from a higher health risk level to a lower health risk level with respect to the physiological condition.

Implementations of the second aspect may include one or more of the following. The method may further include displaying an indication of the urgency index on a user interface of a mobile device. The displaying step may further include overriding other applications or processes operating on the mobile device, such that the indication of the urgency index is displayed regardless of other running applications or processes.

Where the displaying step is caused by a user action, the user action may be selected from the group consisting of: handling the mobile device, unlocking the mobile device, or performing a swiping action on the mobile device.

The indication of the urgency index may be a rendered element, e.g., a color, where the color is rendered as at least a portion of a home screen or a background, native to an operating system of the mobile device. The rendered element may also be an icon, where a position, size, or color of the icon is based on the urgency index.

The method may further include receiving an indication that a user has activated the icon; and displaying additional information or an advanced output about the urgency index.

Where the analyte is glucose, the urgency index may be a glycemic urgency index, and the medicament parameter may correspond to insulin.

The method may include rendering features as follows. For example, a series of elements may be rendered on the user interface of the mobile device, the series of elements indicating past or predicted future values of glucose concentration.

An indication of the urgency index may be provided and may be an audible or visual alert rendered or sounded, respectively, on a mobile device.

The method may further include displaying a prompt for a user to enter data, such that user data may be associated with the urgency index. The prompt for a user to enter data may indicate a type of data, and the type of data may be selected from the group consisting of: exercise or activity level, meal data, insulin data, stress or health data, or emotional data. The method may further include displaying at least one potential action a user may perform in response to the displayed indication of the urgency index. The indication may be an actionable alert. The action may be one that mediates a health risk when the urgency risk is high to bring the analyte concentration toward a normal or more acceptable level in terms of the health risk presented by the physiological condition. The displaying an indication may include displaying occupancy of a band, the band corresponding to a predetermined range of urgency index values.

The method may further include transmitting the stored urgency index to an integrated pump. For example, the method may further include transforming the stored urgency index to a pump action, and transmitting the pump action to an integrated pump. The integrated pump discussed in this paragraph and in the foregoing may be a pump for delivering a medicament for treating the physiological condition.

The method may include providing an indication of the determined urgency index on a mobile device.

The at least two variables may include the first order and/or the second order time derivative of the analyte concentration.

The mobile device may be a smartphone.

The method may comprise, outputting an audible and/or tactile alert on a mobile device and/or overriding other applications or processes operating on the mobile device, such that an indication of the urgency index is displayed on the mobile device regardless of other running applications or processes, when the determined urgency index reaches or exceeds a threshold indicative of the physiological condition reaching a health risk state, e.g. indicative of risk of an extreme hypoglycemic or hyperglycemic state, unless mediating action is taken by the user.

The method may comprise providing an indication of the urgency risk on a mobile device so that the indication is visible to a user even before a lock screen of the mobile device has been passed, at least when the urgency index indicates a high urgency with respect to the physiological condition.

Determination of the urgency index may be performed by an urgency assessment module.

The urgency assessment module may take as an input the analyte concentration, wherein as the data approaches a health risk of the physiological condition, e.g. an extreme hyperglycemic risk or an extreme hypoglycemic risk, the analyte concentration input tends to adjust the urgency index to a value representative of greater urgency.

The urgency assessment module may take as an input the first order and/or second order time derivative in a way that tends to adjust the urgency index to a value representative of lesser urgency when the first and/or second time derivate indicates that a health risk, e.g. risk of extreme hyperglycemia or hypoglycemia, of the physiological condition is moderating or greater urgency when the first and/or second derivate indicates that the health risk, e.g. risk of hyperglycemia or hypoglycemia, of the physiological condition is worsening.

The urgency assessment module may take as an input external data including the past or future meal intake parameter entered by a user or the past or future medicament parameter entered by a user or received from an integrated pump, wherein the input external data tends to adjust the urgency index to a value representative of lesser risk when the effect on the analyte concentration of the medicament or the meal intake will moderate analyte levels with respect to a health risk of the physiological condition and wherein the input external data tends to adjust the urgency index to a value representative of greater risk when the effect on the analyte concentration of the medicament or the meal intake will worsen analyte levels with respect to the health risk. In an embodiment, the medicament parameter is representative of time and/or amount of insulin injection in the body (whether through a body internal pump or a body external injector) and the meal intake parameter is representative of time and/or amount of food/drink ingestion.

The at least two variables may include the first and the second time derivative data and at least one of the meal intake parameter and the medicament parameter.

The urgency assessment module may additionally or alternatively take as an input the an indicator of duration that the analyte concentration has occupied the predetermined range representing a normal range defined for the physiological condition, e.g. the duration that analyte data is continuously representative of a euglycemic state, the duration indicator tending to adjust the urgency index so that a greater urgency is determined as the duration increases.

The step of determining the urgency index may comprise utilizing a mathematical risk function having terms representing each of the analyte concentration, the first order time derivative of the analyte concentration and the second order time derivative of the analyte concentration and/or the duration the analyte concentration has occupied the predetermined range.

Implementations of the third aspect may further include one or more of the following. The physiological condition may be diabetes, the urgency index may be a glycemic urgency index, and the analyte concentration may be glucose concentration. The glucose concentration may be a current measured glucose concentration.

The method may include receiving the meal intake parameter and/or the medicament parameter based on corresponding data entered by a user, such as on a user interface of a mobile device.

The method may further include providing an alert or alarm if the glycemic urgency index reaches a predetermined alerting or alarming threshold, respectively. Such a predetermined alerting or alarming threshold may indicate that the user is in a hypoglycemic or hyperglycemic state.

The method may comprise displaying a numerical value representing the analyte concentration such as in mg/ml and/or displaying an indication of whether a trend of analyte concentration is increasing or decreasing and optionally also displaying an indicator of the rate of increase or decrease of the trend. In one implementation, the trend is displayed by a generally upwardly directed arrow indicating increasing trend and a generally downwardly directed arrow indicating decreasing trend and optionally the angle of the arrow is indicative of the rate, with more vertical indicating greater rate. The indication of the urgency index may be provided by changing color of a display depending on the degree of urgency. Red may be chosen to indicate a highest urgency state. The display may be on a mobile device such as a smart phone.

In a fourth aspect, the invention is directed towards a system for performing any of the above methods of the third aspect.

In a fifth aspect, the invention is directed towards a device, system, or method substantially as shown and/or described in the specification and/or drawings.

In a sixth aspect, the invention is directed towards an electronic device for monitoring data associated with a physiological condition, including: a continuous analyte sensor, where the continuous analyte sensor is configured to substantially continuously measure the concentration of analyte in the host, and to provide continuous sensor data associated with the analyte concentration in the host; and a processor module configured to perform any one of the above-noted methods.

In a seventh aspect, the invention is directed towards an electronic device for delivering a medicament to a host, including: a medicament delivery device configured to deliver medicament to the host, where the medicament delivery device is operably connected to a continuous analyte sensor, where the continuous analyte sensor is configured to substantially continuously measure the concentration of analyte in the host, and to provide continuous sensor data associated with the analyte concentration in the host; and a processor module configured to perform any one of the above-noted methods.

To ease the understanding of the described features, continuous glucose monitoring is used as part of the explanations that follow. It will be appreciated that the systems and methods described are applicable to other continuous monitoring systems. For example, the features discussed may be used for continuous monitoring of lactate, free fatty acids, heart rate during exercise, IgG-anti gliadin, insulin, glucagon, movement tracking, fertility, caloric intake, hydration, salinity, sweat/perspiration (stress), ketones, adipanectin, troponin, perspiration, and/or body temperature. Where glucose monitoring is used as an example, one or more of these alternate examples of monitoring conditions may be substituted. Thus, while a GUI has been described above, in an analogous system, a lactose urgency index, a ketone urgency index, etc., may be defined.

Any of the features of embodiments of the various aspects disclosed is applicable to all aspects and embodiments identified. Moreover, any of the features of an embodiment is independently combinable, partly or wholly with other embodiments described herein, in any way, e.g., one, two, or three or more embodiments may be combinable in whole or in part. Further, any of the features of an embodiment of the various aspects may be made optional to other aspects or embodiments. Any aspect or embodiment of a method can be performed by a system or apparatus of another aspect or embodiment, and any aspect or embodiment of the system can be configured to perform a method of another aspect or embodiment.

Advantages of the systems and methods according to present principles may include one or more of the following. Users may receive an indication of their urgency assessment whenever they glance at their phone. In the same way, users may receive more actionable alerts, decreasing the occurrence of nuisance alerts and increasing usage of CGMs, by the use of intelligent algorithms that consider numerous inputs in the determination of the glycemic urgency state. Because the urgency assessment is based on inputs that have not previously been considered by alerting algorithms, the glycemic urgency index may correlate better with a patients' clinical diabetes management, rather than necessarily correlating with glucose concentration (or derivative) information in isolation. Users may be alerted to glycemic urgency states safely and discreetly, in interesting and customizable ways, and in ways that utilize the native user interface of devices users likely already carry with them, e.g., mobile devices. Other advantages will be apparent from the description that follows, including the figures and claims.

BRIEF DESCRIPTION OF THE DRAWINGS

The present embodiments now will be discussed in detail with an emphasis on highlighting the advantageous features. These embodiments depict the novel and non-obvious urgency assessment and user interfaces shown in the accompanying drawings, which are for illustrative purposes only. These drawings include the following figures, in which like numerals indicate like parts:

Figure 1 is a graph of a CGM trace, illustrating the effect of setting a low threshold at a lower or higher value;

Figure 2 is a graph of a CGM trace, illustrating that an alert can be triggered even at a relatively stable glucose value;

Figure 3 is a block diagram of an integrated system of the preferred embodiments, including a continuous glucose sensor and a medicament delivery device;

Figure 4 is a front elevation view of an electronic device configured for use with the present systems and methods;

Figure 5 is a functional block diagram of the electronic device of Figure 4;

Figure 6 depicts a logical diagram of certain components of the system of Figure 3;

Figure 7 depicts categories of parameters or variables which may be employed in the calculation of the GUI;

Figure 8 is a flowchart illustrating a method according to present principles;

Figure 9 is a graph showing how various combinations of parameters and variables can be employed in the determination of a GUI;

Figure 10 is another graph showing how various combinations of parameters and variables can be employed in the determination of a GUI, in particular using patterns;

Figure 11 is another graph showing how various combinations of parameters and variables can be employed in the determination of a GUI, in particular using data about significant events;

Figures 12A and 12B illustrate glucose concentration, static risk, and dynamic risk, as a function of time;

Figure 13 illustrates how glycemic urgency increases with duration in hypoglycemia;

Figure 14 illustrates how use of acceleration as a parameter or variable in a GUI determination can avoid false alerts about risk states;

Figures 15A-15F illustrate various probability distributions corresponding to exemplary parameters and variables.

Figures 16 - 29 illustrate various user interfaces which may be employed to display GUI notifications and/or actionable alerts based on the calculated GUIs;

Figure 30 is a flowchart of an exemplary method for providing GUI notifications and/or actionable alerts;

Figures 31A-31F are exemplary user interfaces in which a user prompt is invited; and

Figure 32A is a flowchart of another exemplary method, such implementing a retrospective algorithm. Figures 32B and 32C are graphs illustrating the use of the method of Figure 32A.

DETAILED DESCRIPTION

Consider the specific example of continuous glucose monitoring. For diabetics, the glucose monitor can literally be the difference between life and death. However, the value of blood glucose presented on a CGM can be ambiguous. For example, three users may all have the same value of blood glucose as measured by a CGM, but each may require different treatment depending on whether the value of blood glucose is decreasing, staying the same, or increasing. This is particularly true as current CGMs trigger alerts based on low and/or high thresholds, e.g., predicted or actual glucose concentration thresholds, sometimes including a consideration of rate of change. Predicted values are generally highly subject to noise, and the use of such threshold-based alerting often does not provide a user with sufficient time to react before a risky or dangerous situation is encountered.

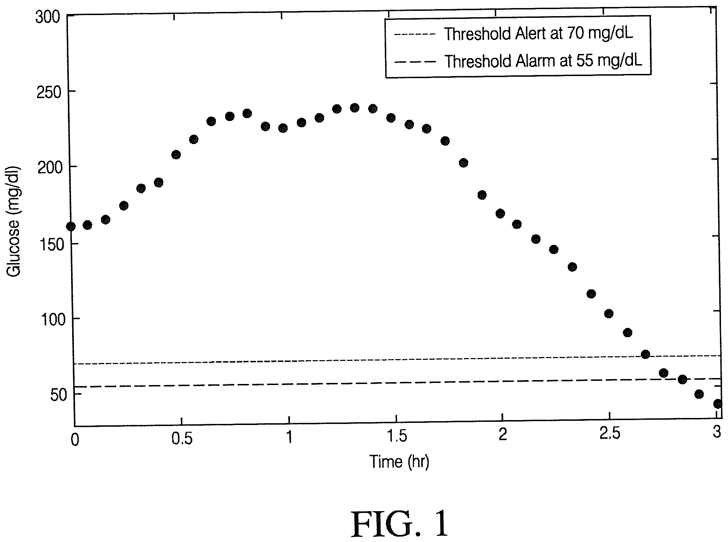

For example, and referring to Figure 1, a standard hypoglycemia threshold alert may be set at 70 mg/dL. If the user's glucose level is dropping quickly, such a low threshold alert may not be able to provide the user with enough time to prevent a very low glucose level, e.g., below 55 mg/dL. Even if the low threshold alert was set at 80 mg/dL, the user would only hear an alert 10 minutes prior to dropping below 55 mg/dL. During the half-hour prior to dropping below 55 mg/dL, the user was dropping at an average rate of 2.5 mg/dL/min, and it is clear from the CGM trace on Figure 1 that the user was in a very risky situation well before hitting the 70 mg/dL threshold.

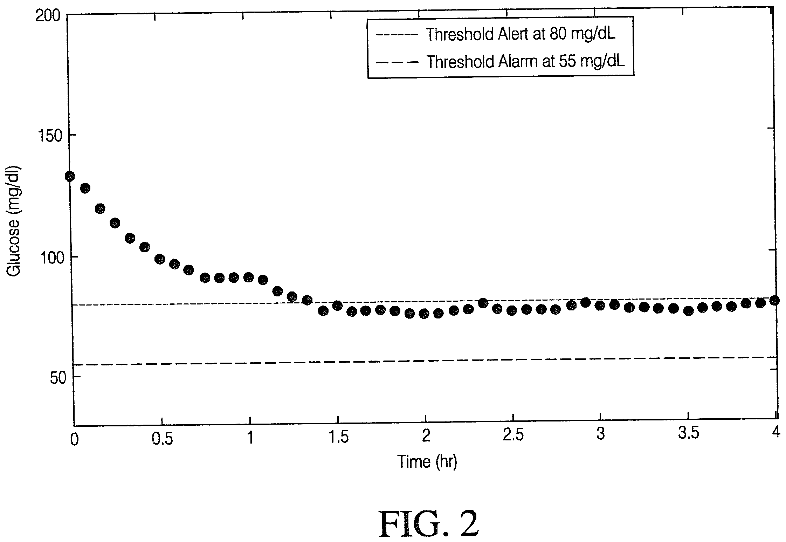

While it is always possible to increase the sensitivity of the sensor, such increases often lead to false alerts and accompanying user "alert fatigue". Such is especially true where the monitor is a smart phone, which usually is already alerting the user in multiple ways, e.g., application notifications, text messages, e-mails, and the like. For example, in an effort to ensure there is enough time to prevent a very low glucose level, if the low threshold is set higher, e.g., at 80 or 90 mg/dL, such will lead to many false alerts. As another example, and referring to Figure 2, there is no need to alert for the stable glucose level hovering around 80 mg/dL, but if the threshold was set at 80 mg/dL, many alerts would be triggered.

In addition, while current systems can present the value of blood glucose to the user, as well as a threshold - based alert, user interfaces associated with such systems do not meet the users' expectations. Both the lack of reliable alerting and the lack of safe and discreet user interfaces hinder usage and adoption of such monitors.

Similarly, current systems that integrate insulin pump actions with CGM use a simple glucose threshold to make decisions such as suspending basal insulin. However, a simple glucose threshold may not provide enough information about a user's urgency state. For example, using a threshold of 70 mg/dL to suspend insulin delivery may be appropriate when glucose is falling gradually, but if glucose is dropping rapidly, then it may be more appropriate to suspend insulin when glucose is at 100 mg/dL, and possibly even earlier if there is a large amount of insulin on board or recent exercise. Even suspending on a predicted glucose value alone has the disadvantage of a large number of false positives.

Other aspects relating to the measurement of blood glucose and providing alerts about the same are described in co-pending US Non-Provisional Patent Application Serial Number 13/742,694, filed January 16, 2013, entitled "SYSTEMS AND METHODS FOR PROVIDING SENSITIVE AND SPECIFIC ALARMS, " owned by the assignee of the present application and herein incorporated by reference in its entirety.

One non-limiting advantage of the features described herein is to provide alerts and alarms which are more useful to the user, i.e., more "actionable", in the sense that users become aware of or can deduce easily an appropriate action to take given the alert or alarm. Such are also more accurate in the sense of more correctly reflecting a current glycemic urgency assessment for the user. Besides providing actionable alerts and/or alarms, the same may provide a continuous notification to the user of their urgency assessment and may be presented to the user in a highly interesting way, using the native user interface of a device the user already generally carries, e.g., a mobile device such as a smart phone, thus negating the need for the user to carry an additional device.

Various terms are described below.

The phrase "continuous glucose sensor" as used herein is a broad phrase, and is to be given its ordinary and customary meaning to a person of ordinary skill in the art (and is not to be limited to a special or customized meaning), and refers without limitation to a device that continuously or continually measures the glucose concentration of a bodily fluid (e.g., blood, plasma, interstitial fluid and the like), for example, at time intervals ranging from fractions of a second up to, for example, 1, 2, or 5 minutes, or longer.

The phrases "continuous glucose sensing" or "continuous glucose monitoring" as used herein are broad terms, and are to be given their ordinary and customary meaning to a person of ordinary skill in the art (and is not to be limited to a special or customized meaning), and refer without limitation to the period in which monitoring of the glucose concentration of a host's bodily fluid (e.g., blood, serum, plasma, extracellular fluid, tears etc.) is continuously or continually performed, for example, at time intervals ranging from fractions of a second up to, for example, 1, 2, or 5 minutes, or longer. In one exemplary embodiment, the glucose concentration of a host's extracellular fluid is measured every 1, 2, 5, 10, 20, 30, 40, 50 or 60 seconds.

The term "substantially" as used herein is a broad term, and is to be given its ordinary and customary meaning to a person of ordinary skill in the art (and is not to be limited to a special or customized meaning), and refers without limitation to being largely but not necessarily wholly that which is specified, which may include an amount greater than 50 percent, an amount greater than 60 percent, an amount greater than 70 percent, an amount greater than 80 percent, an amount greater than 90 percent, or more.

The terms "processor" and "processor module," as used herein are a broad terms, and are to be given their ordinary and customary meaning to a person of ordinary skill in the art (and are not to be limited to a special or customized meaning), and refer without limitation to a computer system, state machine, processor, or the like, designed to perform arithmetic or logic operations using logic circuitry that responds to and processes the basic instructions that drive a computer. In some embodiments, the terms can include ROM and/or RAM associated therewith.

Exemplary embodiments disclosed herein relate to the use of a glucose sensor that measures a concentration of glucose or a substance indicative of the concentration or presence of another analyte. In some embodiments, the glucose sensor is a continuous device, for example a subcutaneous, transdermal, transcutaneous, non-invasive, intraocular and/or intravascular (e.g., intravenous) device. In some embodiments, the device can analyze a plurality of intermittent blood samples. The glucose sensor can use any method of glucose measurement, including enzymatic, chemical, physical, electrochemical, optical, optochemical, fluorescence-based, spectrophotometric, spectroscopic (e.g., optical absorption spectroscopy, Raman spectroscopy, etc.), polarimetric, calorimetric, iontophoretic, radiometric, and the like.

The glucose sensor can use any known detection method, including invasive, minimally invasive, and non-invasive sensing techniques, to provide a data stream indicative of the concentration of the analyte in a host. The data stream is typically a raw data signal that is used to provide a useful value of the analyte to a user, such as a patient or health care professional (e.g., doctor), who may be using the sensor.

Although much of the description and examples are drawn to a glucose sensor capable of measuring the concentration of glucose in a host, the systems and methods of embodiments can be applied to any measurable analyte. Some exemplary embodiments described below utilize an implantable glucose sensor. However, it should be understood that the devices and methods described herein can be applied to any device capable of detecting a concentration of analyte and providing an output signal that represents the concentration of the anal yte.

As noted, in some embodiments, the analyte sensor is an implantable glucose sensor, such as described with reference to U.S. Patent 6,001,067 and U.S. Patent Publication No. US-2011-0027127-A1. In some embodiments, the analyte sensor is a transcutaneous glucose sensor, such as described with reference to U.S. Patent Publication No. US-2006-0020187-A1. In yet other embodiments, the analyte sensor is a dual electrode analyte sensor, such as described with reference to U.S. Patent Publication No. US-2009-0137887-A1. In still other embodiments, the sensor is configured to be implanted in a host vessel or extracorporeally, such as is described in U.S. Patent Publication No. US-2007-0027385-A1. These patents and publications are incorporated herein by reference in their entirety.

The following description and examples described the present embodiments with reference to the drawings. In the drawings, reference numbers label elements of the present embodiments. These reference numbers are reproduced below in connection with the discussion of the corresponding drawing features.

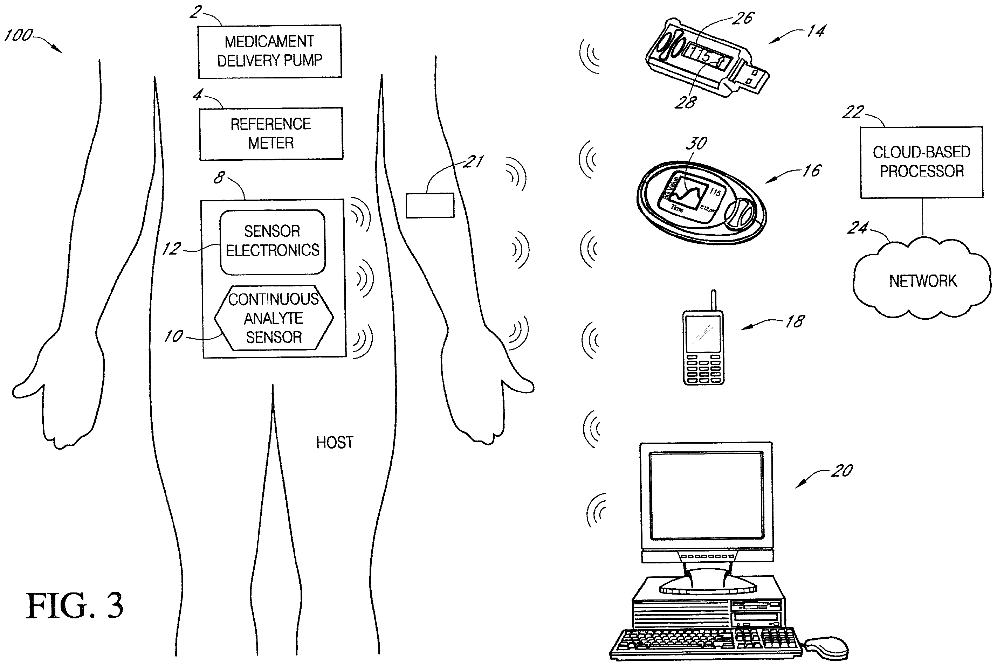

Figure 3 is a block diagram of an integrated system of the preferred embodiments, including a continuous glucose sensor and a medicament delivery device. Such is an exemplary environment in which some embodiments described herein may be implemented. Here, an analyte monitoring system 100 includes a continuous analyte sensor system 8. Continuous analyte sensor system 8 includes a sensor electronics module 12 and a continuous analyte sensor 10. The system 100 can also include other devices and/or sensors, such as a medicament delivery pump 2 and a reference analyte meter 4. The continuous analyte sensor 10 may be physically connected to sensor electronics module 12 and may be integral with (e.g., non-releasably attached to) or releasably attachable to the continuous analyte sensor 10. Alternatively, the continuous analyte sensor 10 may be physically separate from sensor electronics module 12, but electronically coupled via inductive coupling or the like. Further, the sensor electronics module 12, medicament delivery pump 2, and/or analyte reference meter 4, may communicate with one or more additional devices, such as any or all of display devices 14, 16, 18, and/or 20. The display devices 14, 16, 18, and 20 generally include a processor, memory, storage, and other components sufficient to run an application including an urgency assessment module.

In some implementations, the system 100 of Figure 3 may also include a cloud-based processor 22 configured to analyze analyte data, medicament delivery data and/or other user related data provided over network 24 directly or indirectly from one or more of sensor system 8, medicament delivery pump 2, reference analyte meter 4, and display devices 14, 16, 18, 20. Based on the received data, the processor 22 can further process the data, generate reports providing statistics based on the processed data, trigger notifications to electronic devices associated with the host or caretaker of the host, or provide processed information to any of the other devices of Figure 3. In some exemplary implementations, the cloud-based processor 22 comprises one or more servers. If the cloud-based processor 22 comprises multiple servers, the servers can be either geographically local or separate from one another. The network 24 can include any wired and wireless communication medium to transmit data, including WiFi networks, cellular networks, the Internet and any combinations thereof.

In some exemplary implementations, the sensor electronics module 12 may include electronic circuitry associated with measuring and processing data generated by the continuous analyte sensor 10. This generated continuous analyte sensor data may also include algorithms, which can be used to process and calibrate the continuous analyte sensor data, although these algorithms may be provided in other ways as well, such as by the devices 14, 16, 18, and/or 20. The sensor electronics module 12 may include hardware, firmware, software, or a combination thereof, to provide measurement of levels of the analyte via a continuous analyte sensor, such as a continuous glucose sensor.

The sensor electronics module 12 may, as noted, couple (e.g., wirelessly and the like) with one or more devices, such as any or all of display devices 14, 16, 18, and 20. The display devices 14, 16, 18, and/or 20 may be configured for processing and presenting information, such sensor information transmitted by the sensor electronics module 12 for display at the display device. The display devices 14, 16, 18, and 20 can also trigger alarms based on the analyte sensor data.

In Figure 3, display device 14 is a key fob-like display device, display device 16 is a hand-held application-specific computing device 16 (e.g., the DexCom G4® Platinum receiver commercially available from DexCom, Inc.), display device 18 is a general purpose smart phone or tablet computing device 20 (e.g., a phone running the Android® OS, an Apple® iPhone®, iPad®, or iPod touch® commercially available from Apple, Inc.), and display device 20 is a computer workstation 20. In some exemplary implementations, the relatively small, key fob-like display device 14 may be a computing device embodied in a wrist watch, a belt, a necklace, a pendent, a piece of jewelry, an adhesive patch, a pager, a key fob, a plastic card (e.g., credit card), an identification (ID) card, and/or the like. This small display device 14 may include a relatively small display (e.g., smaller than the display device 18) and may be configured to display a limited set of displayable sensor information, such as a numerical value 26 and an arrow 28. Some systems may also include a wearable device 21, such as described in U.S. Provisional Patent Application No. 61/904,341, filed November 14, 2013, and entitled "Devices and Methods for Continuous Analyte Monitoring," the entire disclosure of which is hereby expressly incorporated by reference. The wearable device 21 may include any device(s) that is/are worn on, or integrated into, a user's vision, clothes, and/or bodies. Example devices include wearable devices, anklets, glasses, rings, necklaces, arm bands, pendants, belt clips, hair clips/ties, pins, cufflinks, tattoos, stickers, socks, sleeves, gloves, garments (e.g. shirts, pants, underwear, bra, etc.), "clothing jewelry" such as zipper pulls, buttons, watches, shoes, contact lenses, subcutaneous implants, eyeglasses, cochlear implants, shoe inserts, braces (mouth), braces (body), medical wrappings, sports bands (wrist band, headband), hats, bandages, hair weaves, nail polish, artificial joints/body parts, orthopedic pins/devices, implantable cardiac or neurological devices, etc. The small display device 14 and/or the wearable device 21 may include a relatively small display (e.g., smaller than the display device 18) and may be configured to display graphical and/or numerical representations of sensor information, such as a numerical value 26 and/or an arrow 28. In contrast, display devices 16, 18 and 20 can be larger display devices that can be capable of displaying a larger set of displayable information, such as a trend graph 30 depicted on the hand-held receiver 16 in addition to other information such as a numerical value and arrow.

It is understood that any other user equipment (e.g., computing devices) configured to at least present information (e.g., a medicament delivery information, discrete self-monitoring analyte readings, heart rate monitor, caloric intake monitor, and the like) can be used in addition to or instead of those discussed with reference to Figure 3.

In some exemplary implementations of Figure 3, the continuous analyte sensor 10 comprises a sensor for detecting and/or measuring analytes, and the continuous analyte sensor 10 may be configured to continuously detect and/or measure analytes as a non-invasive device, a subcutaneous device, a transdermal device, and/or an intravascular device. In some exemplary implementations, the continuous analyte sensor 10 may analyze a plurality of intermittent blood samples, although other analytes may be used as well.

In some exemplary implementations of Figure 3, the continuous analyte sensor 10 may comprise a glucose sensor configured to measure glucose in the blood using one or more measurement techniques, such as enzymatic, chemical, physical, electrochemical, fluorescent, spectrophotometric, polarimetric, calorimetric, iontophoretic, radiometric, immunochemical, and the like. In implementations in which the continuous analyte sensor 10 includes a glucose sensor, the glucose sensor may be comprise any device capable of measuring the concentration of glucose and may use a variety of techniques to measure glucose including invasive, minimally invasive, and non-invasive sensing techniques (e.g., fluorescent monitoring), to provide data, such as a data stream, indicative of the concentration of glucose in a host. The data stream may be a raw data signal, which is converted into a calibrated and/or filtered data stream used to provide a value of glucose to a host, such as a user, a patient, or a caregiver (e.g., a parent, a relative, a guardian, a teacher, a doctor, a nurse, or any other individual that has an interest in the wellbeing of the host). Moreover, the continuous analyte sensor 10 may be implanted as at least one of the following types of sensors: an implantable glucose sensor, a transcutaneous glucose sensor, implanted in a host vessel or extracorporeally, a subcutaneous sensor, a refillable subcutaneous sensor, intraocular, or an intravascular sensor.

In some implementations of Figure 3, the continuous analyte sensor system 8 includes a DexCom G4® Platinum glucose sensor and transmitter commercially available from DexCom, Inc., for continuously monitoring a host's glucose levels.

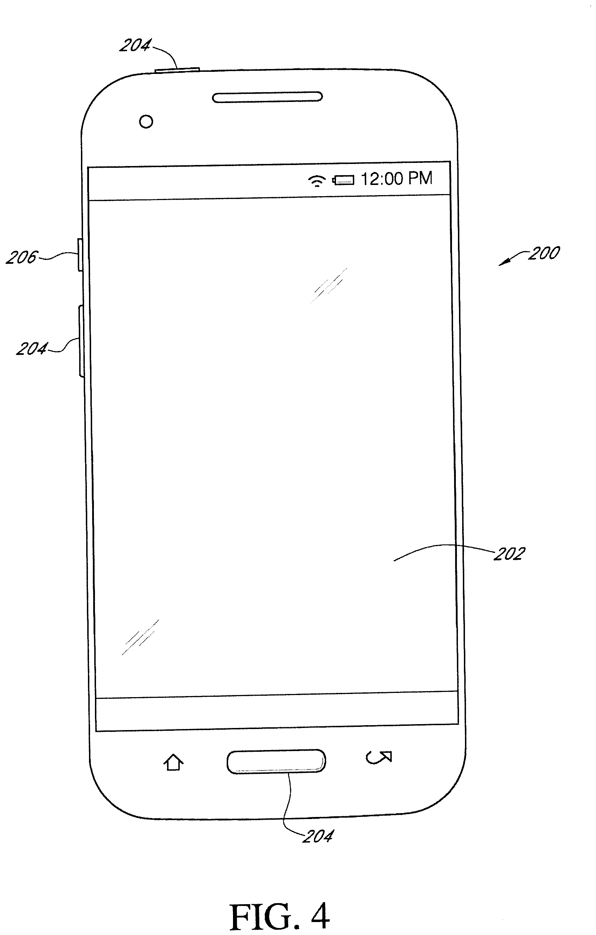

Figure 4 illustrates one embodiment of an electronic device 200 configured for use with the present systems and methods. The electronic device 200 includes a display 202 and one or more input/output (I/O) devices, such as one or more buttons 204 and/or switches 206, which when activated or clicked perform one or more functions. In the illustrated embodiment, the electronic device 200 is a smartphone, and the display 202 comprises a touchscreen, which also functions as an I/O device. In other embodiments, the electronic device 200 may comprise a device or devices other than a smartphone, such as a receiver of a CGM system, a smartwatch, a tablet computer, a mini-tablet computer, a handheld personal digital assistant (PDA), a game console, a multimedia player, a wearable device, such as those described above, a screen in an automobile or other vehicle, etc. While the electronic device 200 is illustrated as a smartphone in the figures, the electronic device 200 can be any of the other electronic devices mentioned herein and/or incorporate the functionality of any or all of the other electronic devices, including wherein some or all of the functionally is embodied on a remote server.

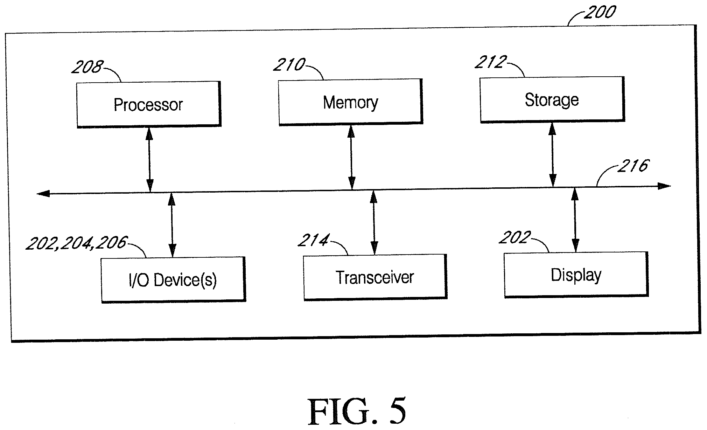

Figure 5 is a block diagram of the electronic device 200 shown in Figure 4, illustrating its functional components in accordance with some embodiments. The electronic device 200 includes the display 202 and one or more input/output ("I/O") device(s) 204, 206, as described above with respect to Figure 4. The display 202 may be any device capable of displaying output, such as an LCD or LED screen and others. The input/output (I/O) devices 202, 204, 206 may comprise, for example, a keyboard (not shown), one or more buttons 204, one or more switches 206, etc. In embodiments including a touchscreen, the display 202 also functions as an I/O device.

The electronic device 200 further includes a processor 208 (also referred to as a central processing unit (CPU)), a memory 210, a storage device 212, a transceiver 214, and may include other components or devices (not shown). The memory 210 is coupled to the processor 208 via a system bus or a local memory bus 216. The processor 208 may be, or may include, one or more programmable general-purpose or special-purpose microprocessors, digital signal processors (DSPs), programmable controllers, application specific integrated circuits (ASICs), programmable logic devices (PLDs), or the like, or a combination of such hardware-based devices.

The memory 210 provides the processor 208 access to data and program information that is stored in the memory 210 at execution time. Typically, the memory 210 includes random access memory (RAM) circuits, read-only memory (ROM), flash memory, or the like, or a combination of such devices.

The storage device 212 may comprise one or more internal and/or external mass storage devices, which may be or may include any conventional medium for storing large volumes of data in a non-volatile manner. For example, the storage device 212 may include conventional magnetic disks, optical disks, magneto-optical (MO) storage, flash-based storage devices, or any other type of non-volatile storage devices suitable for storing structured or unstructured data. The storage device 212 may also comprise storage in the "cloud" using so-called cloud computing. Cloud computing pertains to computing capability that provides an abstraction between the computing resource and its underlying technical architecture (e.g., servers, storage, networks), enabling convenient, on-demand network access to a shared pool of configurable computing resources that can be rapidly provisioned and released with minimal management effort or service provider interaction.

The electronic device 200 may perform various processes, such as, for example, correlating data, pattern analysis, and other processes. In some embodiments, the electronic device 200 may perform such processes on its own. Alternatively, such processes may be performed by one or more other devices, such as one or more cloud-based processors 22 described above. In still further embodiments, these processes may be performed in part by the electronic device 200 and in part by other devices. Various example processes are described herein with reference to the electronic device 200. It should be understood that these example processes are not limited to being performed by the electronic device 200 alone. Further, as used herein, the term "electronic device" should be construed to include other devices with which the electronic device 200 interacts, such as one or more cloud-based processors, servers, etc.

The electronic device 200 may also include other devices/interfaces for performing various functions. For example, the electronic device 200 may include a camera (not shown).

The transceiver 214 enables the electronic device 200 to communicate with other computing systems, storage devices, and other devices via a network. While the illustrated embodiment includes a transceiver 214, in alternative embodiments a separate transmitter and a separate receiver may be substituted for the transceiver 214.

In some embodiments, the processor 208 may execute various applications, for example, a CGM application, which may be downloaded to the electronic device 200 over the Internet and/or a cellular network, and the like. Data for various applications may be shared between the electronic device 200 and one or more other devices/systems, and stored by storage 212 and/or on one or more other devices/systems. This CGM application may include an urgency assessment module and/or may include processing sufficient to operate urgency assessment functions and methods as described below.

In certain of the present embodiments, the sensor 10 of the continuous analyte sensor system 8 of Figure 3 is inserted into the skin of a host. A new sensor session is then initiated with the sensor 10, the sensor electronics 12, and the electronic device 200. Numerous techniques may be employed for initializing the sensor 10. For example, initialization may be triggered when the sensor electronics 12 engages the sensor 10. In another example, initialization may be triggered by a mechanical switch, such as a switch (not shown) on a snap-in base that receives the sensor electronics 12. When the sensor electronics 12 are snapped into the base, the switch is automatically tripped. In another example, initialization may be menu driven, and the user may be prompted by a user interface on the display 202 of the electronic device 200 to begin initialization by making a selection on the user interface, such as by pushing a button or touching a designated area on a display 202 (which may comprise a touchscreen). In another example involving a non-invasive sensor that is applied to the wearer's skin, the sensor 10 may sense when it is in contact with skin and start automatically. Further, the analyte sensor system 8 can detect use of a new sensor 10 using any of the above techniques, automatically prompt the user to confirm the new sensor session by way of a prompt on a user interface of the system 8, and initiate an initialization response to the user confirmation responsive to the prompt. Additional examples of initializing the sensor 10 are found in U.S. Patent Application Serial No. 13/796,185, filed on March 12, 2013, the entire disclosure of which is hereby incorporated by reference herein.

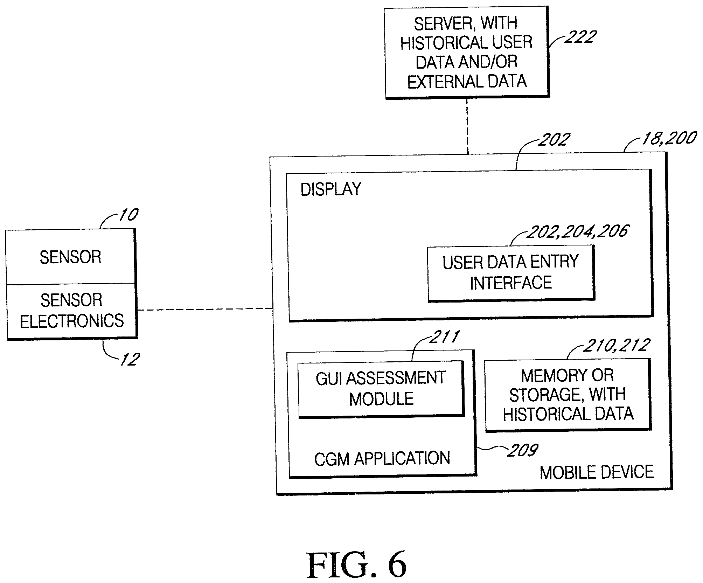

Figure 6 illustrates an exemplary logical diagram for the continuous analyte monitoring system 100, illustrating in particular components involved in determination of sensor results and portrayal of calculations and determinations of urgency based on the results as well as on other factors. In particular, measurements from the sensor 10 are processed by the sensor electronics 12 and sent to the mobile device 18, which is generally a smart phone. While a smart phone is described here, it will be understood that any of the various electronic devices described above may be employed to receive and display sensor or other data and output results, as well as alerts and alarms based thereon. Moreover, the smart phone (or device with similar smart phone capabilities) may transmit displayed notifications, results, alerts, and alarms, to various devices coupled thereto, e.g., via Bluetooth®. Such devices include head mounted displays like Google Glass®, watches, and the like.

The mobile device 18 runs a CGM application 209 by which various monitoring and display functions are provided, based on the signal received from the sensor electronics 12. As part of this CGM application, a GUI assessment module 211, which may also be a processor module, is provided to perform the urgency assessment functions described here. While an assessment module is described, it will be understood that such may be replaced by appropriate functionality to perform the methods described here.

The mobile device 18 includes a display 202 for displaying notifications, results and alerts/alarms. While the display 202 is portrayed as a display screen, and thus generally renders results visually, it will be understood that notifications, outputs, results, and in more urgent cases alerts/alarms may also be communicated using other means, such as audibly. The same may be communicated as an audible version of displayed text or numerals. Alternatively, tones or other sounds, even songs or ring tones, may be rendered to the user as a discrete indication of their blood glucose level.

The mobile device 18 may further include memory 210 or storage 212 for retrieval and usage of historical data, including user-entered data, as will be described in greater detail below. As the mobile device 18 may be in network communication with various servers, historical data may also be retrieved from a network server 222. Besides historical data, the server (or other network source) may further provide other external data that may enter into a determination that leads to the notification presented on the display 202.

The display 202 may itself provide an interface for the user to enter data, e.g., using a touchscreen interface, and the same may also be entered via buttons and switches 204 and 206 respectively. In some smart phones and in many other computing devices, a separate keyboard may be employed for the same purpose.

Signal processing may occur using the sensor electronics 12, using the mobile device 18, or using a combination of the two. Signal processing may also be performed in the cloud, e.g., on the server 222 or other network source. In many cases, however, initial processing of the raw sensor signal, such as calibration, smoothing or filtering, is performed by the sensor electronics 12, and an application on the mobile device 18 transforms the signal received from the sensor electronics 12 into a GUI which is then indicated on the display 202.

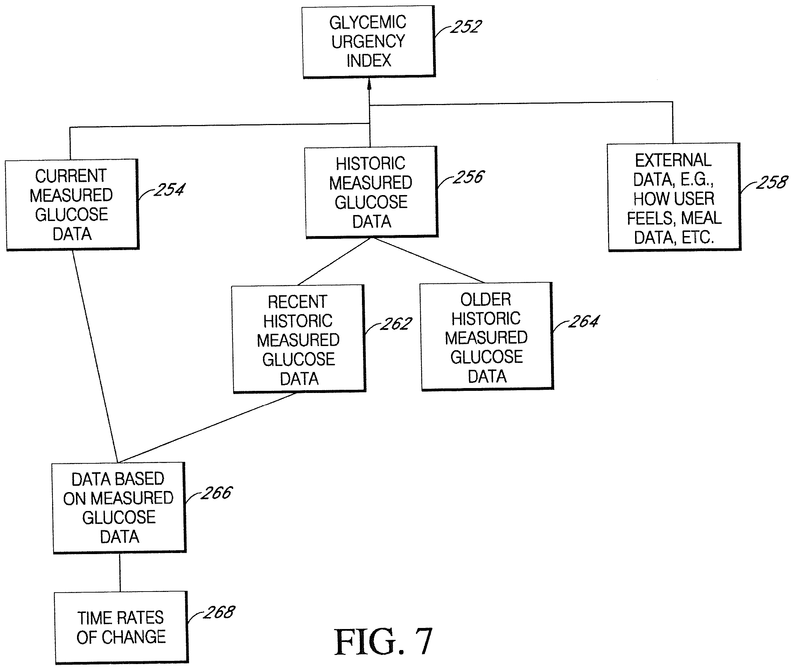

Figure 7 illustrates how the measured blood glucose level can be combined with other parameters or variables to result in a calculated or otherwise determined GUI value 252, which is then presented on the display screen of the mobile device, and which may be the basis for alerting and/or alarming. The calculation or determination is generally performed by the urgency assessment module 211 on the mobile device 18, but may also be determined in whole or in part by the server 222, or even in some cases by the sensor electronics 12. It will be understood that not all parameters and variables will enter into all implementations of determinations of the GUI value 252.

Various parameters and variables will be described, followed by examples of how the same may be combined to result in a GUI value on which a presented notification may be based, and/or on which alerts or alarms may be based to achieve the benefits and advantages described above. Without intending to limit the scope of the arrangements in any way, it is believed that particularly useful combinations will include the current glucose value or combinations of glucose value and a first derivative of glucose value with respect to time. Numerous combinations are useful, however, as will be understood by one of ordinary skill in the art given this teaching, and thus the scope of the invention is not to be limited by the specific examples. Moreover, while a single calculated or determined value of the GUI 252 may be employed in various implementations, it will also be understood that a plurality of values pertaining to glycemic risk or urgency may be calculated or determined, and the same employed in combination to drive alerting or alarming, or indeed the general presentation of results to a user, e.g., *GUI1 = GUI1 (parameters, variables), GUT2 = GUI2(parameters, variables),* and so on. In such cases the combined GUIs may be said to define a glycemic urgency state, which is then the basis of the alerts and/or alarms.

In Figure 7, the GUI 252 is illustrated as being based on at least data 254 corresponding to a current measured value of glucose, and/or data 256 corresponding to historic measured values of glucose, and/or data 258 which is not directly related to the measured values of glucose, and is thus termed "external data". The data 254 is generally the current measured value of glucose, e.g., as measured in mg/dL. The data 256 corresponds to historic measured values of glucose, and the same may be divided into data 262 termed "recent historic" measured glucose data and data 264 termed "older historic" measured glucose data. The recent historic data 262 may be that measured over the course of minutes or hours prior to the current measured glucose data 254, and thus may be particularly useful for current trending analyses. The older historic data 264 may be that measured over the course of days, weeks, months, or even years prior to the current measurement, and thus may be particularly useful in the calculation or determination of overall patterns or trends (data 262 may also be employed in this determination).

The current measured glucose data 254 and the recent historic measured glucose data 262 may be employed to calculate other types of data 266 based on current trends. For example, the same may be used in the calculation of data 268 corresponding to time rates of change of the glucose data, e.g., a first derivative with respect to time, a second derivative with respect to time, and so on.

The data 258 may correspond, e.g., to past or present user indications of how the user feels, what the user ate, and so on. Thus, the data 258 may bear an indirect correlation with glucose levels, but the same are not directly based, in the functional sense, on the measured glucose values. The data 258 may also constitute numerous other variables, as will be described below.

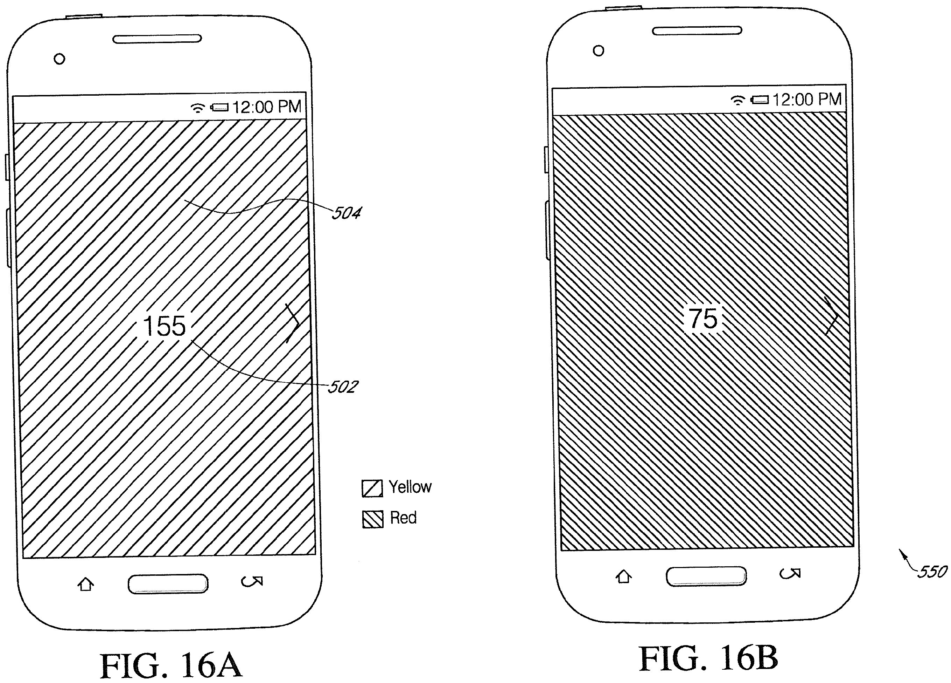

Various parameters and variables are described below that are based on the above types of data. Again it is noted that the determination of a GUI in a particular implementation need not include all the various types of data described, and in many cases will only include two or three types of data. Moreover, as the below description is only exemplary, data types other than those described below may also be employed. In particular, the calculation of a GUI may be performed by an algorithm, e.g., on the mobile device, as described above, and the algorithm may take into account several or numerous variables in its determination of the GUI. While these variables will be evaluated algorithmically at or near the same time, the below description in part discusses the effect of the variables sequentially, on each other, and on the determined GUI. In connection with a user interface for an electronic device such as a smart phone, the calculated GUI leads to a notification presented on a user interface of a mobile device, and which in some cases may further lead to an "actionable alert" (or alarm) which is displayed to the user and which suggests one or more actions to be performed. In some implementations, the notification seen by a user may simply be an indication of the user's status, e.g., that the user has a normal GUI. In other cases, the presentation may be of an alert or alarm condition, e.g., by the screen appearing in a red color, and thus may infer that some action must be taken. By unlocking the mobile device, performing a "swipe" action, or otherwise "drilling down" to the data underlying the existence of the alert or alarm condition, the user can view the unambiguous actions to be performed. Additional details of such user interfaces are described below in connection with Figures 16 - 29.

A first type of data that may be employed, and one that is involved in most implementations, is a measured value of glucose. This first type of data may be in numerical form, with units of mg/dL or otherwise, or may be processed or transformed to derive another type of data, generally correlated with the glucose value. In some cases, the first type of data may also be employed in its raw form, as received from the sensor electronics without significant processing and/or processed by the sensor electronics. Processing if desired may then be performed on the mobile device (or other device) running the urgency assessment module or related application to determine the GUI. The first type of data may further be received from an intermediate module or transformation, e.g., may be received from another application running on the smart phone. Generally this first type of data may undergo processing, e.g., to calibrate, smooth, filter, or otherwise "clean up" the signal representing the data.

While generally a current measured value of glucose is employed, it will be understood that the first type of data may also include one or more past values of glucose, or even future values of glucose as determined by a prediction algorithm. Additional details of prediction algorithms are discussed below.

In the GUI determination, all other factors being equal, a high glucose level tends to move the value of the GUI towards a greater value of urgency, that indicating a hyperglycemic state. Conversely, a low glucose level tends to move the value of the GUI again towards a greater value of urgency, that indicating a hypoglycemic state. A middle glucose level tends to move the value of the GUI towards that indicating a euglycemic state. In a very specific example, a euglycemic state may be associated with the GUI of 0, an extreme hypoglycemic state may be associated with the GUI of -5, and an extreme hyperglycemic state may be associated with a GUI of +5. Of course, numerous other schemes will also be understood and may be employed given this teaching. Although 0-5 have been exemplified with positive and negative values representing hyperglycemic versus hypoglycemic risk, the risk indices could be agnostic to hyperglycemia versus hypoglycemia risks, for example, simply from 0-5, wherein 0 represents no risk and 5 represents the highest risk (whether or hypoglycemia or hyperglycemia). The indices could be categorized qualitatively into risk buckets such as "no risk," 'low risk," "medium risk" and "high risk," for example. Other quantitative or qualitative risk indexes may be envisioned as is appreciated by one skilled in the art, understanding that the risk index is not necessarily correlated with glycemic state, but rather urgency of clinical action to avoid a dangerous glycemic status. Time information such as time to next urgency index or time to a particular glycemic state could also be provided.

Other types of data may be based on this first type of data, e.g., the first derivative of the glucose values with respect to time can be employed to determine a time rate of change of glucose value, i.e., a "velocity" of the glucose value, i.e., if the glucose value is increasing or decreasing, as well as how fast such changes are occurring. Thus, a data value representing the first derivative can be employed in an initial estimate of a prediction of future glucose values, and also in the determination of the GUI. For example, a user with a high glucose value may start with a GUI value of 5, but a negative first derivative may cause the GUI to decrease to 3. Additionally, the direction and amplitude of the first derivative may be used to determine a weight of the same information in the determination of the GUI.

The first derivative of the glucose value, as well as higher order derivatives, particularly with respect to time, requires a certain amount of historical data to be stored and used in a calculation. Such data is generally based on recent historical data, but it will be understood that older historical data may also be used and may also provide useful information with regard to user patterns, as will be described below, where such user patterns may be analyzed in the abstract or with respect to, e.g., time of day.

Another type of data that may be based on the glucose value, and for that matter on the first derivative, is the second derivative of the glucose value with respect to time, i.e., the acceleration. Such provides information about the speed with which changes in glucose level are occurring, and can often advantageously be employed to determine to what extent changes in glucose level will stabilize or will lead to excursions from desirable values.

In the above example, where a glucose value *per se* leads to a GUI of 5 and the first derivative moderates the same to 3, the second derivative may be employed to raise the GUI (where the second derivative indicates the decrease will soon "turn around") or to further lower the GUI (where the second derivative indicates the decrease will accelerate). In some cases, the second derivative may indicate that not only is the user headed towards euglycemia but also that the user may enter a hypoglycemic state, e.g., the GUI may go to 0 but return back into a low, moderate or high GUI as appropriate.

In some implementations, the determination of a glycemic urgency state or index indicating such a state may be based, at least in part, on the measured glucose value and the first or second derivative with respect to time of the measured glucose value, or both. Such implementations allow a significant degree of confidence that alerts or alarms triggered will indeed be situations requiring user (or other) intervention, with a minimum of alerting or alarming in situations which will likely be resolved without user intervention. In some implementations, the calculation of the glycemic urgency state or index may be based on the factors above in combination with other factors, described below. For example, the glucose value, in combination with another type of data based on the glucose value, e.g., a time derivative, may be employed in combination with duration data (discussed below) to determine that a glycemic urgency index has been reached requiring intervention by a user. In the same way, the glucose value and/or a time derivative may be employed in combination with food ingestion data to determine if an alert should be triggered, e.g., if a user has a low glucose value but has just eaten a candy bar, an alert may be suppressed (all other aspects being equal). Insulin data may be used similarly. Other exemplary combinations will be described below.

Here it is noted that the concept of an alert suppression has been used to indicate that an alert condition has been reached but the same is not shown to the user because of various other factors. It should be clear however that in other implementations, the concept of suppression may be replaced with simply recalculating the variable on which the alert or alarm is based, e.g., the GUI, and then basing the alert or alarm on the recalculated value,

Returning to types of data based on glucose values, such may further include higher order derivatives with respect to time, glucose trace graphs over a period of time, the level and duration of the last significant glucose value excursion, e.g., a level of the last glucose peak, and so on. For example, if the user has a GUI of 3, but the last significant glucose value excursion was high and had an extended duration, such may tend to move the GUI upwards in the GUI determination (e.g., to 4 or 5).

Another type of data, tied to the sensor and electronics measuring glucose value, but not necessarily tied directly to the glucose value itself, is the accuracy, confidence level, and/or noise information in the glucose measurement. In particular, glycemic urgency indices are only as accurate as the underlying data that was processed. Thus GUIs and the like may be made more reliable by including accuracy information for certain inputs as available, and in some cases the urgency assessment module can determine how much weight to give a particular input based on the accuracy information. Alternatively, ranges (instead of a single numerical value) may be displayed for various outputs to indicate the output is subject to some uncertainty. The accuracy information may take various forms, including levels of noise, levels of confidence, percentages, numbers, or categories, for example. A particularly important quantity in this regard is the quality of the glucose data a sensor signal itself, including aspects as to signal quality, errors, and confidence levels.

For example, if the GUI value is 5, which is only mildly high, if the sensor data brings into question the accuracy and confidence in sensor values, such may tend to increase the GUI value in order to provide the most conservative and safe measurement for the user. An appropriate alert may be provided, if the situation continues, to reset the sensor or electronics, or the like.

Such data is generally available using data from the sensor and associated sensor electronics, or from analysis of the signal itself. Further details about accuracy, confidence level, and noise information, and the treatment of such in analyte measurements, are disclosed in US Patent Application Serial Number 12/258,345, filed October 24, 2008 and published as 2009/0192366 A1, entitled SYSTEMS AND METHODS FOR PROCESSING SENSOR DATA, incorporated by reference herein in its entirety.

In a related type of data, an input to the urgency assessment module may be provided by the sensor electronics or a processing circuit or software on the mobile device (or server), indicating an amount of processing undergone by the glucose value signal, and thus a value of lag associated with the signal. Such processing may include an amount of calibration, filtering, smoothing, and the like. The more processing a signal is subject to, the more lag is created in the signal. Accordingly, if a large amount of processing is performed or is necessary on the signal, or if processing has been delayed for some reason, it may be assumed that the signal has greater lag, and the signal itself may be deemed more important (or associated with more weight) in the urgency assessment module, as a delayed signal may be considerably more problematic than a non-lagged one. In particular, there is a higher potential for an unknown excursion from the last known value. Thus, e.g., if the GUI value is 3, but a significant lag is determined, the GUI may be caused to tend upwards in the GUI determination for similar reasons as with the low accuracy signal situation. And similarly, such data is generally available from the sensor and associated sensor electronics. However, such data may also be determined from analysis of raw signal data itself, e.g., recent historic values of measured glucose concentration.

For another type of data which may be employed in calculations, the glucose values may further be processed to provide a predicted value of glucose, or a range thereof. In particular, real-time glucose values may be "time lagged" relative to actual glucose values due to physiological and/or data processing reasons. For example, values measured in blood as a result of a finger prick may not be indicative of blood glucose in the brain at the same time as the measurement. Moreover, data processing steps such as calibration, smoothing, and filtering as described above may introduce additional lags. To address both of these problems, predictive algorithms may be employed to determine a predicted glucose value which may then be provided as an input to the urgency assessment module in the determination of a glycemic urgency state or index. Here again it is noted that the GUI is not a predicted value of glucose itself, but rather an index relating to the potential risks, dangers, or urgency of the subject user's glycemic state, and which can provide an actionable alert based on the value of the index. In some cases, rather than a particular predicted value, a range of predicted values may be determined, and the same may be used in the GUI determination. Finally, predictive algorithms may provide additional insight into the glycemic state of the user, which may be useful in combination with the other inputs described herein in the determination of glycemic urgency, even without the benefit of reducing the effects of lag.

Systems and methods according to present principles allow the extension of prediction horizons over those previously possible. For example, while some levels of prediction have previously allowed the detection of hypoglycemic events occurring a certain period of time in the future, by use of several parameters and variables in the determination of a GUI, the prediction horizon can be significantly extended. Exemplary prediction horizons may include those of 10 minutes, 20 minutes, 30 minutes, 45 minutes, one hour, 90 minutes, or even longer.

For example, if the GUI value is 3, but the predicted GUI value indicates that the user is heading towards a more hyperglycemic state, the GUI value may be raised to, e.g., 4 or higher. Again, the GUI itself is not the glucose value, but the glucose value may bear on the GUI.

Data for predictions is generally available via an analysis of the stored glucose values. Further details about predictive algorithms are disclosed in US Patent Application Serial Number 11/007,920, filed December 8, 2004, and granted as US 8,282,549 on October 9, 2012, entitled "SIGNAL PROCESSING FOR CONTINUOUS ANALYTE SENSOR", incorporated by reference herein in its entirety.

The duration over which a measured glucose level occupies a predetermined range is yet another type of data which may be employed in calculations, and the same may be determined by analysis of the glucose values and in particular values over time. The predetermined range may be arbitrarily-defined, but generally may indicate a particular urgency state, e.g., high or low hyperglycemic, high or low hypoglycemic, or euglycemic.

In more detail, to address the problem of the prior lack of consideration of such durations, the time in which a user spends in a range corresponding to an urgency state (or other range) may provide an important input to the urgency assessment module, as the same may correlate to dangers faced by the user, especially where the urgency states are those of hypoglycemia or hyperglycemia. For example, if the user has a GUI (based on other factors) of - 3, but the duration of a relatively low hypoglycemic event is considerable, then the GUI may be further decreased to -4 based on the duration, as excursions further downward become that much more likely, and the urgency should be increased. In using duration as a factor, the urgency assessment module may use as an input the duration itself, or a time over which a particular urgency state has exceeded a threshold duration, or other related parameters. Such data is generally available via an analysis of the stored glucose values over time. Additional details are discussed below in connection with Example 1.

Another type of data which may be employed in calculations or determinations of GUIs corresponds to recent or historic events, and in particular large excursions from an expected or a baseline value of glucose level or GUI. In particular, users who have had recent significant excursions are generally more likely to have significant current or future excursions. To address this problem, the urgency assessment module may take account of such prior historical events in determination of the GUI. For example, the level of the last glucose peak, or its duration (as measured as a time period over a threshold level or within a range), or the like, may be employed in determinations. The level and/or duration of the last significant excursion or deviation of glucose values away from a baseline (or an otherwise expected value) may be employed in the determination as the same are often indicative of a user's current risk of a glycemic excursion, and in particular are an indicator of a greater likelihood of future excursions or deviations. For example, for a determined GUI of 6, but where a user has undergone many recent excursions or deviations, the GUI may be raised to 7. As a subset of this data type, the "last hypo/hyperglycemic event", including its level and duration, may be employed in the determination. In any event, such data is generally available via an analysis of the stored glucose values.

A further type of data which may be employed in GUI determinations employs older historic measured glucose values. In one case, and as mentioned above, patterns of glucose values may be determined and employed to inform a baseline from which, e.g., excursions are measured, which may constitute significant deviations from the baseline. The pattern data may be partially time or time-of-day-based, but are not necessarily so. In particular, users often follow very regular patterns based on eating, exercising, or other activities which can bear on glucose levels, such activities occurring at certain times of the day. These can be advantageously employed in determining whether excursions are expected outside normal levels. If the time of day confirms a pattern, the determined GUI can be more predictive, confident, and can provide more useful feedback. Using pattern data in the GUI algorithm addresses the problem of otherwise normal GUI values causing alerts or alarms, and thus assists avoiding the problem of "alert fatigue". Naturally such pattern data is generally available via an analysis of the stored glucose values.

For example, a user may generally experience a lower glucose value in the morning than in the afternoon. The urgency assessment module may adapt to this pattern, and expect a lower reading in the morning and a higher reading in the afternoon. Similarly, the user may typically consume a meal of oatmeal in the morning, and thus cause a spike in their glucose value. Rather than necessarily causing an alert or alarm, the urgency assessment module may determine that such a meal at approximately the same time every morning constitutes a pattern, and may suppress alert triggering, as the same is simply considered "normal" based on the GUI assessment. As noted above, the "suppression" may simply be a recalculation of the GUI that results in no alert triggering. Considering the patterned values of the baseline would thus cause analysis of the spike to not be labeled a spike at all. Of course, other factors bear on the calculation of the GUI, and if they as a combination determine an urgent GUI, an alert or alarm will be triggered.

In the above situation, a spike in glucose level due to the oatmeal may cause an increase in the GUI away from a euglycemic state without pattern information, but recognition of the pattern in the GUI determination may cause the GUI to more accurately maintain its value.

While eating and sleeping have been disclosed elsewhere herein, it will be understood that patterns may be recognized or generated and employed in GUI determination for other events, such as meetings, work, exercising, and the like. Time-of-day information may be captured from any clock circuit or application, such as those from a server, or from the mobile device or sensor electronics. Patterns may be based on detected events occurring with any sort of periodicity, such as during a cycle of a day, week, or month. Such data is generally available via an analysis of the stored glucose values, and various pattern recognition software applications may be advantageously employed. In some cases, a pattern may be detected, and a user may be prompted to determine if there is a particular cause for the pattern, e.g., a common mealtime, an exercise class occurring at a usual time, and the like. Such prompts may be particularly used when the urgency assessment module is using machine learning to determine daily or other periodic patterns or behaviors of a given user.

In this same way, deviations outside a recognized pattern may cause a similar user prompting. For example, a deviation may cause the urgency assessment module to ask the user "what did you do differently?". Such may allow analysis and disambiguation of, e.g., a missed bolus versus an insufficient bolus.

Such pattern data may even provide prospective notifications or alerts. While details of user interfaces for such notifications, alerts, and alarms are described in greater detail below, here it is noted that pattern data may be employed to suggest to a user where their glucose level (or GUI) is heading, based on past historical data. For example, the urgency assessment module may send a warning such as "it's almost 2 PM and we know at 2 PM you are often low. You should review $X$ and take possible action $Y$", where $X$ is a user - understandable variable such as glucose level and $Y$ is an appropriate action to take given the current determined GUI.

It will also be understood that other types of data may be employed related to deviations from normal glucose patterns but which are not necessarily time - based. Such may include where exercise (detected by motion or heart rate, for example) is usually associated with a lowering of glucose level. "Normal glucose patterns" may be learned for a specific user using known pattern recognition algorithms. A deviation from such normal patterns may then be defined and employed as an input into the determination of the GUI. In some cases, a glycemic event outside the norm may be a predictor of a higher risk state, at least in part, due to the unexpected nature of the event, which might dictate a different type of output to the user, i.e., a different type of notification, alert, or alarm rendered on a display of a mobile device, or output to an insulin delivery device, i.e., pump. In this way, the problem of treating non-time-based patterns may be effectively addressed.

Other types of data based on glucose values, or glucose values as measured over time, will also be understood. For example, a glucose trace over a recent time period, e.g., six hours, may be employed to inform current GUI calculations or determinations.

Other types of data may be employed in the determination of the GUI, and which are not based on glucose values. A first category of types of such data are those based on data from other sensors or sources, or entered by the user. For example, the data may be of a type including anthropometric data, e.g., corresponding to body measurements such as BMI or weight. Anthropometric data may be particularly important for Type II diabetes patients, but may also have some bearing for Type I. In particular, for Type II diabetes patients, changes in anthropometries may have a significant bearing on the GUI determination. For example, an improvement in BMI for a Type II patient should translate into a better GUI on average, all other aspects being equal. Anthropometric data measurements may be captured semi-automatically, e.g., via a connected weight and height scale, or the values for such BMI calculations can be entered manually by the user, e.g., in the user interface of the mobile device. Measurements may also be imported from other systems, including from the cloud. In this way, the problem of treating all users the same, no matter their anthropometric data, may be effectively addressed and resolved.

For example, all other factors being equal, a user may have a GUI determined of 6. If the user is obese, the GUI may be raised to 7 based on that factor, as the urgency or risk to such individuals is greater than for non-obese individuals.

Another type of data which may be employed in the determination of the GUI is user activity level, in particular data about the amount of activity, the type of activity, and a duration of the activity (or a combination of these). In particular, quantification of user activity levels generally may provide a better understanding of glucose value trends. Activity information can feed into the determination of the GUI, and may also be useful to present to a user desiring to receive additional information as to why their GUI has a particular value. Such may also be employed to determine what sorts of questions may be asked to assist the user in managing their diabetes.

For example, a user may have a GUI determined of 0, indicating a euglycemic risk state, but a first derivative of glucose value may indicate that the same is decreasing, potentially causing the GUI to drop to -1. If a determined activity level indicates that the user has recently performed a significant amount of exercise, and the cause of the rise can be attributed to physical activity rather than overdosing of insulin, for example, particularly if the second derivative indicates that the glucose value will be increasing then a GUI of 0 may be maintained.

Measurement of the activity level may be via accelerometer, GPS data, or even WiFi data indicating location. In a specific implementation, the M7 chip on the iPhone® 5 smart phone employs a motion coprocessor, which allows the mobile device to, e.g., count steps, and more generally to determine whether the mobile device user is stationary, walking, running, driving, or the like. In another specific implementation, third-party devices such as FitBit® may be employed. It will be understood that such data may be entered manually as well, e.g., miles run, walked, or biked. Using such systems and methods according to present principles, problems related to hyperglycemic and hypoglycemic events caused by or in concert with user activity levels may be effectively addressed.

A related type of data is information about exercise, which in general is beneficial for diabetes patients, and can help prevent hyperglycemia and hypoglycemia as well as assist in the management of insulin delivery. However, exercise sometimes has long-term effects on diabetes, and can cause severe hypoglycemia hours later in certain users. Accordingly, it is occasionally difficult to identify exercise as a cause of hypoglycemia because of this long time lag. If exercise can be accurately detected, such as by using the measurement devices noted above, predictive analytics may be employed to predict when it may begin to affect the glucose value and thus the associated risk state, e.g., GUI. Exercise can be monitored using generally the same types of devices employed to monitor activity, and may include parameters such as the duration of exercise, the type of exercise, the amount of calories burned, and the like. It will be understood that such data may be entered manually as well.

A further related type of data corresponds to sleep information or state. In particular, diabetes users are known to be more likely to undergo an undetected hypoglycemic event while sleeping. Motion, or the lack thereof, as well as other factors, may be employed to detect sleep and correspondingly evaluate risk. Other factors may include, e.g., heart rate, user input, and the like. The monitoring device, e.g., a mobile device running an urgency assessment module, may be equipped with a "night mode" feature or module, instantiatable by the user, which may be employed to assist in the detection of sleep. Motion detection for such purposes may be performed as noted above, e.g., by use of an accelerometer worn on the body. For example, a CGM sensor or transmitter may incorporate such an accelerometer or other motion detection circuit. A phone or other motion detector placed adjacent the user can detect how often the user moves, again indicating sleep. In some cases, an alarm system's motion detector may be employed to provide such information and data. A heart rate monitor can measure changes in the user's heart rate. The user interface of the mobile device may be employed to assist in the detection of a sleep state as well. For example, if a user is not interacting with the mobile device at all, as determined by button presses, swipes, or other like interactions, such may be associated or consistent with a state of sleep, or the same may be learned by the urgency assessment module to be associated with such a state. Conversely, if a user is interacting with their mobile device, it may be assumed the user is not sleeping.

In an example according to present principles, if the user was in a euglycemic state, e.g., having a GUI of approximately 0, but is currently not moving and their heart rate is dropping, they may be hypothesized to be asleep and thus the urgency assessment module may assess a higher risk of the user undergoing an undetected hypoglycemic event. This risk may be factored into the GUI determination, e.g., causing a more prominent alert or alarm, e.g., one corresponding to a GUI of (-)4 or (-)5. If the mobile device running the urgency assessment module is equipped with a "sleep mode" function, the user may activate such, in which case no assumptions about sleep or sleep detection is necessary.

Such a "sleep mode", "night mode" or sleep detection functionality may afford a number of advantages in certain implementations. In particular, by assigning a higher risk state to a glycemic event during the night versus during the day, the system understands that the user is more likely to be unaware of their diabetic risk state, and thus the glycemic event should be handled differently. In this way, the problem of user inattention during sleep, or excursionary values of glucose encountered during sleep, may be effectively addressed.

Another category of types of data employable in GUI determination corresponds to physiological data. One such physiological data type includes hydration information. In particular, dehydration is often associated with high blood glucose levels. Accordingly, the same may be employed to further inform the GUI determination. Hydration information may be received from, e.g., a Tanita BC-1000 body composition monitor in combination with a Garmin® Connect system. It will be understood that such data may be entered manually as well, at least at a qualitative level. As an example of GUI determination using hydration, a user may have a GUI determined of 3, all other factors being equal. If the user is dehydrated, such may push the GUI up to 4, to indicate the greater likelihood of a hyperglycemic event. While generally sensor data is employed to measure hydration, the same may be entered by a user as well, at least qualitatively.

Another such physiological data type includes heart rate information. Heart rate can be indicative of exercise or indicative of other factors such as stress. Where heart rate or changes therein are due to exercise or other activity, the activity monitors noted above may be employed to quantify the same. Alternatively, heart rate may be communicated wirelessly from a heart rate monitor or other application. In another implementation, the same may be entered manually by the user, using indications such as "high heart rate", "normal heart rate", and the like, or quantitative values if the user can measure such.

Another such physiological data type includes blood pressure information. In particular, the effect of diabetes on blood vessels tends to heighten the risk of high blood pressure. Accordingly, monitoring the same can be useful and can be factored into the determination of the GUI. Various body-worn blood pressure monitors are available, and the same can, in a wired or wireless fashion, communicate blood pressure data to the device running the urgency assessment module. Alternatively, users may measure their own blood pressure and enter the same manually into the device.

A further physiological data type includes body temperature. Body temperature is often an indicator of illness, which can in turn affect the diabetic risk state and thus the GUI. For example, the body temperature and/or underlying illness may cause the glycemic response to various inputs or therapies to be different from what is expected in other users, or from what is expected historically from the same user.

The body temperature data type can be captured by introducing a temperature sensor into the sensor patch or by the use of other such thermometers. This and other types of body temperature monitors may be found in US Patent Application Serial Number 13/747746, filed January 23, 2013, and published as US 2014/0005508A1, entitled "DEVICES, SYSTEMS, AND METHODS TO COMPENSATE FOR EFFECTS OF TEMPERATURE ON IMPLANTABLE SENSOR", owned by the assignee of the present application and herein incorporated by reference in its entirety. Temperature information may also be input manually qualitatively or quantitatively.

To exemplify the above-mentioned parameters or variables, a user with a determined GUI of 3 (without heart rate, blood pressure or temperature inputs) may be determined to have a GUI of 4 if their heart rate, blood pressure, or temperature is particularly high, thus indicating a higher urgency associated with the glycemic state. Using such systems and methods according to present principles, the problem of user glucose monitoring, lacking consideration of such refining parameters or variables, may be effectively addressed.

The level of user interaction with the monitor was mentioned above in connection with a determination or detection of a sleep state. Such may also be employed generally to determine a level at which a user desires to control or being notified about their diabetes, at least with regard to a level of user interaction with their glucose monitor. In particular, the level at which the user interacts with their CGM, e.g., a mobile device running an application in which the GUI is determined by an urgency assessment module, may be employed as a factor in GUI determination. For example, a high level of user interaction may be indicative of a strong awareness of the user's glycemic state, and may correspondingly result in lower risk. In contrast, a low level of user interaction can be indicative of a low awareness or even non-awareness of the glycemic state, and consequently may result in a higher risk assessment and thus GUI, particularly if the glucose is on the "border" of euglycemia, which input (distance from target range) could be included in the GUI determination. Such may be measured by an amount of time the screen is on, a number of button presses or swipes, orientation as determined by an accelerometer, and the like. It should be noted, however, such data may be modified or informed in one way or another by user pattern data. For example, pattern data may indicate that a user does not use their mobile device after 8 PM. In this case, a user may not be considered a "low awareness" user based on lack of user-device interaction in the late evening, as such is simply associated with the pattern. However, if the same user is generally highly interactive with their device during the daytime, but is suddenly non-interactive for a long period of time in the afternoon, such may increase the urgency assessment as the user may be assumed to be unaware of their current glycemic risk state.

For example, a user in a hyperglycemic risk state may be alerted to the same and may be determined to be treating the condition appropriately, e.g., by analysis of one or more time rates of change of the glucose value. If the user is highly interactive with the electronic device, e.g., mobile device, the GUI may maintain a current value, with appropriate ensuing alerts (which may be none). If the user is not as interactive with the monitoring device, then the GUI may tend upwards, causing additional alerts (or an indication of a heightened GUI on a user interface) in order to obtain the awareness of the user.

Using such monitoring device user interface data, the problem of treating patients having differing usage habits of their monitoring devices may be effectively addressed. Data about usage is generally obtained using the operating system of the monitoring device, e.g., mobile device.

In a similar type of data, context and behavioral information can be employed in a GUI determination. In particular, such information may correspond to how a patient uses their mobile device, and thus gives context to certain data determined by the device. Behavior input information may be obtained via the system and can include an amount of interaction, glucose alerts/alarms states, sensor data, number of screen hits, alarm analysis, events (e.g., characteristics associated with the user's response, time to response, glycemic control associated with the response, user feedback associated with the alarm, not acknowledging alerts/alarms within X minutes, time to acknowledgment of alerts/alarms, time of alert state, and so on), diabetes management data (e.g., CGM data, insulin pump data insulin sensitivity, patterns, activity data, caloric data),data about fatty acids, heart rate during exercise, IgG-anti gliadin, stress levels (sweat/perspiration) from skin patch sensor, free amino acids, troponin, ketones, adipanectin, perspiration, body temperature, and the like. The inputs may be provided by a sensor in data communication with the monitoring device. In some implementations, the information may be obtained through an intermediary such as a remote data storage.

Contextual information which may be provided as an input to the GUI determination includes a person's biology, location, sensing surroundings (e.g., light, sound level), environmental data (e.g., weather, temperature, humidity, barometric pressure). The inputs may be received via a peer-to-peer or a mesh network via machine-to-machine communication. Context information can include daily routine information (which may change especially from weekdays to weekends) from a calendaring application. Context information can include a frequency of touching or grabbing the monitoring device, even if not interacted with, based on a sensed motion of the device.

Photos can provide contextual information. For example, photos of one or more of: a glucose meter reading, an insulin pen or pump IOB, a location (e.g., a gym, park, house, Italian restaurant), or a meal may be used to provide context information. The photos may be processed to identify, for example, caloric intake for the meal shown in the photo. The type of insulin used may also be provided to the monitoring system as a useful input to the GUI determination. Context may also be provided by basal or bolus settings provided to or determined by the monitoring device.

Other inputs to the GUI determination which constitute context/behavioral data may include data types referenced elsewhere in non-context/behavioral inputs, such as exercise information from a fitness bike or the like, glucose sensor information from a blood glucose (BG) meter or CGM, insulin delivery amounts from insulin delivery devices, insulin on board calculations for the device, and other device provided or calculated information. Other context/behavioral data inputs to the GUI determination may include: hydration level, heart rate, target heart rate, internal temperature, outside temperature, outside humidity, analytes in the body, hydration inputs, power output (cycling), perspiration rate, cadence, and adrenaline level, stress, sickness/illness, metabolic/caloric burn rate, fat breakdown rate, current weight, BMI, desired weight, target calories per day (consumed), target calories per day (expanded), location, favorite foods, and level of exertion.

For any of the above referenced behavior or contextual inputs, the system may be configured to receive and/or generate analytical metrics based on the inputs. For example, a composite value may be generated based on the glucose level, temperature, and time of data generated index value for the user. The composite value may then be considered in the determination of the GUI.

This information can be collected from various sensors within or outside of the device, such as an accelerometer, GPS, camera data, and the like, as well as third-party tracking applications, including sleep cycle applications. For example, such tracking applications may employ geolocation to determine context and behavior. Moreover, context and behavior may also be determined by use of social networking information available about the user, where a social networking feed, associated with the user, is arranged to provide a source of data to the urgency assessment module and/or for providing output thereto.

Using such systems and methods according to present principles, the problem of lack of consideration of such context/behavior aspects may be effectively addressed. Additional details about context and behavior information may be found in US Patent Application Serial Number N61/898,300, filed October 31, 2013, and entitled "ADAPTIVE INTERFACE FOR CONTINUOUS MONITORING DEVICES", owned by the assignee of the present application and herein incorporated by reference in its entirety, and in particular at Figure 4 and accompanying text.

Other types of data which may be employed in the GUI determination include information about food and drink ingested, and insulin. Variables or parameters pertaining to these data types may include information about their amounts, their types, and the time and duration over which they were received.

In the case of food and drink ingested in meals, such may be captured in a number of ways, e.g., manually by a user entering food and drink information into the device, e.g., on a spreadsheet, using the camera on the mobile device to capture a photograph of the meal, or by entering data from third-party food applications, which may allow, e.g., food items of a given restaurant (having data already entered in the application) to be "checked off" and entered into the determination as they are consumed. In some cases, users may be prompted for such information, e.g., if the device detects a spike in glucose level. Meal data may even be hypothesized (subject to confirmation by the user) by use of GPS or social networking data indicating that a user is near, or has "checked in" at a known favorite restaurant. The user may be prompted to confirm that they have ordered their "usual meal", which may then automatically populate food data with the parameters of that meal or alternatively the prompt may provide an opportunity to enter other food choices if the user has deviated from their usual option. Generally meal data may be provided with details such as the amount ingested, the time of ingestion, and other meal data which allow a clinically significant determination of the GUI. Using such information, problems currently encountered in diabetes care based on the lack of such (and other factors) may be effectively addressed.

In one example of the use of meal data in a GUI determination, a user in a mildly hypoglycemic state may have a GUI of -2. If the user eats a meal with significant carbohydrates and/or sugar intake, the GUI may be modified to -1, to reflect the fact that the urgency assessment of the user has been mollified. It is further noted that the modification may occur immediately upon notification that the user is eating a meal, well before a change would be seen in the blood glucose level.

Another variable or parameter which may factor into the GUI determination is a level of insulin. The data may be provided directly from an integrated insulin pump or from EMR in the cloud. Such data may include information about the amount of insulin on board, insulin sensitivity, and past, present, and future planned basal and bolus levels. Data may be obtained by sensor data or other electronically communicated data, or may be provided by user entry. One type of information which may be obtained from this data includes the time between an insulin bolus and a meal peak, which can be determined using insulin information and glucose information.

For example, a user in a hyperglycemic state may have a GUI of 3. If the user injects a bolus of insulin, the GUI may be modified to 1, to reflect the fact that the urgency assessment of the user has been mollified. With systems and methods according to present principles, the modification may occur immediately upon notification that the user has injected the bolus, well before a change would be detected in the blood glucose level. Using such insulin data, problems encountered in prior diabetes management may be effectively addressed, e.g., a lack of immediate updating of risk state based on user-entered data about meal intake.

A further type of data which may be employed in the GUI determination corresponds to stress level. In particular, stress is known to affect diabetes and thus to affect a user's risk state. In some cases, such data may be provided via a sensor, but in many other cases is captured by asking the user to choose from various emoticons or other indications of emotion. Such may also be inferred from other sources, by analysis of events on a user's calendar or other regularly scheduled activities, e.g., work, exercise, family time, or the like. Stress data may include information about the amount of stress, the type of stress, and how long the stress lasted.

A related type of data which may be employed in the GUI determination corresponds to current health, which may have overlap with current emotional state. Such measurements can be captured manually, via the device, including by using the same sort of emoticons noted above with respect to stress, or may be imported from information in the cloud. Current health and emotion are known to have a significant impact, particularly on Type II glucose control and insulin resistance, similar to anthropometric data. Health data may include information about a current illness, the severity of the illness, how long the user has suffered with the illness, and so on.

For example, a user with an otherwise non-risky GUI may have their GUI increase if they are currently undergoing significant levels of stress or poor health. Such an increase reflects the fact that these factors are known to cause blood sugar levels to deleteriously increase or decrease. Using such data types, problems seen in the past associated with a lack of consideration of a user's current stress or health may be effectively addressed.

Demographic data may also be employed such as age or gender. In particular, demographic data may be collected from online stores, network or cloud sources, or manually entered into the device, and such may provide useful information in the determination of the GUI. For example, it is known that pediatric users are more prone to faster and higher glycemic swings. As another example, it is believed that the risk state of a user, particularly in older users, and particularly those with Type II diabetes, may have higher risk states with certain glycemic excursions as compared to younger users with the same glycemic excursion.

In a particular example, a pediatric user in an elevated risk state, e.g., an otherwise-calculated GUI of 3, may have their risk state elevated to 4, to reflect the tendency of pediatric users towards faster and higher glycemic swings.

Using such data, problems seen in the past with a lack of consideration of such factors may be effectively addressed.

Another factor which may be employed in the GUI determination is the sensor site location. In particular, in some cases the site or location of a CGM sensor may lead to maintained distinctions in blood glucose level with respect to such locations. These distinctions may be factored into determination of the GUI. Such data is generally entered by the user manually, although historic data may be employed to avoid such user input, if such is regular and thus if an unambiguous determination may be made. Additional details about the use of sensor site location may be found in US Patent Application Serial Number 61/904,396, filed November 14, 2013, entitled "INDICATOR AND ANALYTICS FOR SENSOR INSERTION IN A CONTINUOUS ANALYTE MONITORING SYSTEM AND RELATED METHODS", owned by the assignee of the present application and herein incorporated by reference in its entirety.

Another factor which may be brought to bear on the GUI determination is the cause, if known, of a rise or decrease in blood glucose level. In this regard it is noted that some changes in glucose level are brought on by stress and others by food intake. Such data may be preprocessed or pre-associated prior to its input into the urgency assessment module, or may be associated therein. For example, food data may be processed in combination with glucose levels to determine whether a glucose rise resulted from food or from another cause, such as stress. Using such data, problems seen in the past with a lack of consideration of such causes and effects may be effectively addressed.

As noted above glucose values (and derivative data) may be weighted by the assessment module based on signal quality, confidence level, and the like. Such would generally be performed automatically, by the electronic device, based on analysis of the signal data from the sensor electronics. However, any of the above variables or parameters could enter the GUI calculation in a weighted fashion, where the weighting is performed automatically, e.g., by signal analysis from the underlying sensor, e.g., accelerometer, weight scale, etc., or by using data entered manually from, e.g., a physician or the patient. Using such data, problems seen in the past with a lack of consideration of such factors may be effectively addressed.

A summary of the described types of data is provided in Table I below. Note that certain parameters and variables occur in more than one data category.

Table I

| DATA CATEGORY | SUBCATEGORY | PARAMETER OR VARIABLE |
|---|---|---|
| Current Measured Glucose Value | | Current measured glucose value, e.g., data of a first type |
| Historic Measured Glucose Values, e.g., data of a second type | Recent Historic Measured Glucose Values, and data derived from recent historic measured values | First derivative of recent glucose values, e.g., velocity (sign and amplitude / rate of change of glucose concentration) |
| | | Second derivative of recent glucose values, e.g., acceleration of glucose concentration |
| | | Higher order derivatives of recent glucose values |
| | | Glucose trace over last *x* hours, e.g., 6 hours |
| | | Level of last glucose peak, level/duration of last significant glucose value excursion, e.g., recent local maxima or minima in glucose levels |
| | | Predicted values or ranges of glucose data |
| | | Weighting of inputs based on calculated accuracy, confidence level, or noise information, e.g., based on quality of glucose data |
| | | Other data, including processed glucose sensor data, e.g., duration of levels of values (stable or increasing/decreasing) |
| | | Recent (or last) events, e.g., significant events (e.g., within the last 24 hours), which may indicate likelihood of future events. |
| | | Amount of filtering, smoothing, and other data processing, especially with respect to lag |
| | | Duration of current urgency state |
| | Older Historic Measured Glucose Values | Patient pattern data |
| | | Deviation from normal glucose patterns (similar to time of day, but not necessarily time-based) |
| | | Anthropometric data |

(continued)

| DATA CATEGORY | SUBCATEGORY | PARAMETER OR VARIABLE |
|---|---|---|
| External Data, e.g., data of a third type | Data from other sensors / sources | Activity level, e.g., determined or calculated by GPS or an accelerometer within the mobile device, e.g., sleep or exercise |
| | | Physiological data, e.g., heart rate, temperature, blood pressure, hydration |
| | | Food data received from an app running on a mobile device - patient enters data in the app or app predicts data based on historical data, e.g., what the patient has at a given restaurant before, the identity of the restaurant determined by a user check in, GPS, and so on. Food data may also be entered via the mobile device camera. |
| | | Insulin information, such as from a pump or EMR in cloud |
| | | Context / behavior data |
| | | Time of day and patient pattern data |
| | | Level of user interaction |
| | | Age, gender |
| | Data input by user | Weighting of any of the inputs - a user may also weight the measured inputs |
| | | Patient indication of activity level, e.g., exercise |
| | | Patient indication of food/drink ingested |
| | | Anthropometric data |
| | | Insulin information |
| | | Stress level |
| | | Current health |
| | | Age, gender |
| | | Sensor site location |

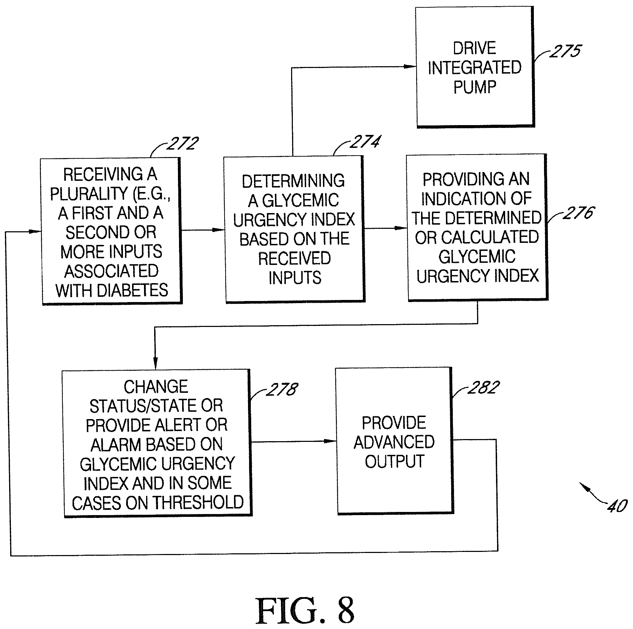

Figure 8 illustrates a flowchart 40 illustrating general use of the parameters and variables discussed above. In a first step, a plurality of inputs are received associated with a malady such as diabetes, the inputs corresponding to variables or parameters, which may be measured, may be entered by a user, or otherwise obtained, e.g., via the cloud or other source (step 272). A GUI is then calculated based on the received inputs (step 274). The GUI may be determined or calculated in a number of ways as will be described below. A next step is to provide an indication of the GUI (step 276), such as an output. Alternatively, or in combination, the status of the GUI may change, or an alert or alarm may be provided, if the GUI reaches a particular value or based on a threshold (step 278). Similarly, various types of advanced outputs (additional processing or additional detail regarding the GUI processing (e.g., information about inputs) may also be provided (step 282). In some implementations, the determined GUI may serve to drive an integrated pump for a medicament (step 275), as will be described in greater detail below.

A number of variations will be understood. For example, notifications, indications, alerts, or alarms, as well as advanced outputs, may be provided to the patient or to another user, e.g., caregiver, physician, family member, or the like. Generally, an indication or notifier of user status is available and provided. In more urgent cases, an alert or alarm may be provided to the user so that the same may take appropriate action. In addition, not all of these need be provided to a user in a given circumstance. In some cases, a user will simply desire to review a user interface of their mobile device to ascertain their status, and in this case an indication is provided even without the occurrence of an alert, alarm, or advanced output. In related cases, only advanced outputs may be desired by a user. In other cases, an alert or alarm may be provided without providing a particular general indication of status, so as to avoid distracting a user when the important information is the alert or alarm. Other variations will also be understood.

Example 1

In one exemplary implementation, the several inputs are used in the determination of a GUI and include at least: a) glucose value (concentration); b) velocity (amplitude and/or direction) of glucose value, i.e., its rate of change; c) acceleration (amplitude and/or direction) of glucose concentration; and a duration of one or more of the above. For example, a first input may be the glucose value, a second input may be a derivative of the glucose value, and a third input may be a duration or other parameter or variable described. In this implementation, an initial notification based on glucose value may be modulated up or down and/or recalculated using a GUI function) based on the derivatives and/or duration of any input. For example, a glucose value may be low or high, but if the same is tending towards a desirable intermediate value, as determined by the first derivative, the base alert may be suppressed because the GUI is determined to be at a no or low risk state. The alert may be further suppressed if the second derivative indicates that the glucose value is not increasing or decreasing in a way tending away from the intermediate value (and vice versa), as determined by the GUI. In alternative implementations, the suppression may be replaced by a recalculation of the GUI that results in a no or low risk state.

Example 1 solves the problem whereby if a "turn around" event is about to occur, the rate of change information alone (the first derivative) may not be able to accurately predict where the glucose value will resolve in the long term. By employing the acceleration information, "turn around" events may be better anticipated so as to avoid over corrections or false alerts.

In a specific implementation, e.g., at 0 mg/dL/min/min (no acceleration or deceleration detected), the urgency assessment module may be able to rely solely on the first and second inputs for glycemic urgency index determination. However, at 1 or 2 mg/dL/min/min, the urgency assessment module may rely in addition on other inputs, including acceleration, to determine the type of "turn around" event, as well as the likely effects thereof.

Example 2

In another exemplary implementation, the first input is the same as Example 1, the second input is the rate of increase or decrease, and the third input is the acceleration. Other inputs may also be considered in the determination of the GUI, including other parameters and variables selected from Table 1.

Example 3

In yet another exemplary implementation, the first input is the same as Example 1, the second input is the velocity or rate of change of the glucose value, and the third input is another parameter or variable selected from Table 1.

Example 4

In yet another exemplary implementation, the first input is the same as Example 1, the second input is the acceleration (which may or may not involve a calculation of the velocity), and the third input is another parameter or variable selected from Table I.

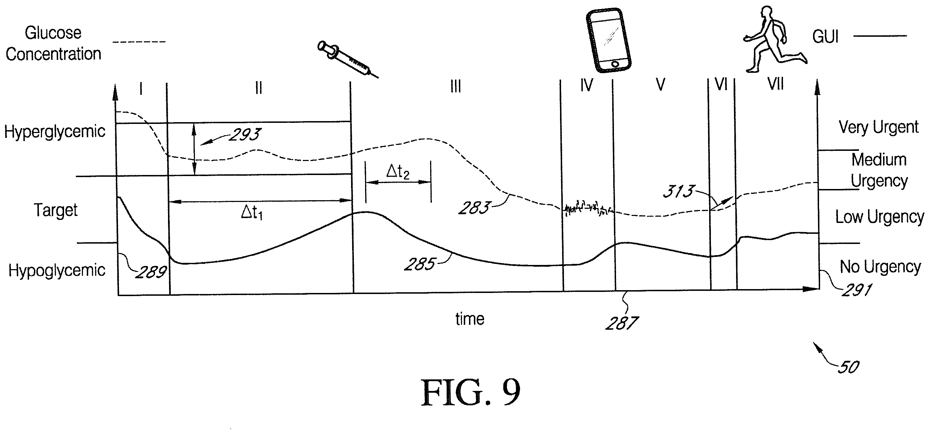

In another specific implementation of the examples, and referring to the graph 50 of Figure 9, a trace 283 of glucose values (applied to axis 289) and a trace 285 of GUI values (applied to axis 291) are illustrated plotted against a time axis 287. As may be seen, in Region 1 a user is initially in a hyperglycemic state, and has a GUI in the low to medium range. Here another type of GUI is illustrated, ranging from zero (low urgency) to higher values (indicating higher urgencies). By consideration of the rate of change of the glucose value, however, which is tending towards the target range, the GUI and thus the urgency assessment may be lowered towards a "no urgency" zone or band. If the glucose value had a positive rate of change, rather than a negative one, or had an acceleration indicating a tendency toward higher values, then the GUI would rise toward a more urgent assessment, even if the glucose value itself was decreasing.

In Region II, the glucose value is seen to be occupying a range of hyperglycemic values (e.g., 180-400 mg/dL) 293 over a period of time $\Delta t_1$. If the time $\Delta t_1$ exceeds a predetermined threshold, and the case of Figure 9 assumes so, then such may indicate a reason for the GUI to rise, even though the user is only mildly hyperglycemic or has not experienced a further increase in their glucose value. Region II of Figure 9 illustrates a hyperglycemic range, and it will be understood that occupation of hypoglycemic ranges similarly raises GUI values, particularly as durations in which a user occupies a hypoglycemic range are associated with likely further hypoglycemic excursions.

In more detail, the duration of the inputs may also be employed in the determination of a GUI by the urgency assessment module. In particular, the longer the duration of a hypoglycemic or hyperglycemic excursion, the greater impact the excursion may have on the GUI. For example, staying at a high glucose state (e.g., above 180 mg/dL) for two hours is more dangerous than staying at the same high glucose level for 20 minutes, at least in terms of long-term complications associated with diabetes. Moreover, glucose levels become logarithmically more risky above 180 mg/dL.

Similarly, staying at a low glucose level (e.g., below 70 mg/dL) for two hours can be more dangerous than staying at the same low glucose level for only 20 minutes, at least in terms of increasing the likelihood that small changes could easily put the user at a dangerously low state. In other words, as time passes at a low glucose level, it becomes more likely and easy to drop to a dangerously low glucose level, e.g., below 55 mg/dL.

By tracking the duration or amount of time spent, e.g., below a threshold for a particular event or time period, a glycemic urgency state or index can be effectively modified or refined to more accurately reflect the risk and clinical significance to the user.

Referring back to Figure 9, Region III indicates a time period at the beginning of which a user has indicated (by entering data into the electronic device) a mitigating factor in the hyperglycemic event, e.g., injection of a bolus of insulin. For example, the user has entered data in response to a prompt by the urgency assessment module or not, indicating a bolus has been provided (an integrated pump may also provide such data). The urgency assessment module may immediately cause a decrease in the GUI, and such may occur long before a decrease is actually seen in the glucose concentration. In the case of Figure 9, such a delay is indicated by a time $\Delta t_2$.

By consideration of parameters and variables beyond just glucose values, and even beyond consideration of merely increases or decreases in the same, notifications including continuous notifications, alerts, and alarms may be more finely tuned to a given user using the systems and methods according to present principles, thus providing a number of advantages, including the reduction of nuisance alarms. For example, using the above system, if the threshold is set at 70, but a user is at 69 and rising, prior systems would continue to alert because the user was still below the threshold. Systems and methods according to current principles recognize that the user has a rising glucose level and thus need not be alerted in the first place. Even if the user was not measured as rising, but has indicated they have just ingested a meal, systems and methods according to present principles may avoid nuisance alerts or alarms that would otherwise be triggered as the same recognize that the user will soon have a rising glucose level based on the GUI.

Region IV indicates a region in which the glucose value has, for one reason or another, become noisy, and thus a low confidence level may be associated with that section of the signal. Accordingly, the GUI may be seen to rise, in recognition that a glucose value with low confidence is associated with an urgency assessment that is higher or more urgent. A similar increase in urgency assessment would occur upon a determination that a significant lag had occurred in the signal.

Region V illustrates another parameter or variable which may bear on the determination of the GUI. In particular, it is assumed in Region V that a user has become highly interactive with their mobile device, as determined by a significant number of key presses, touch screen activations, motion as determined by an accelerometer, or the like. Accordingly, as the user has been determined to be more interactive with their device, and thus is more likely to see urgency assessment updates, alerts, and alarms, the GUI and urgency assessment may decrease, as the user is more likely to be able to quickly take action.

Region VI indicates a situation in which a predicted value 313 of glucose level indicates a rise in the same, such prediction being calculated by predictive analytics tools as described above. In this case, the same may cause an increase in the GUI, in recognition of an expectation of a rise in blood glucose level. Such predictions also may have the advantage of compensating for lags in glucose levels.

Region VII indicates another situation in which a rising glucose level need not necessarily significantly increase the urgency assessment, based on user entered data. In particular, Region VII indicates the user is heading towards a mildly hyperglycemic state. However, where the user has recently entered data indicating he or she is about to perform significant physical activity such as exercise, the tendency towards a mildly hyperglycemic state may be counteracted by the assumed effects of the exercise. Accordingly, the GUI 285 in Region VII need not rise significantly.

Variations will be understood. For example, while a GUI axis 291 has been employed with just one direction, a GUI axis may be employed with two directions (not shown), indicating a hyperglycemic urgency and hypoglycemic urgency. Where a single GUI axis 291 is employed, to disambiguate the type of urgency, and thus to provide a notification or an actionable alert, the algorithm providing the same is aware of the glucose value and other variables and parameters constituting the determination of the GUI, and thus the notification or actionable alert displayed takes into account whether the user is hyperglycemic or hypoglycemic.

Moreover, while in some instances a user may view a trace 285 indicating the GUI, or may see a numerical index representing the same, it is understood that most notifications or actionable alerts will provide an indication of the GUI in another way, such as by the use of color, icons, or the like, as described in greater detail below. in other words, many users do not need to see the GUI itself, but rather what it represents.

It will be understood that Figure 9 is intended to encapsulate in a condensed fashion a number of different types of variables and parameters, and to indicate their effect on the determined GUI, but that in any given implementation not all such parameters and variables need be monitored or employed in the determination.

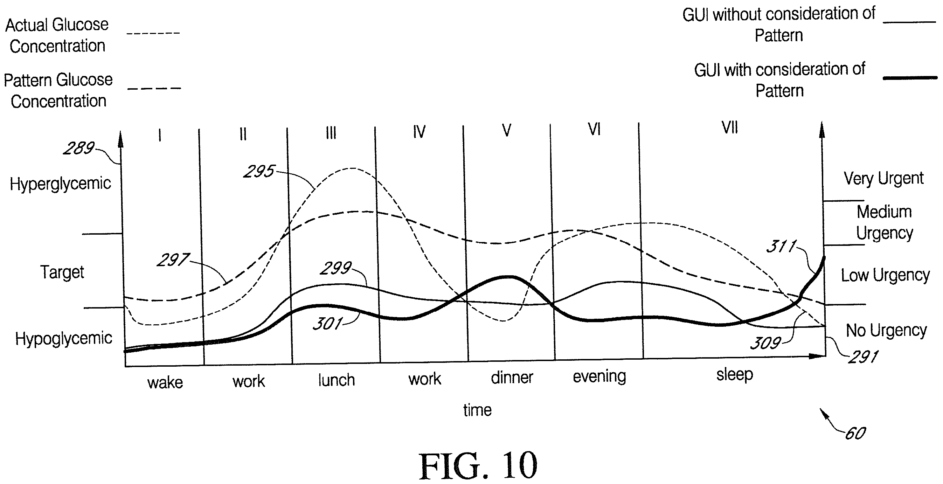

Figure 10 illustrates another graph 60 encapsulating a number of different types of variables and parameters, indicating their effect on the determined GUI. As with Figure 9, not all such parameters and variables need be monitored or employed in the determination. Moreover, parameters and variables depicted in Figure 9 may be combined with those depicted in Figure 10 in any number of ways.

In Figure 10, the time axis has been divided into a number of different sections pertaining to a typical day. An actual glucose concentration 295 is plotted superimposed above a determined or calculated pattern of a glucose concentration 297 for a given user. The pattern glucose concentration may be developed using historical glucose values, and may be time-based or relative, e.g., keyed to an event such as a meal intake, exercise, insulin bolus, or the like. Other patterns will also be understood, including other non-time-based ones. Two types of GUIs are also illustrated on Figure 10. A GUI 299 is shown that does not include consideration of the pattern. In particular, the GUI 299 may be based on the glucose value as well as on other factors noted above, e.g., the rate of change of glucose value, the acceleration, and the like, but is otherwise "absolute" in the sense that it is not pattern-based. A GUI 301 is also illustrated that takes into account the pattern 297. In particular, where a hyperglycemic or hypoglycemic event has been recognized as part of a pattern, the GUI may not increase, i.e., the urgency assessment may stay the same, or only slightly change, in recognition of the fact that the increase or decrease is part of an established pattern. For example, a glucose drop XXX during dinner as shown in section V, which is not correlated with the host's normal glucose profile (pattern), causes an increase in the GUI (unless meal information was input into the GUI).

Figure 10 also illustrates a deviation of a glucose level from an established pattern, in particular an uncharacteristic decrease 309 in glucose value during sleep. As mentioned above, during sleep, hypoglycemic events are often undetected and thus particularly serious. Accordingly, a rise 311 in the GUI may be employed to increase the urgency assessment to alert or alarm the user in such situations.

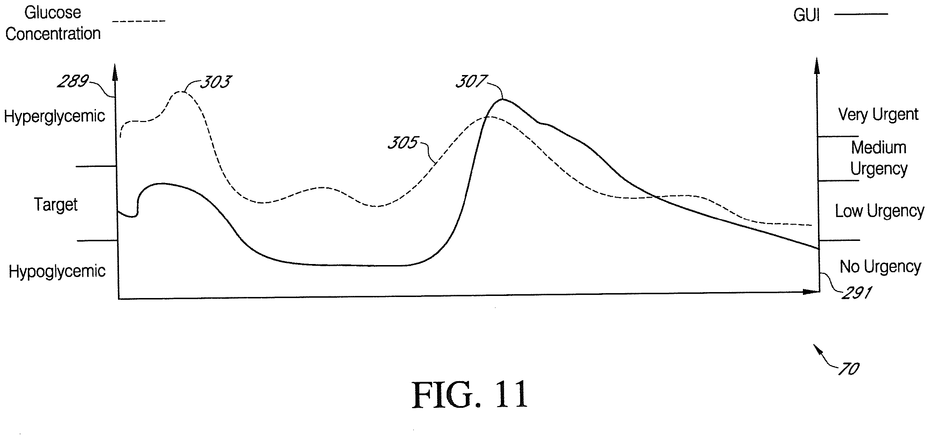

Figure 11 shows a graph 70 illustrating the effect of a prior significant glucose excursion. In particular, a significantly hyperglycemic event 303 is illustrated in the glucose concentration. And the same appears in the graph 70 to have been resolved over time. However, a subsequent rise 305 may be seen, and instead of simply causing a mild increase in the GUI, the same may lead to a spike 307 in the GUI, as the prior significant hyperglycemic excursion may be assumed to be potentially repeating itself (rebounding). Accordingly, the urgency assessment is greater than it would be simply based on glucose value alone.

Other examples will also be understood. For example, a user with an otherwise "low risk" urgency assessment, e.g., based on glucose value and rate of change, may be assigned a higher risk urgency assessment if they are overweight or have a high BMI, high blood pressure, high stress, are dehydrated, or the like. Aspects such as body temperature, anthropometric data, and illness, as well as demographic data, may further modify the GUI, as may contextual and behavioral information. Sensor location may also modify the determined GUI. For example, a user may have a low GUI and thus a low risk urgency assessment, but if the sensor location is such that a significant lag is expected in glucose values, the GUI and urgency assessment may be raised to reflect a lack of confidence in the current measured glucose level.

Using the principles described above, a glycemic risk state in the form of a glycemic urgency index may be calculated based on input parameters and variables using a mathematical approach. The output can be one of a plurality of predefined glycemic states, or the output may be qualitative or quantitative, e.g., in terms of percentages or a number. For example, the output may be a GUI = 1, 2, 3, and so on, or where such numbers are translated into terms which may be more easily understood by a user. The output may further be categorized such as hypo/hyper/euglycemic, or further categorized such as current or predicted, regular or irregular, or in other ways as may be particularly useful to a given user. Names and labels may be applied that include indicators of the influencing real-time events such as "exercise-induced", "turn around", "extended duration" or the like.

Other user interfaces could provide more detail about certain glycemic risk states.

For example, if a user glucose level has been below a predetermined threshold for more than 15 to 20 minutes, even after having ingested carbohydrates, such a situation may be presented on the user interface of the device. In this way, the user is made aware of a significant duration in which a low glucose value has been ineffectively remedied.

As another example, a user glucose value may be above a predetermined threshold, but predicted to go below in the near future. In this case, a predicted value of glucose, e.g., over a 20 minute prediction horizon, gives the user a useful and actionable alert, allowing an unambiguous action (or group of actions) to be taken.

In another example, a user glucose level may be above a predetermined threshold for a long duration. In this case, showing how long the user has been above the threshold helps in alerting the user to the seriousness of the situation.

In yet another example, a user glucose level may be above a predetermined threshold for a long time and not falling. In this case, showing the duration of the elevated value, as well as a rate indicating a lack of return to euglycemia, provide significant and actionable information to the user.

Analysis Frameworks

Several mathematical frameworks and inputs can be employed to determine a user's risk state of hypoglycemia and hyperglycemia. One example of how to estimate a user's risk state is described below, which employs parameters and variables including the current glucose level, the current glucose rate of change, and the glucose change direction to provide a risk value. In certain implementations of systems and methods according to present principles, glucose acceleration, as well as duration of time in a hypoglycemic or hyperglycemic state are added as inputs to arrive at a GUI.

Previously, static and dynamic functions have been proposed, which are mathematical models that map glucose levels and glucose rates of change to a risk function (e.g., from 0-100). For example, Kovatchev ("Risk Analysis of Blood Glucose Data: A Quantitative Approach to Optimizing the Control of Insulin-Dependent Diabetes", Journal of Theoretical Medicine, Vol. 3, pp. 1-10 (2000), incorporated by reference herein) described a static risk function that mapped glucose concentration to a static risk value where extreme hypoglycemia and hyperglycemia would both have a level of 100. Similarly, Guerra ("A Dynamic Risk Measure from Continuous Glucose Monitoring Data", Diabetes Technology & Therapeutics, Vol. 13 (8) (2011), incorporated by reference herein) described a dynamic risk function that mapped glucose concentration and rate of change of glucose concentration to a dynamic risk value by scaling the static risk numbers based on rate of change information. The present implementation can build on the static and dynamic risk functions of Kovatchev and Guerra with additional inputs.

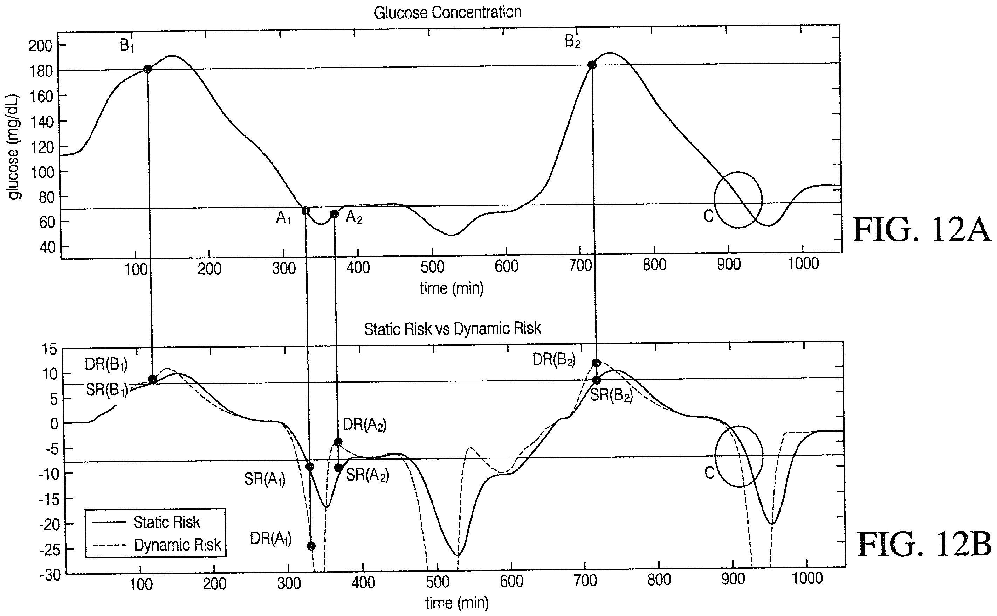

Other inputs that may contribute to a user's risk state include acceleration or duration in hypoglycemic or hyperglycemic states, as well as the duration of a given velocity or acceleration in blood glucose levels. An example is shown in Figures 12A and 12B of a subject's risk increasing as they remain above 180 mg/dL for long periods of time.

$$GUI\left(g, \frac{dg}{dt}, \Delta T\right) = \begin{cases} SR(g) \times e^{+\mu\frac{dr}{dt}} \times e^{+\delta\Delta T} & if \quad SR(g) > 0 \\ SR(g) \times e^{-\mu\frac{dr}{dt}} \times e^{+\delta\Delta T} & if \quad SR(g) < 0 \end{cases} \quad (7)$$

Where $SR(g) = r_h(g) - r_l(g)$

where $r_l(g) = r(g)$ if $f(g) < 0$ and 0 otherwise, and

where $r_h(g) = r(g)$ if $f(g) > 0$ and 0 otherwise,

and where g is the glucose concentration, $\frac{dg}{dt}$ is the rate of change of glucose concentration, $\Delta T$ is the time above 180 mg/dL or the time below 70 mg/dL in hours, and $\delta$ is a tunable parameter for how much weight to give duration in a risky state.

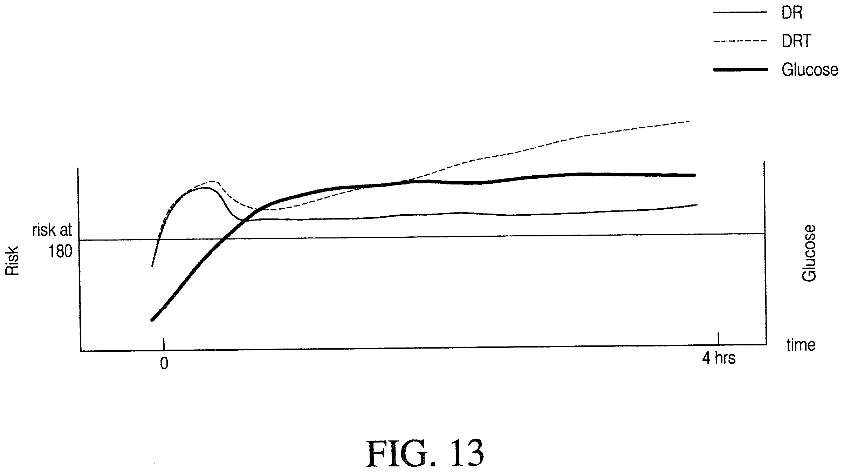

Figure 13 provides a graphical illustration of increasing risk to health state with duration in hyperglycemia. Referring to the figure, a user's glucose level is illustrated as being hyperglycemic but not continuing to increase. As the duration increases, however, the figure shows that the risk continues to increase over time.

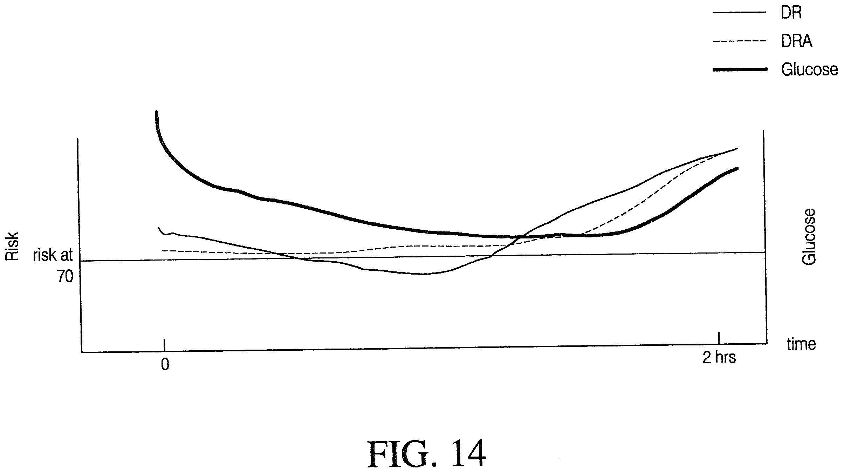

Figure 14 gives an example of avoiding a false risk state by using acceleration as a parameter or variable in the determination of a GUI. In this figure, the acceleration or second time derivative indicates that the glucose value, while decreasing, is also in the process of turning around and heading back in the direction of a euglycemic state. A continued rise, however, may lead to an increased risk assessment and thus GUI due to the potential of a hyperglycemic event.

Equation (8) below describes the situation of Figure 14.

$$DRA\left(g, \frac{dg}{dt}, A\right) = \begin{cases} SR(g) \times e^{+\mu\frac{dr}{dt}} \times e^{+\sigma A} & if \quad SR(g) > 0 \\ SR(g) \times e^{-\mu\frac{dr}{dt}} \times e^{-\sigma A} & if \quad SR(g) < 0 \end{cases} \quad (8)$$

Where A represents the acceleration of glucose in mg/dL/min and $\sigma$ is a tunable parameter for how much weight to give acceleration and risky states.

Other functionality may also be brought to bear in the analysis framework. For example, adaptive learning may be applied, which is broadly described in US Provisional Patent Application Serial Number 61/898,300, filed October 31, 2013, entitled "ADAPTIVE INTERFACE FOR CONTINUOUS MONITORING DEVICES", and US Application Serial Number 13/827,119 filed March 14, 2013, entitled "ADVANCED CALIBRATION FOR ANALYTE SENSORS", owned by the assignee of the present application and herein incorporated by reference in its entirety. In one application of adaptive learning, the monitoring device, e.g., mobile device, may adaptively learn aspects of users over time as they experience hypoglycemic and hyperglycemic events. For example, every time the user goes below 55 mg/dL, the data preceding that event can be used by a machine learning algorithm, e.g., a support vector machine (SVM) or linear discriminant analysis (LDA), as a positive test case. In addition, cases where the user's glucose level remained between 70 and 110 can be used by the machine learning algorithm as negative test cases. The machine learning algorithm could perform periodic or occasional training, e.g., every month, to optimize classification of near-term hypoglycemia. For example, the algorithm could learn the state one hour or one half hour before a hypoglycemic event for that particular user. Exemplary inputs to the machine learning algorithm to be used in classification could include: glucose trace over last 6 hours, current glucose level, current rate of change of glucose level, current glucose acceleration, time of last insulin bolus, size of last insulin bolus, number of carbohydrates declared, time of carbohydrate declaration, level of last glucose peak, last time user interacted with monitoring device, time last exercised, time of day, time between insulin bolus and meal peak, and the like. Once the classifier is optimized, the same can be applied on data in real time to determine whether or not hypoglycemia is likely within some perspective time window.

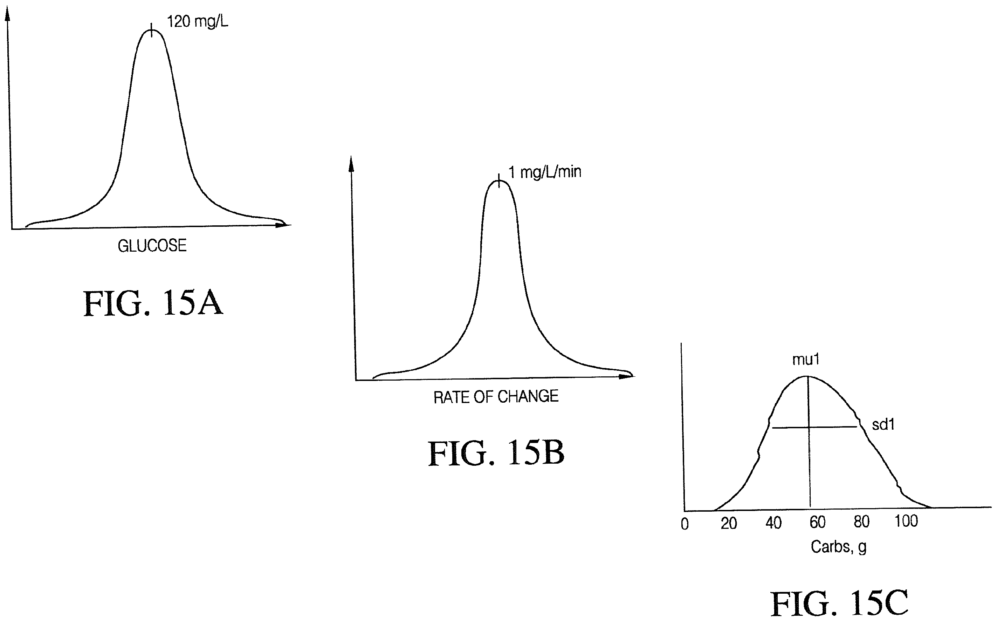

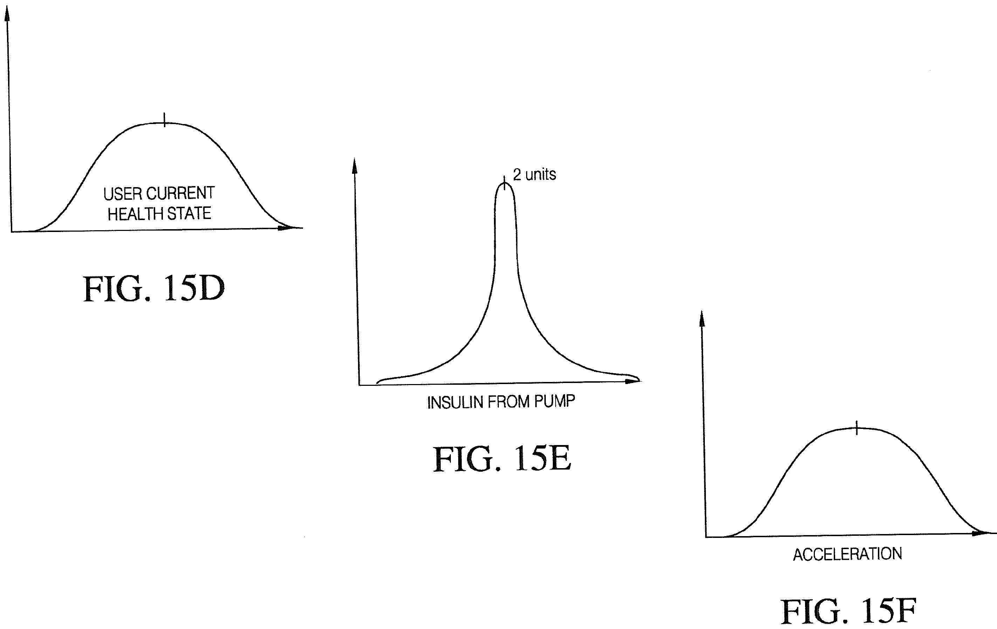

Another type of functionality which may be employed uses a Bayesian approach. Such provides probabilistic ways of quantifying the risk of an event on a particular day or night, based on prior distributions. Figures 15A-15F are exemplary distributions applied to a plurality of GUI inputs, for example: (A) a distribution applied to the glucose concentration based on prior reliability information ; (B) a distribution of the rate of change value based on noise in the data and/or the amplitude of the rate of change; (C) a distribution of the carbohydrate information, entered by the user, based on prior knowledge of carbohydrate estimations by the user; (D) a distribution of user's current health state; (E) a distribution of insulin on board based on integrated pump data; and (F) a distribution of acceleration data, for example. Distributions may be employed for any of the inputs, and probability algorithms employed to determine risk index therefrom. Such probability algorithms may include joint probability algorithms or more complex analyses such as involving Bayesian statistics.

Yet another type of functionality that may be employed involves "Decision Fusion" methods. In particular, decision fusion provides another framework to determine a user's risk state from multiple inputs. Decision fusion uses a statistical model to optimally combine risk information from multiple inputs and produces a likelihood value that some event will occur, like hypoglycemia. Such methods are particularly useful in combining heterogeneous inputs, like glucose rate of change and number of receiver button presses of the last 20 minutes, into a single likelihood scale. Prior information on the sensitivity and specificity of each input in predicting the undesired event, e.g., hypoglycemia, is used to determine how much weight to give each input in the final risk output, as is described in greater detail below.

Decision fusion methods may be employed to, e.g., determine whether a given user is likely to be below 55 mg/dL within the next hour. In such methods, different data parameters can be used to make decisions about whether hypoglycemia will occur in a given time period, e.g., within the next hour. Data analysis may be performed to determine the optimal detection parameters and their optimal yes/no decision thresholds, as well as associated sensitivity.

Exemplary parameters that can be used for making decisions on whether glucose levels are likely to go below 55 mg/dL within the next hour are shown in Table II below, along with their thresholds for decisions and the associated sensitivities and specificities. Note that while some parameters may be very sensitive (e.g., glucose values will always be below 80 mg/dL prior to going below 55 mg/dL), they may not be very specific, i.e., there may be lots of occurrences where glucose goes below 80 mg/dL but then does not go below 55 mg/dL within the next hour. The best predictors generally have both high sensitivity and specificity, such as predicted glucose level is below 55 mg/dL.

TABLE II

| PREDICTOR | SENSITIVITY | SPECIFICITY |
|---|---|---|
| Insulin bolus within past 1.5 hours | 80% | 10% |
| Last carbohydrate declaration more than a half-hour | 80% | 10% |
| Glucose level predicted in 20 minutes is less than 55 mg/dL | 90% | 70% |
| Acceleration < 0 mg/dL/min$^2$ | 50% | 5% |
| Exercise within the past six hours | 50% | 10% |
| Current glucose rate of change < -2 mg/dL/min | 60% | 10% |
| Current glucose level < 80 mg/dL | 100% | 30% |
| Last user interaction with CGM > 30 minutes ago | 80% | 5% |

In real time each parameter can be compared to its threshold and a yes/no decision made about whether or not a hypoglycemic event is about to occur.

In this analysis, a true case of "yes" (hypoglycemia) is given as Ht, and a true case of "no" (normoglycemia) is given as H0, i.e. the null hypothesis. Decisions are made for each parameter (d = 1 or d = 0) and the parameter's sensitivity and specificity are used to convert each decision to a likelihood value, $\lambda$

$$\lambda(d) = \frac{P(d \mid H_1)}{P(d \mid H_0)}$$

The likelihood value is the probability of making the decision in the case that the subject really will become hypoglycemic, divided by the probability of making the decision in the case that the subject will not become hypoglycemic. For a decision of "yes" or 1, the likelihood value may be thought of as the sensitivity divided by (1 - the specificity), i.e., the probability of a false alarm.

$$\lambda(d) = \frac{P(d \mid H_1)}{P(d \mid H_0)} = \begin{cases} \dfrac{Sensitivity}{1 - Specificity} & \text{if } d = 1 \\ \dfrac{1 - Sensitivity}{Specificity} & \text{if } d = 0 \end{cases}$$

For a test with a high sensitivity and a high specificity, λ will be very high for a decision of 1 and very small for a decision of 0. This weighting of each decision based on test performance thus enters the calculation. Once each decision is converted to a likelihood value, all the likelihood values may be simply multiplied together. The final likelihood value is then a range where low numbers mean that hypoglycemia is very unlikely to happen and high numbers mean that hypoglycemia is very likely to happen. These likelihood numbers can be used to inform the GUI as an input to present risk or urgency to a user.

Heuristic techniques are yet another sort of mathematical method that may be applied to the analysis framework. In such techniques, experience informs the development of potential solutions. For example, the urgency assessment module may have experiential data that implies that when a given user is at a given set of GPS coordinates, which happens to be a coffee bar, and wherein the user further has a staff meeting planned for the following day, the user usually has a high glucose value. The glucose value may be stress-related or eating related. In the development of such heuristic solutions, related techniques may be employed such as: MPC, if/then logic, expert systems, logistic regression, neural networks, fuzzy logic, weighted functions (were weighting may be applied to the riskier glycemic risk input), as well using regression models. As a specific example of the use of regression models, A1C may be assumed as a current indicator of good/bad diabetes management. Several or numerous parameters or variables as disclosed herein may be obtained from a statistically significant number of users and a regression analysis may be run to determine which of those factors affects A1C significantly. The resulting number multiples may then be adjusted to represent a risk score that is easily interpretable, e.g., on a 1-100 scale.

Thus, the GUI may be calculated in a number of ways, and by use of a number of different variables and parameters, some of which are measured and others of which are entered by a patient or other user. However the GUI is calculated, the same may then be employed to dynamically provide an indication to the patient (or caregiver) of a glycemic urgency state, and may iteratively update the urgency state over time. While the urgency state may be associated with hypoglycemia, hyperglycemia, or euglycemia, the same provides far more sophisticated information than simply whether a glucose value (or predicted glucose value) has passed a threshold. The indication to the user is generally a graphical indicator of urgency, and may advantageously employ native features on a mobile device such as a smart phone. The user interface of the smart phone may also be employed to provide alerts/alarms to the user.

The additional information provided, which is not provided in systems that simply indicate glucose values passing thresholds, may include one or more of the following. The displayed indicated information can include a prediction as to whether the glucose value will likely rebound to a desired value or will continue an excursion away from euglycemia. The displayed information can include (or take account of) predictions as to future urgency states, as distinguished from simply providing a predicted glucose value. The displayed information can include consideration of whether the glucose value is following a known pattern, or whether it is straying from a known pattern.

The urgency assessment module running on the mobile device (or elsewhere) can provide a framework to distinguish levels of danger and urgencies of action that a simple threshold cannot, thus providing actionable alerts to a user but not over-alerting the user when not necessary, thus preventing alert fatigue. In this regard it is noted that when smart phones are used for glucose monitoring, it becomes increasingly important to distinguish which alerts are critical for users to take notice of, as users continuously receive notifications from their phone for, e.g., e-mails, texts, phone calls, applications, and the like. It is important that especially dangerous conditions that require immediate attention be differentiated from such general cell phone noise, perhaps by reserving special sounds or vibrations or even lights/colors to alert the user. Furthermore, it is important that alerts be escalated in prominence if the user does not respond immediately, and the parameters and variables described above can be employed to determine when such escalations should occur. The indications may be provided in a number of ways, such as using color, vibration, icons, heat maps, predictive representations, a representation of risk as a number, voice prompts, pop-up messages, and the like.

In some cases it is desirable for a user to be alerted, but in a discrete fashion. Such may be particularly appropriate if a user is currently in the company of those unaware of the user's condition. Accordingly, an alert may be provided that is a vibration, but an especially long vibration which can alert the user to examine their mobile device, to determine what action is necessary. A separate vibration may be used for alarms, e.g., a long vibration but one which is applied periodically until stopped by the user, with a period of, e.g., 5 seconds, 10 seconds, and so on, and a frequency of once per minute, twice per minute, and so on.

If vibration based alerts and alarms do not result in an action by the user, at least by a user pushing a button to indicate recognition of the same, then the alert or alarm may be given audibly, by a ring tone or the like, including a ring tone chosen especially for such a condition.

If neither the vibration or the audible sound causes user interaction, an automated phone call or text message may be placed to an alternate number, a physician or family member may be alerted, or the like.

Assuming the user does respond to the alert or alarm, an initial typical response may be to pick up or otherwise handle their smart phone. In some cases the user must perform a swiping action or enter a code to enable access to the user interface of the smart phone. An indication of the urgency state may be provided on the user interface in a number of ways, given this scenario. First, the indication may be given whether or not the phone is handled. This may be appropriate in the case where a user leaves their phone in a position where the same may be used, e.g., face up on their desk, in a docking station, or the like (e.g., background screen, home screen, periodic push notification, etc). Second, the indication may be given upon the signal being received by the user interface that the phone is being handled, e.g., via an onboard accelerometer or other detector of motion. Third, the indication may be given after access to the smart phone user interface is gained, e.g., via the swiping motion, the entering of the code, or the like.

For the first and second ways, the indication given on the user interface may include somewhat less information than with the third, in recognition of the fact that others may be able to view the indication as well. For the third, where the user has already picked up the phone and performed actions with the same, it may be presumed that the user has obtained the desired level of privacy. Thus, an indication in the first and second ways may be, e.g., a color or other indicator, which the user may become familiar with, indicating the urgency state. For example, a bright red color on the user interface may be indicative of high urgency, e.g., a coming hypoglycemic or hyperglycemic event. The red color may be arranged by the urgency assessment module by manipulation of the background or wallpaper of the mobile device, or the same may be a background of an application running the urgency assessment module, e.g., a CGM application, this background and application being arranged to be prominent at times of elevated urgency.

In another implementation, the color may be accompanied by a number or an arrow, such announcing additional details about whether the GUI is assessing a significant risk of a hyperglycemic event versus a hypoglycemic event. Such may provide useful information to a user while still keeping discreet specific details of the user's condition.

No matter how the indication occurs, by user operation of the application, the user can become aware of pertinent details of their condition, as well as potential steps to take in mediation thereof. In other words, a user can decide if they wish to "dig into" the numbers in more detail, in which case operation of the application would allow them to do so. Variations in the above example will also be seen. For example, instead of a red screen, a red border around a user's home screen may be employed, or a large red circle. Different colors or positions may be employed to indicate hyperglycemia versus hypoglycemia, or the user may be required to instantiate the application in order to find out their current condition. Different positions or crosshatching may be advantageously employed as an option for users who are colorblind.

A number of types of features employable on mobile device user interfaces will now be described for indicating actionable alerts based on GUI values. It will be understood that such user interfaces are purely exemplary, and other such user interfaces are also possible. The user interfaces show various levels of information, and in particular may show a glycemic urgency index and glycemic or glucose information, in some cases together on the screen. The indications of GUI values may be made in a number of ways, such as the use of visualizations or representations, such as colors or elements such as icons. While certain icons are discussed below, it will be understood that the same may vary in numerous ways, e.g., the same may be portrayed by a happy face (euglycemic) or a sad face (hypoglycemic or hyperglycemic), a comic book hero (low urgency or risk assessment) or a comic book villain (high urgency or risk assessment), a depiction of a meal (hypoglycemic) or a syringe or pump (hyperglycemic), a numerical value, and so on.

A concept of "reads" or the like may be employed, where "reads" are defined as levels of information communicated to a user, also known as information hierarchy, which is the arrangement of elements or content on a screen in such a way that it reveals an order of importance. Reads can consist of anything including typography, graphics, colors, contrast, weight, position, size and space (including negative space). The reads are presented to accomplish the order of importance or actionability. A first read may be the first item a user sees or is shown (most visible or noticeable). If there is only one level of information shown, there need not be a second read. However, devices can have multiple levels or reads. A device that has a number in large text showing in bright red font and then a smaller number showing in a lighter font would have two reads. The large number is the first read (what the user sees first) and then the smaller number is the second read (what the user sees or notices or wants to see less frequently, as compared to the first read). In general, the first read provides lower resolution information, although more actionable information or glanceable information, such as a representation indicative of GUI, a glycemic state or glucose level, which may be read with a quick glance. In general, the second read provides high resolution (more detailed) information, which may be read with a longer gaze or requiring longer thought processes. Additional reads can be provided with more or different levels of detail, as may be appreciated by one skilled in the art. In some implementations, the first read is provided on a first viewable screen (e.g., background or home screen of a mobile device or software app) and the second read is provided on the same screen in a configuration and position that is less glanceable or easily readable. In some implementations, the first and second reads are on different screens that require a user to access them separately. Additionally a first read could be a simple light, such as a red, blue or yellow LED, for example on the side of a phone, on a smart watch and/or a wearable device as described in the patent application incorporated by reference above. In an implementation of a wearable device, such as a wrist band, the first read could be provided as a color LED, which may be displayed on the wearable device, wherein the second read is only accessible via another device, such as a software app on a smart phone. The first read may advantageously be benign or at least discreet as to not draw attention or discussions about diabetes.

Referring to Figures 16 A and 16 B, a user interface 550 is illustrated presenting two different conditions. In Figure 16 A, a first read 504 is illustrated by the color of the device (as indicated by crosshatching). For example, the user interface of the device in Figure 16 A may be yellow, while the user interface of the device in Figure 16 B may be red. From far away, or by a quick glance, the user may thus be informed of their GUI or glycemic state. In this case, the urgency assessment may be yellow to indicate to the user that their glucose value is relatively normal or at slightly elevated or nearing boundaries of euglycemia. In Figure 16 B, a red urgency assessment may indicate to the user that their glycemic state requires urgent attention based on the GUI. Additionally or alternatively, rather than the yellow or red color indicating glucose states of high, low, etc., colors could be used to communicate other types of information such as positive (purple, for example) or negative (orange, for example) rate of change. The user would understand the meanings of the colors, but others would not realize diabetes information was being communicated due to the abstract nature of the background design.

In the implementation of Figure 16, an optional second read 502 is also provided, which represents the glucose value itself. As noted above, the urgency assessment generally uses the glucose value in its determination, but uses other values as well, which may be equally important in the determination. Additional reads may be accessed via the CGM, e.g., additional information about the GUI inputs, patterns, insights, therapy recommendations, or the like.

In this and other implementations, the home screen of the mobile device, with the urgency assessment module or CGM application running in the background, may persistently be a colored screen indicative of glucose status. The colored screen generally indicates the GUI status even in the absence of alerts or alarms, e.g., indicating a "normal" or non-alert-worthy GUI. The user need not have to perform a swiping action or enter a password to access this information. Such can be seen quickly by pushing an activation button on the mobile device, or as noted above by the accelerometer determining that the mobile device is being handled.

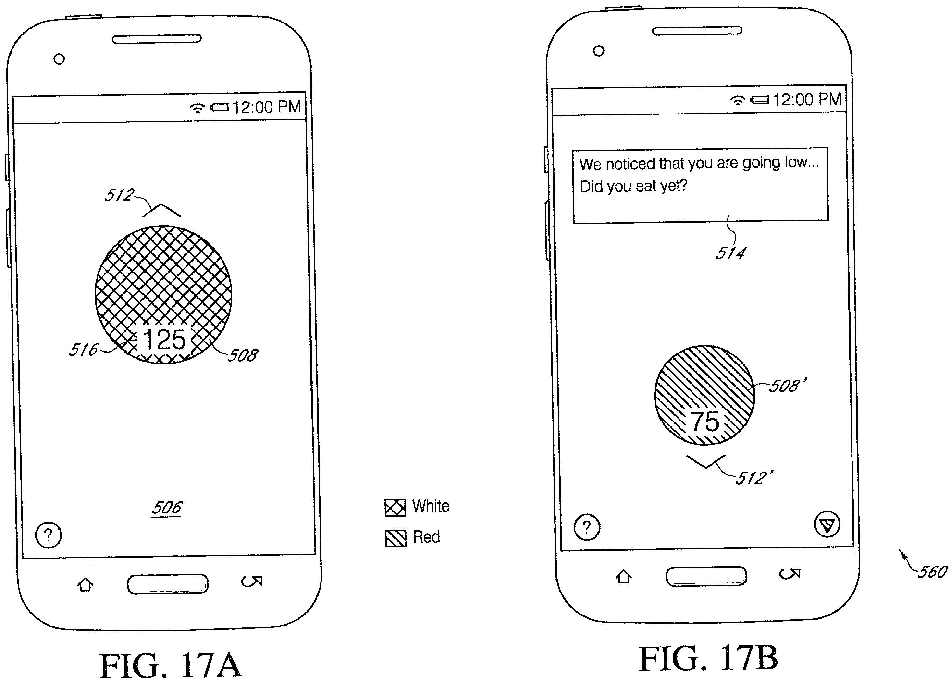

Referring to the user interface 560 of Figure 17, color is still employed, but in this case the first read is not a color of a home screen 506 but rather the color of a circle (circle 508 in Figure 17 A and circle 508' in Figure 17 B) located on the home screen. The second read may be velocity or acceleration, illustrated by the size of the circle 508 or 508', e.g., its radius, as well as an arrow 512 or 512' indicating direction. For example, the arrow may illustrate the first derivative, whether the glucose value is increasing or decreasing, and the size of the circle may indicate how quickly it is increasing or decreasing (the second derivative). Alternatively, the circle may qualitatively represent a potential danger due to the increase or decrease. For example, a large circle may represent that the increase is accelerating away from normal, while a small circle may represent that the increase is decelerating. A third read 516 is also illustrated, this indicating the actual measured value of glucose. Finally, in Figure 17 B, an advanced output 514 is illustrated, this indicating a degree of analysis as part of or contemporaneous with the GUI determination, and providing a suggestion or prompt to the user of potential steps to take.

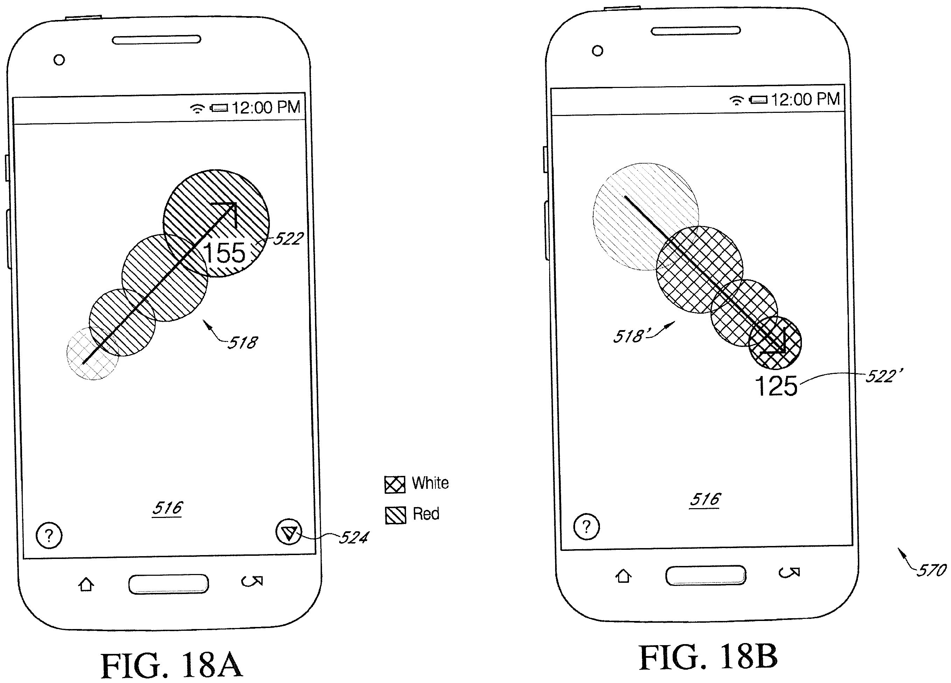

Referring to Figures 18 A and B, an alternative user interface 570 is illustrated presented on the home screen 516 of a mobile device. Figure 18 A illustrates the case of a rising glucose level, and Figure 18 B illustrates a decreasing one. In particular, an arrow and a series of circles 518 (518'), as well as the color of the same, may be employed to indicate to the user that their urgency assessment is rising (Figure 18 A) or falling (Figure 18 B). In particular, and referring the Figure 18 A, the rise itself is illustrated by the arrow, and the color progression, from off-white to red, illustrates that the urgency assessment is also rising, i.e., the situation is becoming more urgent to the user. The glucose value itself 522 (522') is presented as an additional read. In the case of Figure 18 B, the color progression from red to white indicates a return to a more desirable urgency state. The arrow, as well as a presented fading of the circles corresponding to the older-measured urgency assessments, as well as the progression from left to right, indicates the progression from a prior urgency state to later urgency states and finally to a current urgency state. Alternatively, a series of circles (or icons) 518 may represent predicted glucose values or ranges as a first read, which may include an indication of the predicted glucose value at 522 and the prediction horizon (time to predicted value) indicated by the number of circles (or icons), such as 5 minute per circle, or 15 minutes in this example. A second read may be access with a swipe or other user action that may provide additional insights or information associated with the prediction.

Variations will be understood and are similar to certain aspects noted above. Such variations will also be understood to apply to other embodiments described herein. Shapes or icons or visuals other than circles could also be used. For example, it could be images of the moon shown in its phase progression from new moon to full moon. For example, the size of the circles, as well as their color, may indicate the urgency assessment. The sequence of subsequent sizes may indicate how quickly the value is changing. For example, a progression from very small circles to very large circles may indicate a rapid increase in urgency assessment. Conversely, a progression from "medium small" circles to "medium large" circles may indicate a much more mild increase. An additional indicator or read 524 may be employed to provide an icon which may be read at-a-glance to quickly indicate to a user the urgent nature of their assessment. Such may be displayed automatically, without handling of the mobile device, or following handling as determined by an accelerometer or other sensor, or alternatively after swiping/unlocking steps. The same may be accompanied by an audible alert to notify the user of the urgent status.

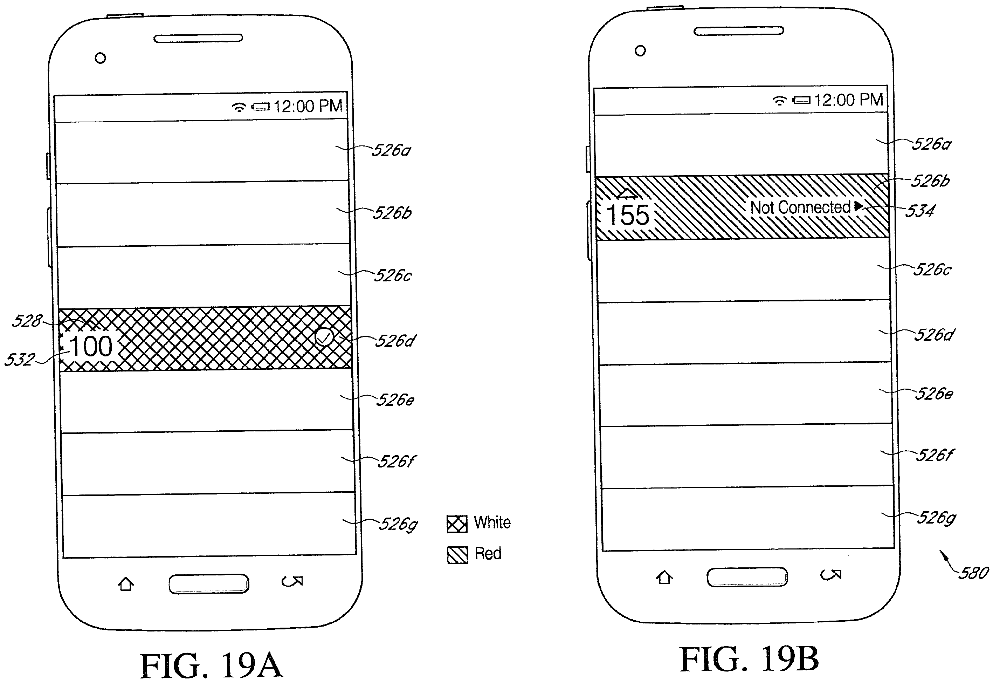

Figures 19 A and B illustrate another user interface 580 which may be employed on a monitoring device, e.g., a mobile device. In Figure 19, the home screen is divided into a number of zones 526a-526g (Figure 19 A) and 526a'-526g' (Figure 19 B). The zones may be equally divided or not, and the number of zones may vary. The number of zones may further vary based on user input, e.g., if a user desires a finer granularity in presented data.

In Figure 19, seven zones are presented, with a low or no-risk zone 526d at the middle of the range of zones. For the urgency assessment given in Figure 19 A, there would be little or no urgency assessed, i.e., little risk to the user. The urgency assessment given in Figure 19 B, however, represents a more urgent state, and in this case one associated with an elevated glucose level. The position and color of the highlighting may be employed to provide the indication. In Figure 19 A, the highlighting is in the middle and the color is white, indicating the low urgency. In Figure 19 B, the highlighting is within the high zone and is colored red, indicating a more urgent condition. Where highlighting, or other read indicators, our position or textually based, rather than color based, the same may be advantageously employed for colorblind individuals. Figure 19 also indicates an arrow 528 and a numerical depiction 532 of a glucose value. From the numerical depiction 532, the user may be notified of their glucose value. From the arrow 528, the user may be notified of the direction their glucose value is heading. A more solid arrow (shown in Figure 19 B) may indicate a more rapid increase or an accelerated increase. These factors, and generally more, enter into the determination of the GUI, and the representation of the result of this determination is the highlighted range, indicating the determined urgency assessment.

An advanced output 534 is also indicated, such providing additional information to the user about potential causes of the hyperglycemia, potential steps to lower the urgency assessment, and so on.

It will be understood that the ranges within the different zones need not represent equally spaced levels of urgency, and that the same may provide quantitative or qualitative levels of urgency based on the determined GUI. The ranges may be set by a user or by a physician or other caregiver, and thus may be customized to the needs of the particular user. Similar customizability will apply to other embodiments of user interfaces disclosed herein.

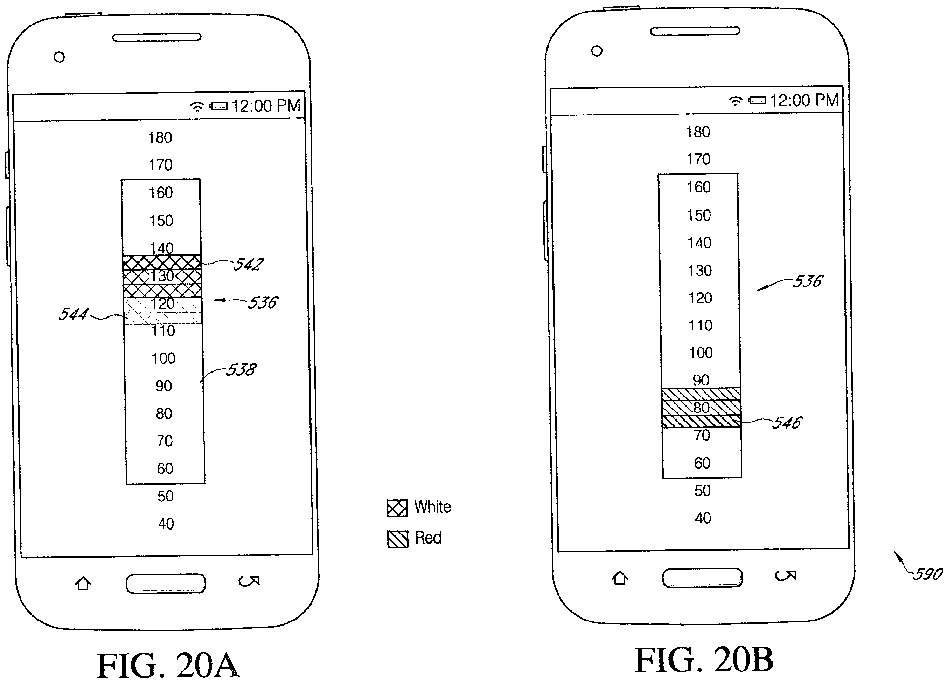

In Figures 20 A and B, a user interface 590 is illustrated having a scale 536, similar to a thermometer, and in which a rectangle 538 is depicted showing a generally desired glucose range. This user interface does not require the use of arrows. A current level of glucose may be indicated by a highlighted bar 542, and a past level of glucose may be indicated by a more faded bar 544, with bars of various gradations in between indicating changes in the glucose values over time. In some implementations, the solid background color may be indicative of a 3rd read or 3rd level of information, such as Wifi status, communication of glucose with other mobile devices (data sharing), or the like.

The color of the bars, both current and past, may indicate relative or absolute urgency assessments. For example, the color of the bar 542 may be white while the bar 546 (Figure 20 B) may be red. The color values indicate the urgency assessments, of which the glucose value is a factor but only one of several or many. Thus, the bar 542 is depicted as white in the figure but may be depicted as red in an alternative situation, even with the same glucose value, but where the value was increasing and accelerating (or taking other excursions).

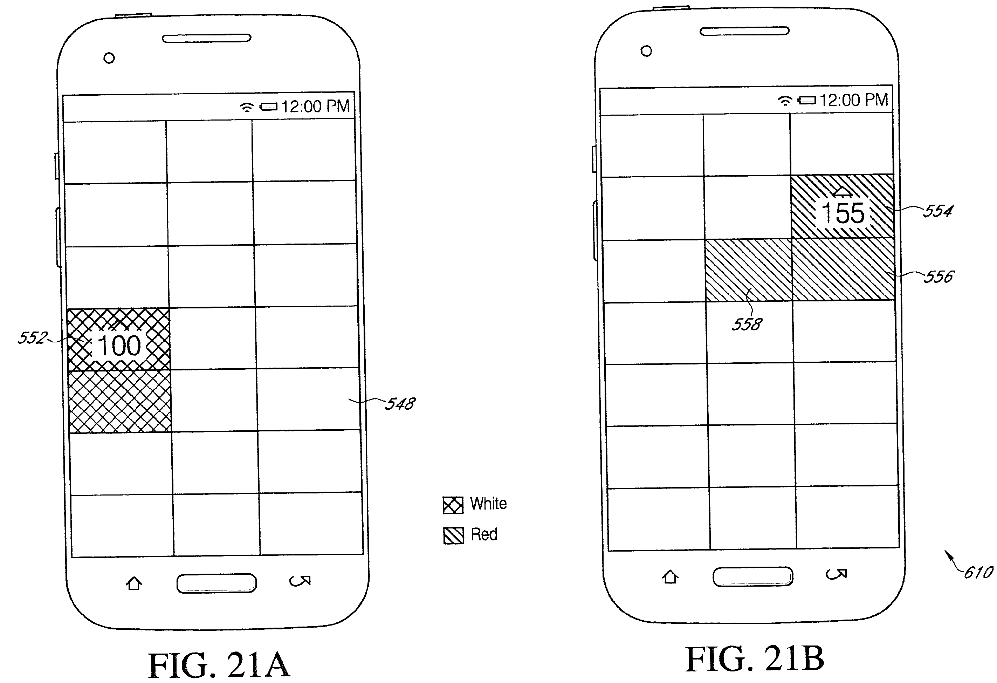

Figures 21 A and B illustrate another way of representing urgency on a user interface 610 of a mobile device. The user interface 610 depicts one way in which an especially discrete representation may be provided. In particular, a grid pattern 548 is provided which may be customized by the user or on behalf of the user. The user may associate set grid spaces with particular urgency indications. For example, the lower horizontal row may represent hypoglycemic urgency assessments, the middle zones may be target urgency assessments, and the upper horizontal row may represent hyperglycemic urgency assessments.

In some implementations, the columns may indicate snapshots in time or recent historical urgency assessments. For example, the leftmost column may represent a recent past assessment, the middle column a current assessment, and the right column a predicted future assessment. Alternatively, the rightmost column may represent a current assessment, and the left and middle columns representing recent historical assessments. Other variations will also be understood.

The user may be aware of the numerical thresholds, but the same are not placed on the screen, for various reasons, including discretion, prevention of questions from others, and/or general purposes of clarity. The grid spaces may be populated based on the urgency assessment, including, e.g., direction and magnitude of increase, and other variables. To an outsider, the user interface 610 may simply appear as a design pattern, or even a game screen.

As in other embodiments, but in this case with regard to a grid space 552 and 554, a highlight occurs with a particular color, an indication of the glucose value, and an indication of the direction the glucose value is moving. As seen in Figure 21 B, highlighting may also be provided in other grid spaces, here shown as grid spaces 556 and 558, to indicate recent past historical values of measured glucose.

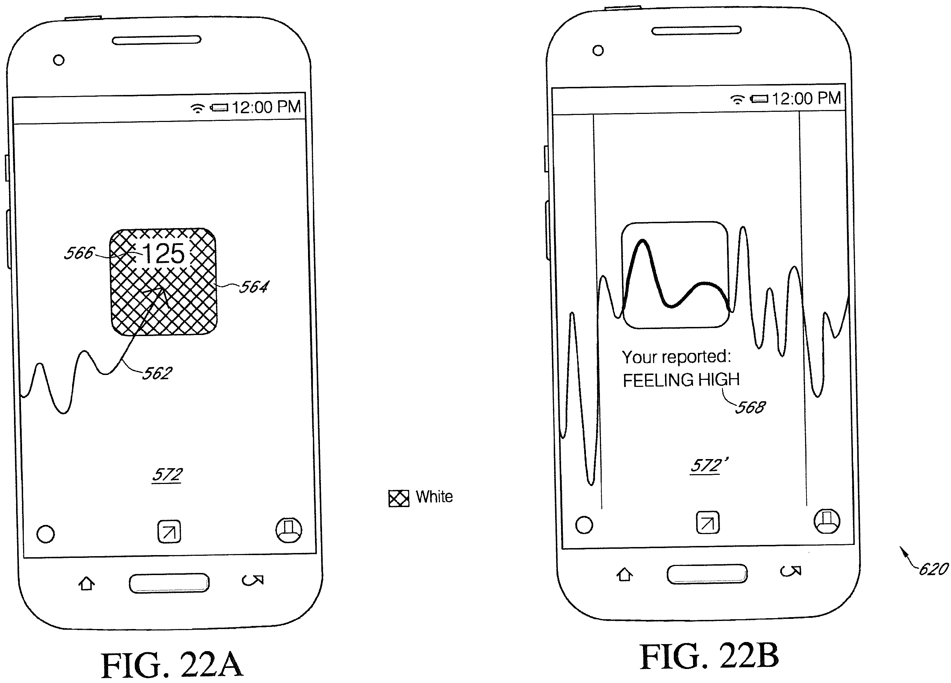

Figures 22 A and B illustrate another user interface 620 which may be employed to represent urgency. In these figures, a graph of glucose values 562 is provided to allow a user to see recent historical values. Such may be particularly important for users who desire a higher degree of knowledge about their current levels. As illustrated in Figure 22 A, a current glucose value 566 may also be provided, as well as a box 564 framing the value and generally indicating where on the graph the current time is represented. To represent the urgency assessment, which is based on the GUI, a color 572 of the screen may be employed. For example, in Figure 22 A, a light red background 572 may indicate a rising level but a low urgency assessment. In Figure 22 B, a blue background 572' may indicate euglycemia and a zero urgency assessment. Figure 22 B also shows that the box 564 can be used to indicate a section of the glucose trace graph, e.g., in higher fidelity, with additional processing (e.g., detected pattern), or the like. In the user interface of Figure 22, no horizontal threshold bars are required, and yet through use of a colored background, the user is still made aware of the "zone" of their urgency assessment.

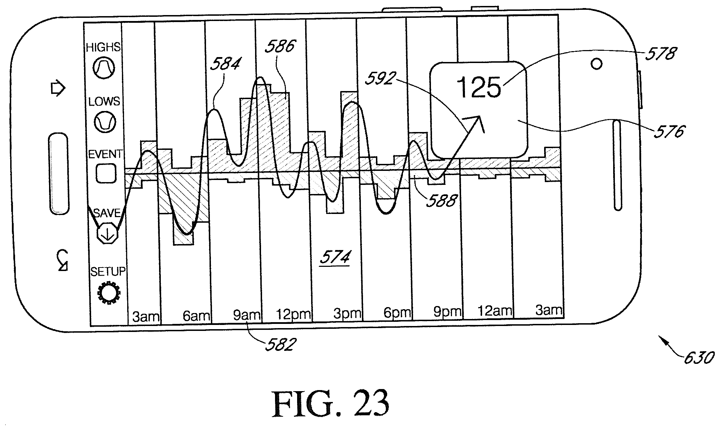

Figure 23 shows a user interface having a greater level of detail, and in particular showing averages and a predicted glucose level, which may lead to a GUI and thus urgency assessment based on factors such as historical performance for that time of day. In particular, the user interface 630 includes a background 574 which may indicate the urgency assessment, e.g., blue, green, yellow, red, and so on. Alternatively, a color of the box 576 may indicate the urgency assessment, the box also being where a current (or, alternatively, predicted) glucose value 578 is displayed. The horizontal scale 582 is with respect to time of day, and a trace graph of the glucose level 584 may be displayed thus as a function of the time of day. Historical values (average glucose profile or normal patient pattern) for the given time of day are illustrated by histograms 586 and 588, which illustrate hypoglycemic and hyperglycemic ranges seen historically for a given time of day for a given user.

A portion of the graph, e.g., a portion within the box 576, may represent a projected glucose level, determined using predictive analytics as described above, and which may or may not be involved in the urgency assessment. This projected value is illustrated as trace 592, which in the exemplary Figure 23 has a different line width. Areas of particular lows or highs may be portrayed in different colors, e.g., yellow for moderately high/low and red for very high/low.

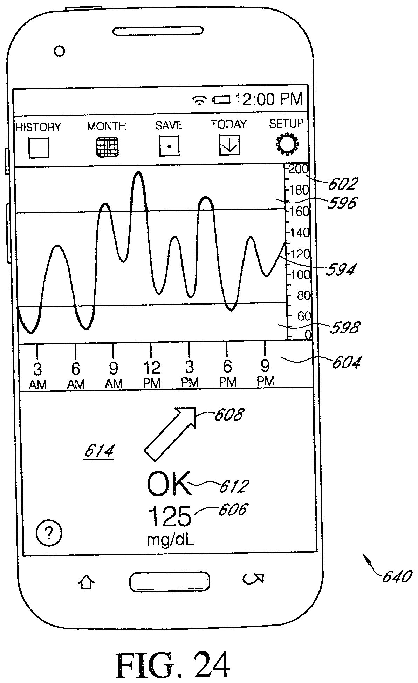

Figure 24 illustrates a user interface 640 showing a user even greater detail, or dashboard, about their glycemic state and other glycemic or product status. This screen portrays multiple pieces of information in one place to avoid multiple button presses or swipes to access statuses otherwise found in many different locations in an app or website. In particular, the user interface 640 includes a trace graph 594 in which excursions from normal glycemic values are shown by traces within ranges 596 (hyperglycemic) and 598 (hypoglycemic). The trace 594 shown in Figure 24 is of glucose values on scale 602 shown with respect to time as indicated by a timescale 604, but it will be understood that other variables may also be portrayed, e.g., deviations from normal glucose values, and so on.

The glucose values and other factors as have been described bear on the determination of a GUI and thus an urgency assessment. The urgency assessment may be indicated to the user via the user interface 640 by the color of the background 614 or by an indication such as a textual indication 612, which indicates in Figure 24 that the assessment is that the user is "OK". The user interface 640 also indicates a current glucose value 606, as well as an indication of the direction the glucose value is heading, shown by an arrow 608. In some implementations, the slope, size, or other aspect of the arrow 608 may indicate how fast the glucose value is increasing or decreasing.

Variations will be understood. In particular, it is noted here that in this and other implementations not all aspects shown are required to be displayed. For example, in Figure 24, the numerical glucose value 606 may be omitted as the user may obtain similar information from a consideration of the trace 594, or just by the textual indication 612.

In this as well as other implementations, rate of change as well as acceleration information may be added to the trace graph arrow. Of course, the rate of change may be evident from the trace graph itself, but colored or a curved arrow may be employed to indicate acceleration or deceleration of glucose level. In one implementation, a red color arrow may indicate an undesirable value of the acceleration, while a blue color indicates desirable. In another implementation, a curving of the arrow away from normoglycemia may indicate an undesirable acceleration, while a curving towards normoglycemia may indicate a desirable trending.

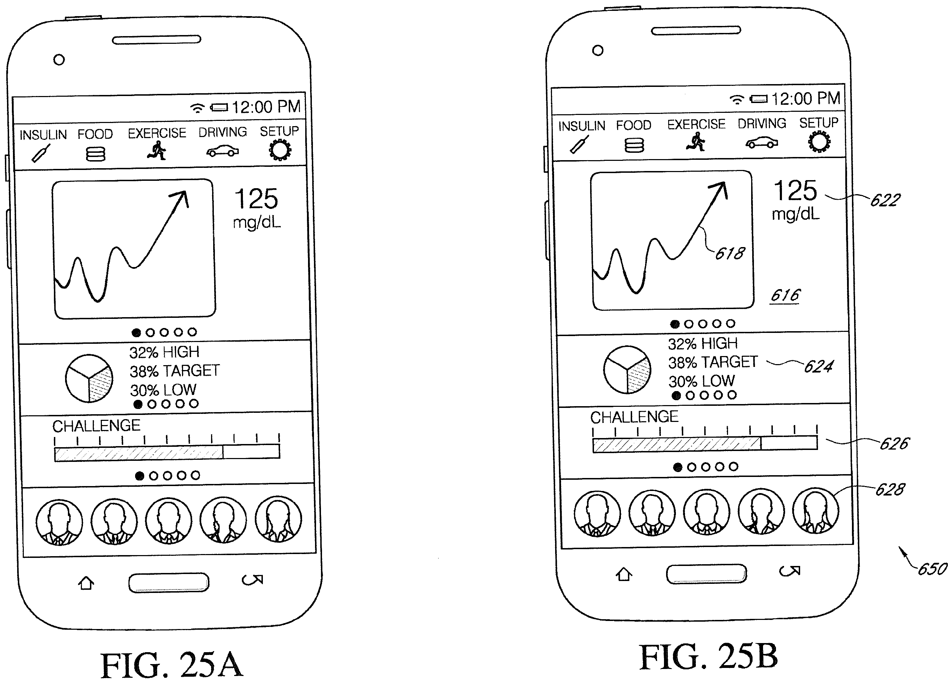

Referring to Figures 25 A and B, a user interface 650 is displayed on the mobile device containing additional data. In particular, the user interface 60 includes a trace graph 618 corresponding to glucose levels, as well as a numerical value 622. The numerical value 622 can provide the user with an instantaneous or current level, while the trace graph 618 can give the user, even without a y-axis, an idea of what the values have recently been. The color of the screen 616 may provide another read, and may indicate a current urgency assessment. The qualitative chart 624 may be employed to give users an at-a-glance read as to the zone they are occupying: target, hyperglycemic, or hypoglycemic. In the figure, the pie pieces may represent a percentage of time in those zones per day, per month, or over any other period of time. Alternatively, not shown, the pie graph may be "overlaid" onto a clock, to show, e.g., that in a green segment 12-2 the user had a high GUI, then from 2-4 they had a low GUI, and so on. An area of the user interface 650 may be provided as a challenge area 626, indicating to the user qualitatively how well they are meeting a challenge they have set for themselves, generally to maintain a glucose value within a target range. A section of the user interface 60 may be provided to indicate friends or followers 628 of the user. By swiping the user interface 650 to the right or left, data corresponding to the friends or followers may be displayed and reviewed. For example, each can see how well the others are meeting their set goals or challenges.

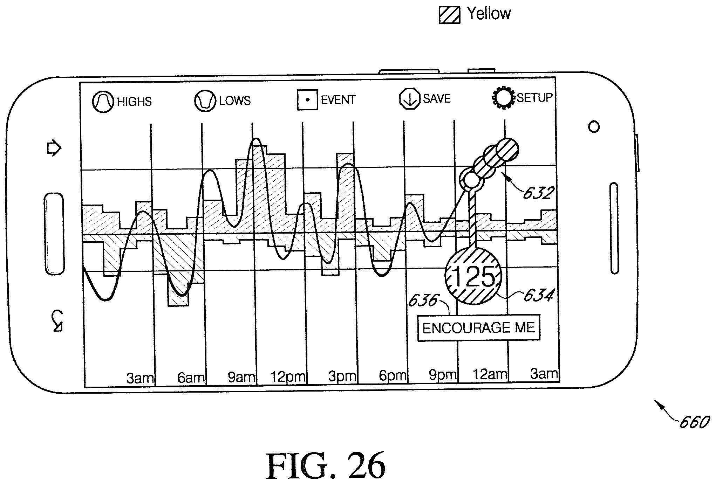

Figure 26 illustrates another user interface 660 which may be advantageously employed. The user interface 660 is similar to that of the user interface 630 of Figure 23, but additional details of predictions are included. In particular, the user interface 660 includes points 632 which are plotted so as to correspond to predicted glucose levels, based on predictive analytics as described above. An instantaneous glucose level 634 is displayed, as a numerical value, to provide the user with an easy-to-read indication.

An advanced output 636 is also illustrated and may be employed by the urgency assessment module in various ways. For example, where a number of friends or followers are associated with the user, the same may provide a notification to the friends or followers to take some action with respect to the user. For example, the friends or followers may be called on to encourage the user where the user's urgency assessment is trending high or low. The advanced output 636 may also be employed to encourage the user himself or herself, e.g., to suggest a course of action or to otherwise provide encouragement. Additional details of advanced outputs are provided below.

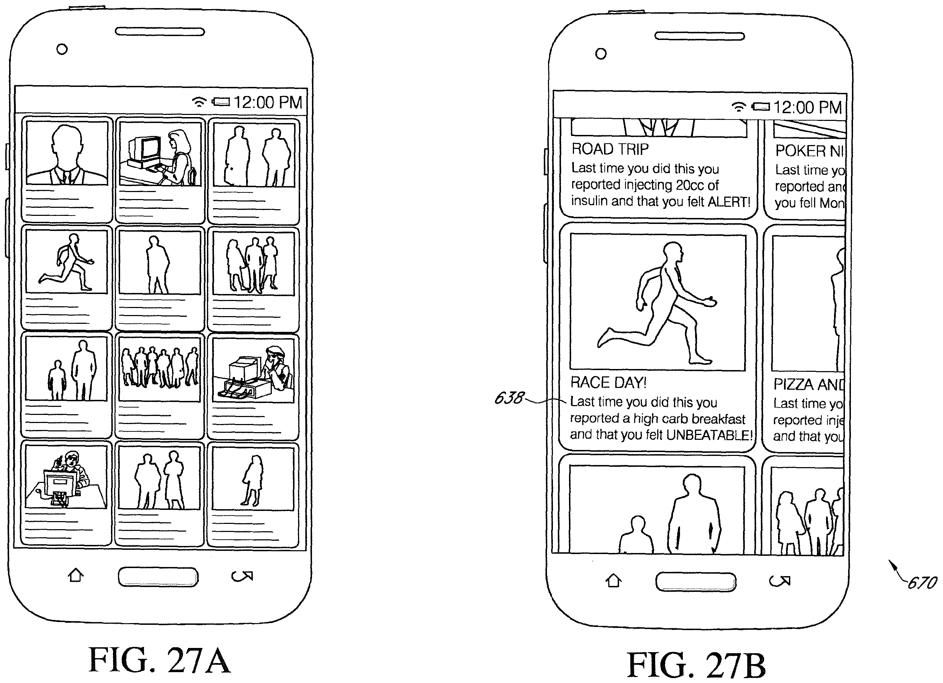

Figures 27 A and B illustrate various activities or postings which may be provided to or from a feed associated with a social networking service. At a most passive level, users may receive updates from friends or followers to which they are associated within the social network. This group may correspond to all friends or maybe a subset of friends, e.g., those also living with diabetes. At a more advanced and interactive level, user postings may be employed within the determination of a GUI, e.g., a posting about participation in a race, combined with glucose and other data known pertaining to the user on that day, may provide enhanced data for the social networking feed. Similarly, the same may be used in combination with historical data about similar circumstances to provide and post historical comparisons. For example, in Figure 27 B a posting is seen (posting 638) of "RACE DAY! Last time you did this you reported a high carb breakfast and that you felt UNBEATABLE!".

Other aspects pertinent to social networking will be understood given this teaching. For example, in various other embodiments, the assessment module 211 may be used in conjunction with the contacts module to provide updates to a social network. In some embodiments, the smart phone 200 can use the user's location and/or other attributes associated with the user (such as type of diabetes, age, sex, demographic, etc.) to find other people in the area, with similar attributes and/or using a similar CGM device or smart phone application, to recommend as a social connection. For example, the CGM application and/or a social media site in concert with the CGM application may enable the user to select from options such as find other people with diabetes, find other people with diabetes near me, or find recommendations of diabetes-friendly restaurant in the area.

In some embodiments (see Figure 6), the CGM application 209 (alone or in combination with the assessment module 211, which generally but not necessarily runs within the CGM application 209) enables a user to selectively upload or share information about their assessment electronically and/or via a social network site. Example information that could be shared includes a success metric, a current EGV value, a screen shot, an achievement, an award, a pattern trend graph, activity information, location information, and/or any other parameter described as a possible input into or output from the assessment module 211 elsewhere herein. For example, the CGM application 209 (it will be understood here and below that such may include the assessment module 211) may have user selectable actions such as share EGV on Facebook, share EGV on Twitter, share screen via Facebook, Twitter, e-mail, MMS, send trend screen to printer, etc. Additionally, or alternatively, the CGM application 209 may enable a user to add preset and/or custom captions, or change a status with their shared information, such as check out my no hitter, which can be shared selectively (by a user or based on parameters) and/or automatically. In one example, a user can "like" a particular GUI or its indication directly to a particular social site. In certain embodiments, when the user selects to share information, options may be shown on the display 202 (Figure 6) that enable the user to select what to share and with whom. The user may predefine groups and or individuals to share information with. For example, the user may create a group of friends, and when the user chooses to share something he or she selects to share it with the predefined friends, and a notification is then sent to each person in the group. This functionality is useful, for example, for parents who want to monitor their child's blood glucose. The child can choose to share a BG value, and then select parents, or mom, or dad, and the BG value is then sent to the selected person(s).

In some embodiments, the CGM application 209, in concert with a social network, can be configured to allow users to compare EGV, trends, number of lows, etc. with friends or a group on the social network site (e.g., Facebook). In some embodiments, the CGM application 209, using CGM information from a plurality of users, compares one or more parameters to determine a comparison of data from a single person to the average (grouped by some similarity, for example). In some embodiments, the CGM application 209 calculates achievements, points, badges, or other rewards based on predetermined criteria (keeping blood glucose in a target range, use of CGM, etc.), which can be selectively or automatically posted to a social network site (e.g., Facebook). In some embodiments, when a user would like to share a learning from the CGM application 209 or assessment module 211, e.g., a picture of food and resulting EGV or trend graph, the CGM application 209 enables the user to selectively upload the information to a site. In this context a learning is an event or a first situation, and the effect, output, or trend resulting therefrom.

Additionally or alternatively, data from CGM users can be aggregated, whereby the CGM application 209 is configured to enable a user to query for current active CGM users, e.g., a xx% of people using a CGM that are in range, other CGM users with a similar glucose as him or her (within a margin of error, such as 80 mg/dL ± 5). These queries can also be narrowed by geography, by doctor, age, gender, ethnicity, diabetes type, therapy type (pump, syringes, exenatide, metformin, etc.), etc.

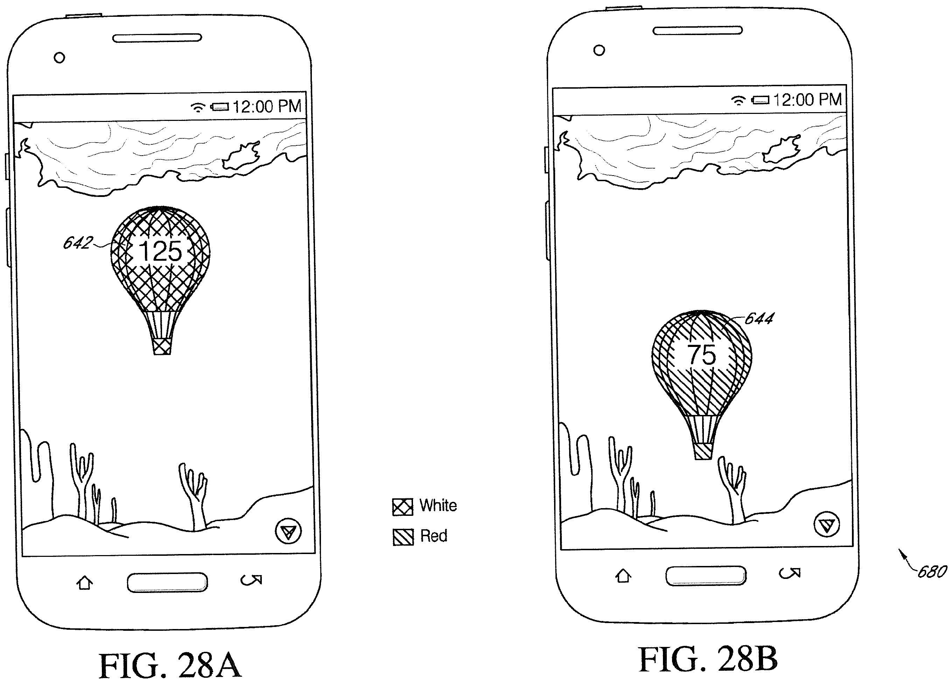

Continuing the discussion of user interfaces, Figures 28 A and B display to potential results of a user interface 680. The user interface 680 shows particularly discreet results, ones that would likely only be known to the user. In this way, the health status of the user is not displayed in a particularly overt way on the user interface. In particular, and referring to Figure 28 A, a balloon is shown which has a color 642 and an optional numerical value 644. The color displays in a discreet and benign way the results of the GUI determination. The numerical value 644 provides an additional read, such pertaining to a current glucose value in this implementation. It will be understood that besides a balloon, numerous other icons or images may be employed, and in fact may be selected by the user. In this way, for example, the user may select a car, where the color of the car pertains to the GUI determination (e.g., red, yellow, green, etc.). In the example of a balloon, additional information or reads may also be provided. For example, the height of the balloon may indicate if the GUI is becoming more urgent or less urgent, or if the glucose value is increasing or decreasing. Other variations will also be seen.

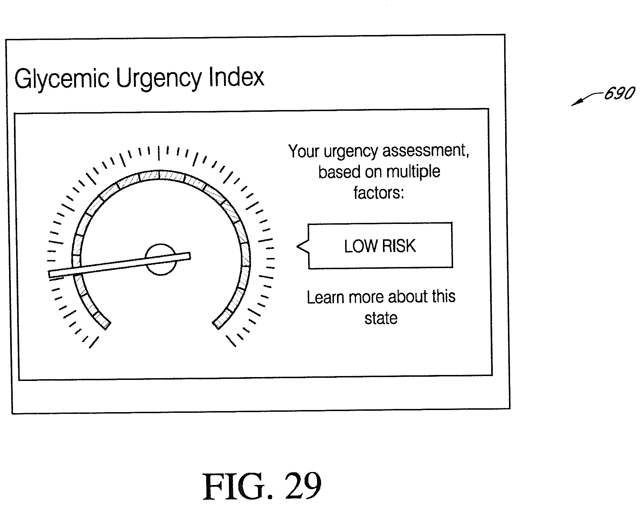

Figure 29 illustrates another user interface 690 which may be employed to portray a level of urgency to a user. In the user interface 690, a tachometer-like display has a green section representing low urgency states, a yellow section representing medium urgency states, and a red section representing higher urgency states. The position of the needle indicates the current state, i.e., is tied to the determined GUI.

It will be understood that the above user interface depictions are purely exemplary and that numerous variations will be seen. For example, the GUI determination and actionable alert may be provided within a game, either to hide the data such that only the user can discern the same, or in such a way that favorable GUI determinations lead to favorable game outcomes. In other words, if the user controls their urgency assessment, they win the game. Moreover, the notifications and actionable alerts may be provided in a way differentiated from that of other alerts on the mobile device, e.g., alerts from text messages, application updates, phone calls, voicemails, and the like. The user may configure the CGM application 209 such that alerts from the urgency assessment module 211 override certain or all other alerts, or must be cleared before the user can use the phone, so that urgency alerts or alarms are not inadvertently missed. As noted the alert or alarm may escalate as the urgency assessment rises, i.e., becomes more urgent. While initially an alert may be shown on the user interface upon unlocking the device, such may escalate to an audible alarm as the urgency increases.

Further information provided by the user interface may include rebound highs and rebound lows, such that the user can be notified of such statuses and can provide potential user actions. In so doing, the user can easily compare and become aware of the cause and effect of such, easily relating the cause to the action, as both are still fresh in the user's mind.

In some implementations, the data that is output to a user may be adaptively driven by creating various modes in which the urgency assessment module may operate. In addition, the urgency assessment module may adapt to real-time inputs by the user or physician/caregiver, or on other criteria envisioned as useful.

In particular, users may not want to be alerted if their GUI has a small excursion, e.g., into a mildly urgent state, if the same is simply following a known pattern. A user may not want to be notified that their glucose is momentarily high after eating a meal because they expect it to be high, which GUI may be determined based on pattern inputs as described in more detail elsewhere herein. Users may not want to be reminded of glucose fluctuations if they are having a day with bad glucose control as a result of "bad behavior", e.g., choosing to eat birthday cake and having a few drinks with a friend celebrating their birthday, when they are knowingly take a day off from trying to achieve optimal control. The user wants to be protected, but not necessarily always reminded. Thus, in one implementation, the user can set the urgency assessment module into an "action mode", where the user is only notified of potentially risky scenarios such as "below 55", "below 70 for more than 30 minutes", or "above 300 and still rising quickly", and the like. Providing the urgency assessment module with such a capability solves the problem that CGM users are often given information at inopportune or potentially annoying times. The same also allows the display of appropriate information or reminders when users are not checking their CGM as well as when they are regularly checking it.

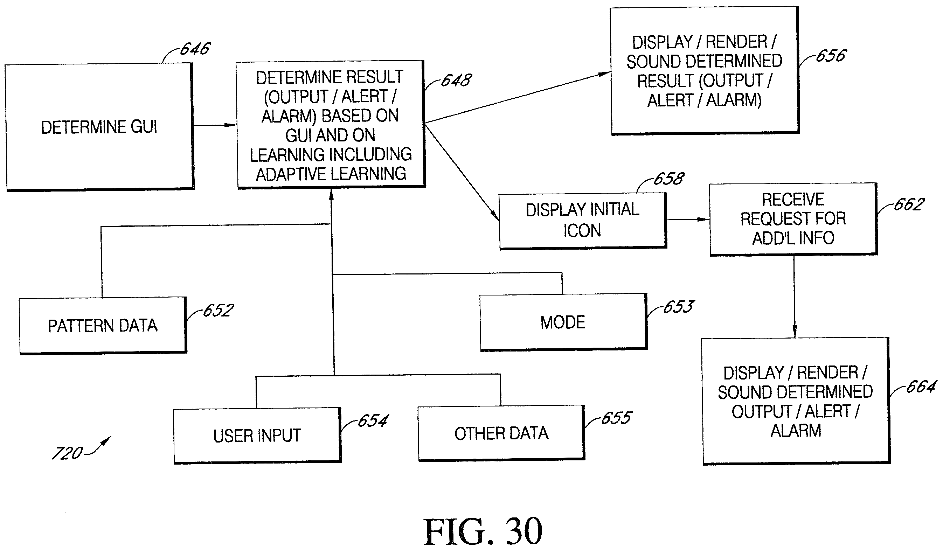

Referring to the flowchart 720 of Figure 30, a method according to present principles is shown for displaying alerts. In a first step, a GUI is determined as described above (step 646). A result is then determined of the GUI, and the result may be an alert, alarm, or just an output of the current state of urgency (step 648). The output may also be based on adaptive learning, and in particular learning about characteristics of the user, including patterns and trends 652 of the GUIs, as well as of glucose levels, mobile device usage characteristics, and other parameters and variables described above. Such may include user input 654, e.g., where a user indicates a desire to only be notified of dangerous conditions, and the like. The adaptive learning may also be based on the mode 653 in which the urgency assessment module is operating. For example, if the mode indicates that the user only wants to be alerted on dangerous conditions, such may be factored into the determination of the result in step 648. In the same way, the user may enter a mode 653 in which they desire significant levels of encouragement or suggestions. Other modes will similarly be understood given this teaching. Other data 655 may also be employed in the determination of a result based on learning.

Once a result is determined, the same may be presented to the user or physician/caregiver (step 656). In the presentation, the result may be displayed on the screen, sounded audibly, or otherwise rendered. The result may be a general presentation of data, an alert, an alarm, or the like. Alternatively, the result may simply be the display of an initial icon (step 658). The icon may provide an indication of the urgency assessment, but may be nondescript. In this way, the user makes the decision as to whether to receive additional information, i.e., "drill down" to additional information. Such may be requested in a number of ways, such as by swiping the icon, navigating to an app, or the like. The urgency assessment module receives the request (step 652), and provides the additional information (step 664), which in some cases may be the same or similar to that provided in step 656.

The request to receive additional information may be responded to in a number of other ways as well, and in some cases may coincide with certain advanced outputs noted above. One potential output type involves prompts and questions/answers, and in some cases may involve avatars so as to more fully engage certain users, e.g., children.

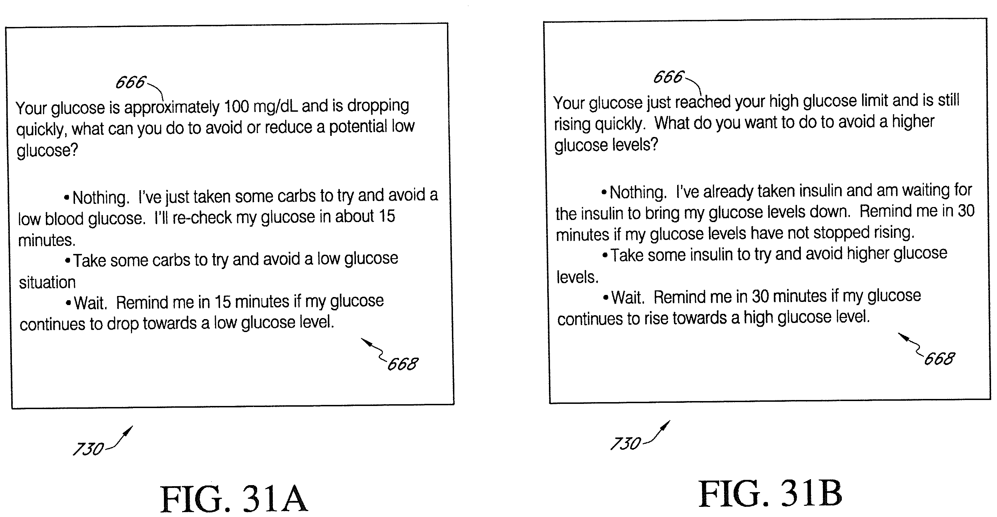

In another implementation of a user interface, illustrated by a user interface 730 as shown in Figures 31 A and B, various scenarios are presented to the user based on the urgency assessment. In this way, rather than reinforcing a particular measurement, focus is directed to potential actions to take. For example, referring to Figure 31 A, the user interface 730 may indicate the situation 666, and present various scenarios δ68 from which the user may choose. Figure 31 B shows an alternate situation.

The potential actions may be ordered based on most safe to most aggressive. Alternatively, "what would you do if?" questions may be posed to the user, which response may feed back into the assessment module as an input into the GUI determination.

The potential actions may in some cases be hard notifications, similar to alerts, or may alternatively be a soft prompt which the user only sees when they look at their CGM screen. In such cases the data pushed may be updated for that moment in time. The criteria may also be set for notifications based on potential risk scenarios such as a severely low glucose level, and the like.

As noted above activities may be recognized, such as sleeping, exercising, or the like. The outputs may be keyed to such detected activities. For example, if a patent is sleeping, e.g., it is 2am and the sensor and/or mobile device has been stationary for $X$ minutes, where $X$ is 10 minutes, 20 minutes, 30 minutes, 1 hour, etc., then sleep may be assumed and the output may be audible and loud enough to wake the user. When the user is driving, the output may also be audible and/or significantly elevated in volume. When the user is deviating from a normal pattern, the output may provide additional detail or may ask the user questions. Other variations will be understood.

Besides providing a user or caregiver/physician information regarding an assessment of urgency, the GUI may be translated or transformed to an insulin pump command using an appropriate mapping, look-up table, or function. The same may be displayed to the user, to enter as a pump command on a separate pump, or may be directly provided to a pump to dispense insulin in a closed loop system. In more detail, the same may be used to moderate insulin delivery, e.g., suspending, reducing, or increasing basal or bolus insulin delivery, based on glycemic risk state. By basing pump functionality on an urgency assessment such as the GUI, a user's urgency state is considered in a much more useful way, using other factors than just current (or even predicted) glucose levels passing a threshold. For example, a particular implementation may call for dispensation of a bolus of insulin upon a GUI indicating a hyperglycemic state. Besides pumps, it will be understood that the urgency assessment module may interact with other devices as well, including devices communicatively coupled via a network.

Guidelines for notifications, as well as for alerting and alarming, based on GUI, may be factory-set or may be customizable by the user, who may also set threshold values at which alerts and alarms occur. The system may also provide for changes in alerts based on trends in the GUIs or other factors, either automatically or instigated by the user. As such, the system allows for the dynamic and/or iterative update of urgency indices and states with time, as additional data is received, and based on user actions.

For example, a user may have an estimated glucose of 100 mg/dL, but may be dropping at a rate of 2 mg/dL/min, so a hypoglycemic urgency (e.g., a yellow state) is estimated in 15 minutes (30 mg/dL drop) at 70 mg/dL and a severe hypoglycemic urgency (e.g., a red state) is estimated at 22.5 minutes. If a red urgency state is defined as 20 minutes to 55 mg/dL, the urgency assessment module would display a yellow state at the moment, but if the user does not or has not already acted, the user would likely see a change from the yellow state to a red state within a few minutes.

The urgency assessment module may allow for different sensitivities based on, e.g., time of day (day versus night), while driving, during exercising or sports, while napping, during times of illness, and so on. Sensitivities may also be adjusted based on the desires of the user. For example, some users desire significant amounts of feedback, including positive feedback, and such users may adjust the sensitivity to a high setting (alternatively, a low discrimination level) in order to enable significant amounts of information to be presented. Other feedback provided may be positive in nature, such that, as soon as the assessed urgency begins to be resolved, the user interface may display a message indicating the improvement of the situation, e.g., "looks like your treatment is working". In this way the user is advantageously encouraged, even prior to the user reaching an optimal correction point, further advantageously preventing insulin and food stacking, which may lead to a deleterious overcompensation. Further details of such feedback types are described below.

In contrast, other users may only desire alerts or information when they are entering a dangerous or potentially dangerous urgency assessment. For such users, the sensitivity may be set to a low setting (alternatively, a high discrimination level) in order to minimize the number of alerts or alarms received.

Other types of encouragement may also be employed within the feedback or output from the urgency assessment module. For example, when a user goes from a more urgent glycemic state to a less urgent glycemic state, i.e., from more risk to less risk, the output could similarly indicate that their treatment has begun correcting the urgent glycemic risk state, again preventing insulin and food stacking.

Other types of advanced outputs are possible will also be understood. For example, based on a current urgency assessment, as well as data about insulin on board or food intake, recommendations may be provided for treatment, i.e., ways to lower the urgency assessment. As another type of advanced output, a link may be provided which when clicked leads the user to additional information about the current condition or urgency assessment. Future or predictive trend graphs (or other ways of providing such information, e.g., numerical indices or scores, colors, or the like) may be provided, indicating a direction in which the GUI, i.e., urgency assessment or state, is expected to go, or could be made to go with different treatment options. A future trend graph, in glucose level or other parameters, may also be provided.

By use of these types of outputs, and by continuously updating the glycemic state, trending information of the GUI or the urgency assessment, clearly distinguished from just an output based on a threshold crossing of a glucose value or even a glucose trend, can provide information to the user that they otherwise would not receive.

While the above information is generally based on a prospective glycemic urgency assessment, selective alerts may also be based on a retrospective algorithm, looking for certain types of glycemic excursions which may indicate long-term glycemic complications. Such was described above in connection with a GUI determination, but here it is noted that the same may also be the basis of various user interface displays and/or prompts. For example, a retrospective investigation may reveal large excursions from low to high or high to low. One example is illustrated below based on a retrospective alert system intended to engage users with CGM events. The retrospective alert may issue an alert on a user's smart phone when a meaningful piece of information is discovered in their data.

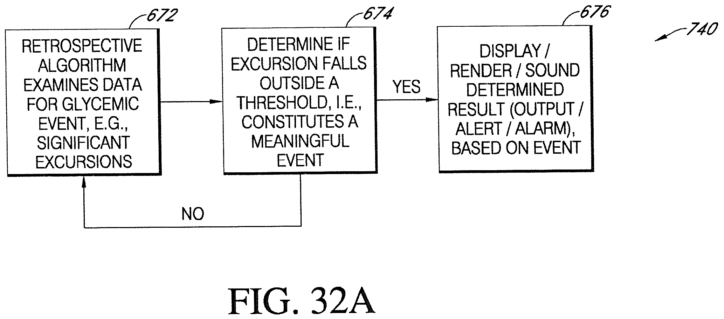

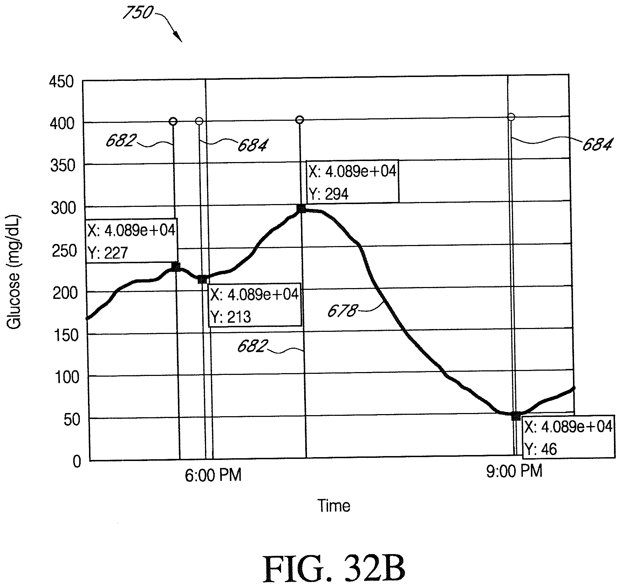

An exemplary such method is illustrated by the flowchart 740 of Figure 32 A. In a first step, the retrospective algorithm examines data for various glycemic events, e.g., significant excursions from normal values or from values determined to be typical according to a baseline, a developed pattern, or the like (step 672). In so doing, the algorithm may examine local recent minima and maxima whenever a new sensor packet of data arrives. An example is illustrated by the graph 750 in Figure 32 B. The trace dots 678 illustrate a CGM trace, bars 682 represents a start of an event, and bars 684 represents the end of an event. Two events are shown, one starting at 227 mg/dL and ending at 213 mg/dL, and another starting at 294 mg/dL and ending at 46 mg/dL.

The retrospective algorithm then checks whether the current event excursion falls outside a threshold (step 674). The threshold may be based on the difference between values in mg/dL, based on a percentage difference between the start and the end of an event, or on other factors, e.g., if the excursion is greater than a standard deviation outside a typical excursion, and so on. By putting such a threshold in place, the system ensures that only meaningful events are displayed to the user and further that nuisance alerts are minimized. Advantageously, easy collection of user information useful in pattern recognition may be enabled, as well as generally educating the user of personal glycemic events, patterns or profiles.

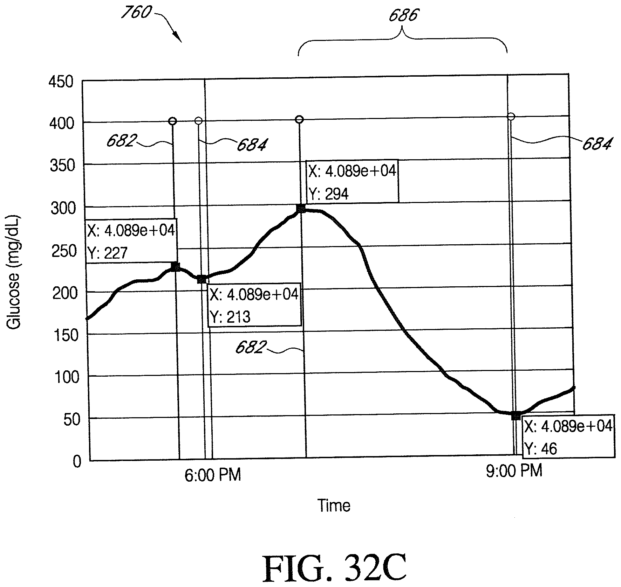

If an event falls outside the threshold and is thus meaningful, the same triggers an alert or other resulting output (step 676). For example, a push notification may be delivered, an icon on a trend graph may be portrayed, a batch number may increase, or the like. Other such notifications will also be understood. The alert displayed will generally vary based on the type of event. For example, if the event indicates that the user has traversed from a high glucose value to a low glucose value, e.g., 294 mg/dL to 46 mg/dL, the message may be "We noticed a wide glucose excursion - would you like to enter carb and/or insulin information for this event?". A trend graph segment could be highlighted in another color while the alert is active, indicating that the user has not cleared the same. Such may be indicated in the graph 760 of Figure 32 C by a different color (line or points) within segment 686.

What has been disclosed are systems and methods for dynamically and iteratively evaluating a glycemic urgency index, tied to an urgency assessment. A variety of methods have been disclosed for determining the glycemic urgency index, as well as for displaying a determined urgency assessment to a user.

Variations will be understood to one of ordinary skill in the art given this teaching. For example, while trends, and especially trends in the determined GUI, may be employed to present information to a user on the user interface of a mobile device, trends may be identified and used as a teaching tool for a physician or caregiver, to note patterns, incidences or events to which a user should pay attention.

The connections between the elements shown in the figures illustrate exemplary communication paths. Additional communication paths, either direct or via an intermediary, may be included to further facilitate the exchange of information between the elements. The communication paths may be bi-directional communication paths allowing the elements to exchange information.

As used herein, the term "determining" encompasses a wide variety of actions. For example, "determining" may include calculating, computing, processing, deriving, investigating, looking up (e.g., looking up in a table, a database or another data structure), ascertaining and the like. Also, "determining" may include receiving (e.g., receiving information), accessing (e.g., accessing data in a memory) and the like. Also, "determining" may include resolving, selecting, choosing, establishing and the like.

As used herein, the term "message" encompasses a wide variety of formats for transmitting information. A message may include a machine readable aggregation of information such as an XML document, fixed field message, comma separated message, or the like. A message may, in some implementations, include a signal utilized to transmit one or more representations of the information. While recited in the singular, it will be understood that a message may be composed/transmitted/stored/received/etc. in multiple parts.

The various operations of methods described above may be performed by any suitable means capable of performing the operations, such as various hardware and/or software component(s), circuits, and/or module(s). Generally, any operations illustrated in the figures may be performed by corresponding functional means capable of performing the operations.

The various illustrative logical blocks, modules and circuits described in connection with the present disclosure (such as the blocks of Figures 5 and 6) may be implemented or performed with a general purpose processor, a digital signal processor (DSP), an application specific integrated circuit (ASIC), a field programmable gate array signal (FPGA) or other programmable logic device (PLD), discrete gate or transistor logic, discrete hardware components or any combination thereof designed to perform the functions described herein. A general purpose processor may be a microprocessor, but in the alternative, the processor may be any commercially available processor, controller, microcontroller or state machine. A processor may also be implemented as a combination of computing devices, e.g., a combination of a DSP and a microprocessor, a plurality of microprocessors, one or more microprocessors in conjunction with a DSP core, or any other such configuration.

In one or more aspects, the functions described may be implemented in hardware, software, firmware, or any combination thereof. If implemented in software, the functions may be stored on or transmitted over as one or more instructions or code on a computer-readable medium. Computer-readable media includes both computer storage media and communication media including any medium that facilitates transfer of a computer program from one place to another. A storage media may be any available media that can be accessed by a computer. By way of example, and not limitation, such computer-readable media can comprise RAM, ROM, EEPROM, CD-ROM or other optical disk storage, magnetic disk storage or other magnetic storage devices, or any other medium that can be used to carry or store desired program code in the form of instructions or data structures and that can be accessed by a computer. Also, any connection is properly termed a computer-readable medium. For example, if the software is transmitted from a website, server, or other remote source using a coaxial cable, fiber optic cable, twisted pair, digital subscriber line (DSL), or wireless technologies such as infrared, radio, and microwave, then the coaxial cable, fiber optic cable, twisted pair, DSL, or wireless technologies such as infrared, radio, and microwave are included in the definition of medium. Disk and disc, as used herein, includes compact disc (CD), laser disc, optical disc, digital versatile disc (DVD), floppy disk and Blu-ray® disc where disks usually reproduce data magnetically, while discs reproduce data optically with lasers. Thus, in some aspects a computer readable medium may comprise non-transitory computer readable medium (e.g., tangible media). In addition, in some aspects a computer readable medium may comprise transitory computer readable medium (e.g., a signal). Combinations of the above should also be included within the scope of computer-readable media.

The methods disclosed herein comprise one or more steps or actions for achieving the described methods. The method steps and/or actions may be interchanged with one another without departing from the scope of the claims. In other words, unless a specific order of steps or actions is specified, the order and/or use of specific steps and/or actions may be modified without departing from the scope of the claims.

Certain aspects may comprise a computer program product for performing the operations presented herein. For example, such a computer program product may comprise a computer readable medium having instructions stored (and/or encoded) thereon, the instructions being executable by one or more processors to perform the operations described herein. For certain aspects, the computer program product may include packaging material.

Software or instructions may also be transmitted over a transmission medium. For example, if the software is transmitted from a website, server, or other remote source using a coaxial cable, fiber optic cable, twisted pair, digital subscriber line (DSL), or wireless technologies such as infrared, radio, and microwave, then the coaxial cable, fiber optic cable, twisted pair, DSL, or wireless technologies such as infrared, radio, and microwave are included in the definition of transmission medium.

Further, it should be appreciated that modules and/or other appropriate means for performing the methods and techniques described herein can be downloaded and/or otherwise obtained by a user terminal and/or base station as applicable. For example, such a device can be coupled to a server to facilitate the transfer of means for performing the methods described herein. Alternatively, various methods described herein can be provided via storage means (e.g., RAM, ROM, a physical storage medium such as a compact disc (CD) or floppy disk, etc.), such that a user terminal and/or base station can obtain the various methods upon coupling or providing the storage means to the device. Moreover, any other suitable technique for providing the methods and techniques described herein to a device can be utilized.

It is to be understood that the claims are not limited to the precise configuration and components illustrated above. Various modifications, changes and variations may be made in the arrangement, operation and details of the methods and apparatus described above without departing from the scope of the claims.

Unless otherwise defined, all terms (including technical and scientific terms) are to be given their ordinary and customary meaning to a person of ordinary skill in the art, and are not to be limited to a special or customized meaning unless expressly so defined herein. It should be noted that the use of particular terminology when describing certain features or aspects of the disclosure should not be taken to imply that the terminology is being re-defined herein to be restricted to include any specific characteristics of the features or aspects of the disclosure with which that terminology is associated. Terms and phrases used in this application, and variations thereof, especially in the appended claims, unless otherwise expressly stated, should be construed as open ended as opposed to limiting. As examples of the foregoing, the term 'including' should be read to mean 'including, without limitation,' 'including but not limited to,' or the like; the term 'comprising' as used herein is synonymous with 'including,' 'containing,' or 'characterized by,' and is inclusive or open-ended and does not exclude additional, unrecited elements or method steps; the term 'having' should be interpreted as 'having at least;' the term 'includes' should be interpreted as 'includes but is not limited to;' the term 'example' is used to provide exemplary instances of the item in discussion, not an exhaustive or limiting list thereof; adjectives such as 'known', 'normal', 'standard', and terms of similar meaning should not be construed as limiting the item described to a given time period or to an item available as of a given time, but instead should be read to encompass known, normal, or standard technologies that may be available or known now or at any time in the future; and use of terms like 'preferably,' 'preferred,' 'desired,' or 'desirable,' and words of similar meaning should not be understood as implying that certain features are critical, essential, or even important to the structure or function of the invention, but instead as merely intended to highlight alternative or additional features that may or may not be utilized in a particular embodiment of the invention. Likewise, a group of items linked with the conjunction 'and' should not be read as requiring that each and every one of those items be present in the grouping, but rather should be read as 'and/or' unless expressly stated otherwise. Similarly, a group of items linked with the conjunction 'or' should not be read as requiring mutual exclusivity among that group, but rather should be read as 'and/or' unless expressly stated otherwise.

Where a range of values is provided, it is understood that the upper and lower limit and each intervening value between the upper and lower limit of the range is encompassed within the embodiments.

With respect to the use of substantially any plural and/or singular terms herein, those having skill in the art can translate from the plural to the singular and/or from the singular to the plural as is appropriate to the context and/or application. The various singular/plural permutations may be expressly set forth herein for sake of clarity. The indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. Any reference signs in the claims should not be construed as limiting the scope.

It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to embodiments containing only one such recitation, even when the same claim includes the introductory phrases "one or more" or "at least one" and indefinite articles such as "a" or "an" (e.g., "a" and/or "an" should typically be interpreted to mean "at least one" or "one or more"); the same holds true for the use of definite articles used to introduce claim recitations. In addition, even if a specific number of an introduced claim recitation is explicitly recited, those skilled in the art will recognize that such recitation should typically be interpreted to mean at least the recited number (e.g., the bare recitation of "two recitations," without other modifiers, typically means at least two recitations, or two or more recitations). Furthermore, in those instances where a convention analogous to "at least one of A, B, and C, etc." is used, in general such a construction is intended in the sense one having skill in the art would understand the convention, e.g., as including any combination of the listed items, including single members (e.g., "a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (e.g., "a system having at least one of A, B, or C" would include but not be limited to systems that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A" or "B" or "A and B."

All numbers expressing quantities of ingredients, reaction conditions, and so forth used in the specification are to be understood as being modified in all instances by the term 'about.' Accordingly, unless indicated to the contrary, the numerical parameters set forth herein are approximations that may vary depending upon the desired properties sought to be obtained. At the very least, and not as an attempt to limit the application of the doctrine of equivalents to the scope of any claims in any application claiming priority to the present application, each numerical parameter should be construed in light of the number of significant digits and ordinary rounding approaches.

All references cited herein are incorporated herein by reference in their entirety. To the extent publications and patents or patent applications incorporated by reference contradict the disclosure contained in the specification, the specification is intended to supersede and/or take precedence over any such contradictory material.

Headings are included herein for reference and to aid in locating various sections. These headings are not intended to limit the scope of the concepts described with respect thereto. Such concepts may have applicability throughout the entire specification.

Furthermore, although the foregoing has been described in some detail by way of illustrations and examples for purposes of clarity and understanding, it is apparent to those skilled in the art that certain changes and modifications may be practiced. Therefore, the description and examples should not be construed as limiting the scope of the invention to the specific embodiments and examples described herein, but rather to also cover all modification and alternatives coming with the true scope and spirit of the invention.

The present description also includes the following numbered clauses:

1. A method of assessing a user's urgency state associated with a physiological condition, comprising:

   a. receiving data of a first type associated with a physiological condition;
   b. calculating data of a second type associated with the physiological condition;
   c. receiving data of a third type associated with the physiological condition;
   d. determining an urgency index based at least on the received data of the first, second, and third types; and
   e. providing an indication of the determined urgency index on a mobile device.

2. The method of clause 1, wherein the physiological condition is diabetes, the urgency index is a glycemic urgency index, and the data of the first type is a glucose concentration.

3. The method of clause 2, wherein the glucose concentration is a current measured glucose concentration, a past measured glucose concentration, or a future predicted glucose concentration.

4. The method of clause 1, 2, or 3, wherein the data of the second type is derived from the data of the first type.

5. The method of clause 4, wherein the data of the second type derived from the data of the first type is a first derivative or a second derivative with respect to time of the data of the first type.

6. The method of clause 4, wherein the data of the second type derived from the data of the first type includes: deviations from normal glucose patterns, pattern data of glucose values over a time period, predicted glucose values, a duration over which a glucose value is within a predetermined range, weightings of parameters or variables to be considered in the determining of the urgency index, or local maxima or minima in the data of the first type.

7. The method of any preceding clause, wherein the receiving data of a third type includes receiving data entered by a user.

8. The method of clause 7, wherein the receiving includes receiving data entered by a user on a user interface of a mobile device.

9. The method of clause 7 or 8, wherein the physiological condition is diabetes, the urgency index is a glycemic urgency index, and the data of the first type is a glucose concentration, wherein the received data entered by a user includes a user weight, a user indication of activity level, a user indication of food or drink ingested or to be ingested, anthropometric data, data about prior insulin provided to the user, stress data, health data, data about a placement of the sensor measuring the data of the first type, age, or gender.

10. The method of clause 7, 8, or 9, wherein the received data entered by a user includes a user indication of food or drink ingested or to be ingested, or data about prior insulin provided to the user or to be provided to the user, and further comprising determining the glycemic urgency index to be a lower urgency based on the received data.

11. The method of any preceding clause, wherein the receiving data of a third type includes receiving data from a sensor.

12. The method of clause 11, wherein the physiological condition is diabetes, the urgency index is a glycemic urgency index and the data of the first type is a glucose concentration, and wherein the sensor includes at least one of the following: a scale, a glucometer, a thermometer, an accelerometer, a camera, a GPS device, or a microphone.

13. The method of clause 12, wherein the received data of a third type includes a user weight, a user indication of activity level, an indication of food or drink ingested or to be ingested, anthropometric data, data about prior insulin provided to the user, physiological data, stress data, or health data.

14. The method of any preceding clause, wherein the receiving data of a third type includes receiving data from a query processing engine, an electronic device configured for machine to machine communication, or an electronic user record.

15. The method of any preceding clause, wherein the data of the third type is received from the mobile device, and corresponds to a level of user interaction with an application through which the indication was provided.

16. The method of any preceding clause, further comprising providing an alert or alarm if the urgency index reaches a predetermined alerting or alarming threshold, respectively.

17. The method of clause 16, wherein the urgency index is a glycemic index and the predetermined alerting or alarming threshold indicates that the user is in a hypoglycemic or hyperglycemic state.

18. The method of clause 1, wherein the physiological condition comprises one or more of obesity, malnutrition, hyperactivity, depression, or fertility.

19. The method of any preceding clause, further comprising providing an advanced output based on the received inputs.

20. A system for performing the method of any of clauses 1- 19, optionally comprising computer implemented control means configured to perform the method.

21. A method of determining a user's urgency state associated with a physiological condition, comprising:

a. determining an urgency index based on an analyte concentration and at least two variables selected from the group consisting of: a first or second time derivative of the analyte concentration, a duration the analyte concentration has occupied a predetermined range, a duration the first or second time derivative of the analyte concentration has occupied a predetermined range, a past or future meal intake parameter entered by a user, a past or future medicament parameter entered by a user or received from an integrated pump; and
b. storing the determined urgency index in a storage of a mobile device.

22. The method of clause 22, further comprising displaying or otherwise outputting an indication of the urgency index on a user interface of the mobile device.

23. The method of clause 22, wherein the displaying step further comprises overriding other applications or processes operating on the mobile device, such that the indication of the urgency index is displayed regardless of other running applications or processes.

24. The method of clause 22, wherein the displaying step is caused by a user action, the user action selected from the group consisting of: handling the mobile device, unlocking the mobile device, or performing a swiping action on the mobile device.

25. The method of clause 21, 22, 23, or 24, wherein the indication of the urgency index is a rendered element.

26. The method of clause 25, wherein the rendered element includes a color.

27. The method of clause 25, wherein the rendered element includes an icon.

28. The method of clause 27, wherein a position, size, or color of the icon is based on the urgency index.

29. The method of clause 27 or 28, further comprising:

a. receiving an indication that a user has activated the icon; and
b. displaying additional information or an advanced output about the urgency index.

30. The method of clause 26, 27, 28, or 29, wherein the rendered element is rendered as at least a portion of a home screen or a background, native to an operating system of the mobile device.

31. The method of any one of clauses 21 to 30, wherein the analyte is glucose, the urgency index is a glycemic urgency index, and the medicament parameter corresponds to insulin.

32. The method of clause 31, further comprising rendering a series of elements on the user interface of the mobile device, the series of elements indicating past or predicted future values of glucose concentration.

33. The method of clause 22 or any one of clauses 23 to 32 as dependent on clause 22, wherein the indication of the urgency index includes an audible or visual alert rendered or sounded, respectively, on the mobile device.

34. The method of any one of clauses 21 to 33, further comprising displaying a prompt for a user to enter data, such that user data may be associated with the urgency index.

35. The method of clause 34, wherein the prompt for a user to enter data indicates a type of data, and wherein the type of data is selected from the group consisting of: exercise or activity level, meal data, insulin data, stress or health data, or emotional data.

36. The method of any one of clauses 21 to 35, further comprising displaying at least one potential action a user may perform in response to the displayed indication of the urgency index.

37. The method of clause 22 or any one of clauses 23 to 36 as dependent on clause 22, wherein the indication includes an actionable alert.

38. The method of clause 22, any one of clauses 23 to 37 as dependent on clause 22, wherein the displaying an indication includes displaying occupancy of a band, the band corresponding to a predetermined range of urgency index values.

39. The method of any one of clauses 21 to 38, further comprising transmitting the stored urgency index to an integrated pump.

40. The method of any one of clauses 21 to 39, further comprising transforming the stored urgency index to a pump action, and transmitting the pump action to an integrated pump.

41. A system for performing the method of any of clauses 21- 40, optionally comprising computer implemented control means configured to perform the method.

42. A device substantially as shown and/or described in the specification and/or drawings.

43. A system substantially as shown and/or described in the specification and/or drawings.

44. A method substantially as shown and/or described in the specification and/or drawings.

45. An electronic device for monitoring data associated with a physiological condition, comprising:

a. a continuous analyte sensor, wherein the continuous analyte sensor is configured to substantially continuously measure the concentration of analyte in the host, and to provide continuous sensor data associated with the analyte concentration in the host; and

b. a processor module configured to perform any one of the methods of clauses 1-19, 21 - 40, or 44.

46. The device of clause 45, wherein the analyte is glucose.

47. An electronic device for delivering a medicament to a host, the device comprising:

a. a medicament delivery device configured to deliver medicament to the host, wherein the medicament delivery device is operably connected to a continuous analyte sensor, wherein the continuous analyte sensor is configured to substantially continuously measure the concentration of analyte in the host, and to provide continuous sensor data associated with the analyte concentration in the host; and

b. a processor module configured to perform any one of the methods of clauses 1-19, 21 - 40, or 44.

48. The device of clause 47, wherein the analyte is glucose and the medicament is insulin.

**Claims**

1. A method of assessing a user's urgency state associated with a physiological condition, comprising:

   a. receiving data of a first type associated with a physiological condition;
   b. calculating data of a second type associated with the physiological condition;
   c. receiving data of a third type associated with the physiological condition;
   d. determining an urgency index based at least on the received data of the first, second, and third types; and
   e. providing an indication of the determined urgency index on a mobile device.

2. The method of claim 1, wherein the physiological condition is diabetes, the urgency index is a glycemic urgency index, and the data of the first type is a glucose concentration, and/or, wherein the glucose concentration is a current measured glucose concentration, a past measured glucose concentration, or a future predicted glucose concentration.

3. The method of claim 1 or 2, wherein the data of the second type is derived from the data of the first type.

4. The method of claim 3, wherein the data of the second type derived from the data of the first type is a first derivative or a second derivative with respect to time of the data of the first type, or, wherein the data of the second type derived from the data of the first type includes: deviations from normal glucose patterns, pattern data of glucose values over a time period, predicted glucose values, a duration over which a glucose value is within a predetermined range, weightings of parameters or variables to be considered in the determining of the urgency index, or local maxima or minima in the data of the first type.

5. The method of any preceding claim, wherein the receiving data of a third type includes receiving data entered by a user.

6. The method of claim 5, wherein the receiving includes receiving data entered by a user on a user interface of a mobile device, and/or, wherein the physiological condition is diabetes, the urgency index is a glycemic urgency index, and the data of the first type is a glucose concentration, wherein the received data entered by a user includes a user weight, a user indication of activity level, a user indication of food or drink ingested or to be ingested, anthropometric data, data about prior insulin provided to the user, stress data, health data, data about a placement of the sensor measuring the data of the first type, age, or gender.

7. The method of claim 5 or 6, wherein the received data entered by a user includes a user indication of food or drink ingested or to be ingested, or data about prior insulin provided to the user or to be provided to the user, and further comprising determining the glycemic urgency index to be a lower urgency based on the received data.

8. The method of any preceding claim, wherein the receiving data of a third type includes receiving data from a sensor.

9. The method of claim 8, wherein the physiological condition is diabetes, the urgency index is a glycemic urgency index and the data of the first type is a glucose concentration, and wherein the sensor includes at least one of the following: a scale, a glucometer, a thermometer, an accelerometer, a camera, a GPS device, or a microphone, and optionally, wherein the received data of a third type includes a user weight, a user indication of activity level, an indication of food or drink ingested or to be ingested, anthropometric data, data about prior insulin provided to the user, physiological data, stress data, or health data.

10. The method of any preceding claim, wherein the receiving data of a third type includes receiving data from a query processing engine, an electronic device configured for machine to machine communication, or an electronic user record, and/or, wherein the data of the third type is received from the mobile device, and corresponds to a level of user interaction with an application through which the indication was provided.

11. The method of any preceding claim, further comprising providing an alert or alarm if the urgency index reaches a predetermined alerting or alarming threshold, respectively.

12. The method of claim 11, wherein the urgency index is a glycemic index and the predetermined alerting or alarming threshold indicates that the user is in a hypoglycemic or hyperglycemic state.

13. The method of claim 1, wherein the physiological condition comprises one or more of obesity, malnutrition, hyperactivity, depression, or fertility.

14. The method of any preceding claim, further comprising providing an advanced output based on the received inputs.

15. A system for performing the method of any of claims 1-14, optionally comprising computer implemented control means configured to perform the method.

FIG. 1

FIG. 2

FIG. 3

EP 4 503 047 A2

**FIG. 4**

FIG. 5

FIG. 6

GLYCEMIC
URGENCY
INDEX — 252

CURRENT
MEASURED
GLUCOSE
DATA — 254

HISTORIC
MEASURED
GLUCOSE
DATA — 256

EXTERNAL
DATA, E.G.,
HOW USER
FEELS, MEAL
DATA, ETC. — 258

RECENT
HISTORIC
MEASURED
GLUCOSE
DATA — 262

OLDER
HISTORIC
MEASURED
GLUCOSE
DATA — 264

DATA BASED
ON MEASURED
GLUCOSE
DATA — 266

TIME RATES
OF CHANGE — 268

FIG. 7

EP 4 503 047 A2

```
                                    ┌──────────────┐
                                    │    DRIVE     │  ┌275
                                    │  INTEGRATED  │
                                    │     PUMP     │
                                    └──────────────┘

      ┌272              ┌274              ┌276
┌──────────────┐  ┌──────────────┐  ┌──────────────┐
│ RECEIVING A  │  │  DETERMINING │  │ PROVIDING AN │
│ PLURALITY    │  │  A GLYCEMIC  │  │ INDICATION OF│
│ (E.G.,       │  │ URGENCY INDEX│  │ THE DETERMINED│
│ A FIRST AND A│──│  BASED ON THE│──│ OR CALCULATED│
│ SECOND OR    │  │   RECEIVED   │  │   GLYCEMIC   │
│ MORE INPUTS  │  │    INPUTS    │  │ URGENCY INDEX│
│ ASSOCIATED   │  │              │  │              │
│ WITH DIABETES│  │              │  │              │
└──────────────┘  └──────────────┘  └──────────────┘

      ┌278                    ┌282
┌──────────────┐        ┌──────────────┐
│   CHANGE     │        │              │
│ STATUS/STATE OR│      │   PROVIDE    │
│ PROVIDE ALERT OR│     │  ADVANCED    │
│ ALARM BASED ON │─────│   OUTPUT     │
│ GLYCEMIC URGENCY│     │              │
│ INDEX AND IN SOME│    └──────────────┘
│ CASES ON THRESHOLD│
└──────────────┘                           ┌40
```

FIG. 8

FIG. 9

EP 4 503 047 A2

Actual Glucose Concentration - - - - - - -

Pattern Glucose Concentration - - - - -

GUI without consideration of Pattern _____

GUI with consideration of Pattern ▬▬▬▬

289

Hyperglycemic

Target

Hypoglycemic

295

297

299

301

I  II  III  IV  V  VI  VII

311

309

291

Very Urgent

Medium Urgency

Low Urgency

No Urgency

wake  work  lunch  work  dinner  evening  sleep

time

60

FIG. 10

EP 4 503 047 A2

FIG. 11

FIG. 12A

FIG. 12B

EP 4 503 047 A2

FIG. 13

FIG. 14

FIG. 15A

FIG. 15B

FIG. 15C

USER CURRENT
HEALTH STATE

FIG. 15D

2 units

INSULIN FROM PUMP

FIG. 15E

ACCELERATION

FIG. 15F

EP 4 503 047 A2

FIG. 16A

FIG. 16B

EP 4 503 047 A2

FIG. 17B

FIG. 17A

⊠ White
▨ Red

FIG. 18A

FIG. 18B

⊠ White

⊘ Red

FIG. 19A

FIG. 19B

EP 4 503 047 A2

FIG. 20B

FIG. 20A

FIG. 21B

FIG. 21A

White

Red

FIG. 22A

FIG. 22B

⊠ White

EP 4 503 047 A2

FIG. 23

FIG. 24

FIG. 25B

FIG. 25A

FIG. 26

EP 4 503 047 A2

FIG. 27A

ROAD TRIP
Last time you did this you
reported injecting 20cc of
insulin and that you felt ALERT!

POKER NI
Last time yo
reported an
you fell Mon

638

RACE DAY!
Last time you did this you
reported a high carb breakfast
and that you felt UNBEATABLE!

PIZZA AN
Last time yo
reported inje
and that you

670

FIG. 27B

FIG. 28A

FIG. 28B

680

White
Red

642
644

Glycemic Urgency Index

Your urgency assessment, based on multiple factors:

LOW RISK

Learn more about this state

EP 4 503 047 A2

FIG. 29

FIG. 30

*666*

Your glucose is approximately 100 mg/dL and is dropping quickly, what can you do to avoid or reduce a potential low glucose?

- Nothing. I've just taken some carbs to try and avoid a low blood glucose. I'll re-check my glucose in about 15 minutes.
- Take some carbs to try and avoid a low glucose situation
- Wait. Remind me in 15 minutes if my glucose continues to drop towards a low glucose level.

*668*

*730*

# FIG. 31A

*666*

Your glucose just reached your high glucose limit and is still rising quickly. What do you want to do to avoid a higher glucose levels?

- Nothing. I've already taken insulin and am waiting for the insulin to bring my glucose levels down. Remind me in 30 minutes if my glucose levels have not stopped rising.
- Take some insulin to try and avoid higher glucose levels.
- Wait. Remind me in 30 minutes if my glucose continues to rise towards a high glucose level.

*668*

*730*

# FIG. 31B

EP 4 503 047 A2

```
┌──────────────────┐        ┌──────────────────┐              ┌──────────────────┐
│    ╱─672         │        │    ╱─674         │              │    ╱─676         │
│  RETROSPECTIVE   │        │  DETERMINE IF    │              │    DISPLAY /     │
│   ALGORITHM      │        │ EXCURSION FALLS  │              │ RENDER / SOUND   │
│ EXAMINES DATA    │───────▶│   OUTSIDE A      │     YES      │   DETERMINED     │
│ FOR GLYCEMIC     │        │ THRESHOLD, I.E., │─────────────▶│ RESULT (OUTPUT / │
│  EVENT, E.G.,    │        │  CONSTITUTES A   │              │  ALERT / ALARM), │
│  SIGNIFICANT     │        │   MEANINGFUL     │              │  BASED ON EVENT  │
│   EXCURSIONS     │        │     EVENT        │              │                  │
└──────────────────┘        └──────────────────┘              └──────────────────┘
        ▲                            │
        │                           NO
        └────────────────────────────┘
```

╱─740

# FIG. 32A

FIG. 32B

EP 4 503 047 A2

FIG. 32C

**EP 4 503 047 A2**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61978151
- US 74269413
- US 6001067 A
- US 20110027127 A1
- US 20060020187 A1
- US 20090137887 A1
- US 20070027385 A1
- US 61904341
- US 79618513

- US 25834508
- US 20090192366 A1
- US 00792004
- US 8282549 B
- US 74774613
- US 20140005508 A1
- US 61898300
- US 61904396
- US 82711913

**Non-patent literature cited in the description**

- **KOVATCHEV**. Risk Analysis of Blood Glucose Data: A Quantitative Approach to Optimizing the Control of Insulin-Dependent Diabetes. *Journal of Theoretical Medicine*, 2000, vol. 3, 1-10

- **SIMILARLY** ; **GUERRA**. A Dynamic Risk Measure from Continuous Glucose Monitoring Data. *Diabetes Technology & Therapeutics*, 2011, vol. 13 (8)